(19) Europäisches Patentamt

European Patent Office

Office européen des brevets

(11) **EP 4 610 355 A1**

## (12) EUROPEAN PATENT APPLICATION
published in accordance with Art. 153(4) EPC

(43) Date of publication:
**03.09.2025 Bulletin 2025/36**

(21) Application number: **23881753.0**

(22) Date of filing: **20.10.2023**

(51) International Patent Classification (IPC):
*C12N 15/113* (2010.01)     *C12N 15/85* (2006.01)
*C12N 5/10* (2006.01)      *A61K 31/7105* (2006.01)
*A61K 48/00* (2006.01)     *A61P 3/10* (2006.01)
*A61P 13/12* (2006.01)     *A61P 17/06* (2006.01)
*A61P 19/02* (2006.01)     *A61P 27/02* (2006.01)
*A61P 35/00* (2006.01)     *A61P 37/02* (2006.01)

(52) Cooperative Patent Classification (CPC):
**A61K 31/7105; A61K 48/00; A61P 3/10;
A61P 13/12; A61P 17/06; A61P 19/02; A61P 27/02;
A61P 35/00; A61P 37/02; C12N 5/10;
C12N 15/113; C12N 15/85**

(86) International application number:
**PCT/CN2023/125680**

(87) International publication number:
**WO 2024/088175 (02.05.2024 Gazette 2024/18)**

(84) Designated Contracting States:
**AL AT BE BG CH CY CZ DE DK EE ES FI FR GB
GR HR HU IE IS IT LI LT LU LV MC ME MK MT NL
NO PL PT RO RS SE SI SK SM TR**
Designated Extension States:
**BA**
Designated Validation States:
**KH MA MD TN**

(30) Priority: **26.10.2022   CN 202211320859
18.01.2023   CN 202310088096
12.04.2023   CN 202310396140**

(71) Applicant: **Guangzhou Reforgene Medicine Co.,
Ltd.
Guangzhou, Guangdong 510535 (CN)**

(72) Inventors:
• **LIANG, Junbin
  Guangzhou, Guangdong 510535 (CN)**

• **ZHANG, Hong
  Guangzhou, Guangdong 510535 (CN)**
• **XU, Hui
  Guangzhou, Guangdong 510535 (CN)**
• **LUO, Jiawen
  Guangzhou, Guangdong 510535 (CN)**
• **JI, Cunhua
  Guangzhou, Guangdong 510535 (CN)**
• **HUANGFU, Desheng
  Guangzhou, Guangdong 510535 (CN)**

(74) Representative: **Murgitroyd & Company
165-169 Scotland Street
Glasgow G5 8PL (GB)**

Remarks:
The complete document including Reference
Table(s) and the Sequence Listing(s) can be
downloaded from the EPO website

## (54) GENE EDITING SYSTEM AND USE THEREOF

(57)     Disclosed in the present disclosure are a gRNA targeting VEGFA, a gene editing system, and the use thereof. The gRNA in the present disclosure comprises a guide sequence which has at least 80% sequence identity to any one of SEQ ID NOs: 10-18 and 40-230. The gene editing system in the present disclosure comprises the gRNA of the present disclosure and an encoding sequence thereof, and an RNA-guided nuclease and an encoding sequence thereof.

**Description**

The present application claims the priorities of Chinese patent application 2022113208591 with the filing date of October 26, 2022, Chinese patent application 2023100880960 with the filing date of January 18, 2023, and Chinese patent application 2023103961404 with the filing date April 12, 2023. The entire contents of the above Chinese patent applications are incorporated herein by reference.

TECHNICAL FIELD

The present disclosure belongs to the field of gene editing technology, specifically relates to a gene editing system and a use thereof; more specifically, to a gRNA targeting VEGFA, a CRISPR system and a use thereof.

BACKGROUND

The angiogenesis signaling pathway in which vascular endothelial growth factor A (VEGFA) participates regulates a variety of physiological processes and disease-related signaling pathways in organisms, such as the angiogenesis process from embryonic development to various organs and tissues. When specific tissues or organs are in a state of hypoxia, VEGFA in the cells will be activated by the hypoxia-inducible factor HIF transcription factor and its expression is upregulated. The upregulated VEGFA binds to its receptor, activates the angiogenesis signaling pathway, and provides nutrients to the body. In various cancers and other diseases, tissues are in a state of hypoxia, leading to the significant upregulation of VEGFA expression and the activation of the angiogenesis pathway. Studies have found that VEGFA-related angiogenesis signaling pathways are upregulated in various pathological processes, such as lung cancer, breast cancer, liver cancer, glioblastoma and other cancer processes, as well as diabetic nephropathy, neovascular age related macular degeneration, neovascular glaucoma, rheumatoid arthritis, psoriasis, ovarian hyperstimulation syndrome and other major diseases, in which VEGF expression is significantly upregulated.

The VEGF antibody Aflibercept, already on the market, and the anti-VEGF overexpression drug RGX-314, currently in clinical trials, are both used to treat diabetic macular edema (DME), macular edema following retinal vein occlusion (RVO), diabetic retinopathy (DR), *etc.,* and have good effects. Bayer's anti-VEGF Aflibercept is in Phase III clinical trials for colorectal cancer and non-small cell lung cancer. Genentech's Anti-VEGF Bevacizumab is used to treat colon cancer, lung cancer, breast cancer, glioblastoma and kidney cancer, *etc.* Clinical trials for the treatment of colon cancer and breast cancer have entered Phase III.

Age related macular degeneration (AMD) has become the leading cause of blindness in people over 50 years old worldwide, causing irreversible visual impairment. AMD is divided into dry (atrophic) and wet (neovascular) subtypes. Neovascular age related macular degeneration (nAMD), accounting for 10 to 15% of cases, is one of the primary causes of vision loss. The clinical symptoms of nAMD patients are gradual or sudden severe visual impairment, fundus hemorrhage, exudation, accompanied by choroidal neovascularization and disciform scarring in the macular region. Neovascular age related macular degeneration begins at the age of 50, with an incidence rate as high as 13% in the elderly population over 60 years old. Current studies have demonstrated that the key molecular mechanism of nAMD is that retinal pigment epithelium (RPE) cells overexpress vascular endothelial growth factor (VEGFA) due to genetic or external stress stimulation, thereby activating the angiogenesis signaling pathway, causing retinal hemorrhage and severe visual impairment. Therefore, intravitreal injection of VEGF antibody drugs can effectively inhibit retinal and choroidal neovascularization and the deterioration of central vision damage.

Intravitreal injection of VEGF antibody drug is currently a treatment option with good efficacy and the largest market share in the treatment of nAMD. VEGF antibody drugs generally need to be injected into the vitreous humour every 1-3 months.

CRISPR/Cas (clustered regularly interspaced short palindromic repeats/CRISPR-associated proteins) system is currently the most widely used gene editing technology. For disease therapies based on CRISPR technology, it is still very difficult to obtain "Cas protein + gRNA" drug molecules with high editing efficiency and high safety, which is a problem that the field has been working to solve.

SUMMARY

The present disclosure provides a guide RNA targeting VEGFA, a gene editing system and a use thereof.

The first aspect of the present disclosure provides a guide RNA (gRNA) for a gene editing system, comprising a guide sequence having at least 80% sequence identity to the sequence as shown in any one of SEQ ID NOs: 10-18 and 40-230.

In some embodiments, the guide sequence is engineered to hybridize with a target RNA.

In some embodiments of the present disclosure, the guide sequence has at least 85%, at least 90%, at least 95%, at least 96%, at least 97%, at least 98% or 100% sequence identity to the sequence as shown in any one of SEQ ID NOs: 10-18 and 40-230. In some embodiments of the present disclosure, the guide sequence has 100% sequence identity to the sequence as shown in any one of SEQ ID NOs: 10-18 and 40-230. In some embodiments of the present disclosure, the guide sequence is selected from the sequence as shown in any one of SEQ ID NOs: 10-18 and 40-230. In some embodiments, the guide sequence is engineered to hybridize with a target RNA.

In some embodiments of the present disclosure, the guide sequence has at least 80% sequence identity to the sequence as shown in any one of SEQ ID NOs: 12, 13, 15, 40-43, 45-47, 52-60, 64, 65, 73, 216, 219, 220, 223, 227, 228, 229 and 230. In some embodiments of the present disclosure, the guide sequence has at least 85%, at least 90%, at least 95%, at least 96%, at least 97%, at least 98% or 100% sequence identity to the sequence as shown in any one of SEQ ID NOs: 12, 13, 15, 40-43, 45-47, 52-60, 64, 65, 73, 216, 219, 220, 223, 227, 228, 229 and 230. In some embodiments of the present disclosure, the guide sequence has 100% sequence identity to the sequence as shown in any one of SEQ ID NOs: 12, 13, 15, 40-43, 45-47, 52-60, 64, 65, 73, 216, 219, 220, 223, 227, 228, 229 and 230. In some embodiments of the present disclosure, the guide sequence is selected from the sequence as shown in any one of SEQ ID NOs: 12, 13, 15, 40-43, 45-47, 52-60, 64, 65, 73, 216, 219, 220, 223, 227, 228, 229 and 230. In some embodiments, the guide sequence is engineered to hybridize with a target RNA.

In some embodiments of the present disclosure, the guide sequence has at least 80% sequence identity to the sequence as shown in any one of SEQ ID NOs: 12, 13, 15, 47, 58, 59, 227, 228 and 230. In some embodiments of the present disclosure, the guide sequence has at least 85%, at least 90%, at least 95%, at least 96%, at least 97%, at least 98% or 100% sequence identity to the sequence as shown in any one of SEQ ID NOs: 12, 13, 15, 47, 58, 59, 227, 228 and 230. In some embodiments of the present disclosure, the guide sequence has 100% sequence identity to the sequence as shown in any one of SEQ ID NOs: 12, 13, 15, 47, 58, 59, 227, 228 and 230. In some embodiments of the present disclosure, the guide sequence is selected from the sequence as shown in any one of SEQ ID NOs: 12, 13, 15, 47, 58, 59, 227, 228 and 230. In some embodiments, the guide sequence is engineered to hybridize with a target RNA.

In some embodiments of the present disclosure, the guide sequence has at least 80% sequence identity to the sequence as shown in SEQ ID NO: 12. In some embodiments of the present disclosure, the guide sequence has at least 85%, at least 90%, at least 95% or 100% sequence identity to the sequence as shown in SEQ ID NO: 12. In some embodiments of the present disclosure, the guide sequence has 100% sequence identity to the sequence as shown in SEQ ID NO: 12. In some embodiments of the present disclosure, the guide sequence is the sequence as shown in SEQ ID NO: 12. In some embodiments, the guide sequence is engineered to hybridize with a target RNA.

In some embodiments of the present disclosure, the guide sequence has at least 80% sequence identity to the sequence as shown in SEQ ID NO: 13. In some embodiments of the present disclosure, the guide sequence has at least 85%, at least 90%, at least 95% or 100% sequence identity to the sequence as shown in SEQ ID NO: 13. In some embodiments of the present disclosure, the guide sequence has 100% sequence identity to the sequence as shown in SEQ ID NO: 13. In some embodiments of the present disclosure, the guide sequence is the sequence as shown in SEQ ID NO: 13. In some embodiments, the guide sequence is engineered to hybridize with a target RNA.

In some embodiments of the present disclosure, the guide sequence has at least 80% sequence identity to the sequence as shown in SEQ ID NO: 15. In some embodiments of the present disclosure, the guide sequence has at least 85%, at least 90%, at least 95% or 100% sequence identity to the sequence as shown in SEQ ID NO: 15. In some embodiments of the present disclosure, the guide sequence has 100% sequence identity to the sequence as shown in SEQ ID NO: 15. In some embodiments of the present disclosure, the guide sequence is the sequence as shown in SEQ ID NO: 15. In some embodiments, the guide sequence is engineered to hybridize with a target RNA.

In some embodiments of the present disclosure, the guide sequence has at least 80% sequence identity to the sequence as shown in SEQ ID NO: 40. In some embodiments of the present disclosure, the guide sequence has at least 85%, at least 90%, at least 95% or 100% sequence identity to the sequence as shown in SEQ ID NO: 40. In some embodiments of the present disclosure, the guide sequence has 100% sequence identity to the sequence as shown in SEQ ID NO: 40. In some embodiments of the present disclosure, the guide sequence is the sequence as shown in SEQ ID NO: 40. In some embodiments, the guide sequence is engineered to hybridize with a target RNA.

In some embodiments of the present disclosure, the guide sequence has at least 80% sequence identity to the sequence as shown in SEQ ID NO: 41. In some embodiments of the present disclosure, the guide sequence has at least 85%, at least 90%, at least 95% or 100% sequence identity to the sequence as shown in SEQ ID NO: 41. In some embodiments of the present disclosure, the guide sequence has 100% sequence identity to the sequence as shown in SEQ ID NO: 41. In some embodiments of the present disclosure, the guide sequence is the sequence as shown in SEQ ID NO: 41. In some embodiments, the guide sequence is engineered to hybridize with a target RNA.

In some embodiments of the present disclosure, the guide sequence has at least 80% sequence identity to the sequence as shown in SEQ ID NO: 42. In some embodiments of the present disclosure, the guide sequence has at least 85%, at least 90%, at least 95% or 100% sequence identity to the sequence as shown in SEQ ID NO: 42. In some embodiments of the present disclosure, the guide sequence has 100% sequence identity to the sequence as shown in SEQ ID NO: 42. In some embodiments of the present disclosure, the guide sequence is the sequence as shown in SEQ ID NO: 42. In some embodiments, the guide sequence is engineered to hybridize with a target RNA.

In some embodiments of the present disclosure, the guide sequence has at least 80% sequence identity to the sequence as shown in SEQ ID NO: 43. In some embodiments of the present disclosure, the guide sequence has at least 85%, at least 90%, at least 95% or 100% sequence identity to the sequence as shown in SEQ ID NO: 43. In some embodiments of the present disclosure, the guide sequence has 100% sequence identity to the sequence as shown in SEQ ID NO: 43. In some embodiments of the present disclosure, the guide sequence is the sequence as shown in SEQ ID NO: 43. In some embodiments, the guide sequence is engineered to hybridize with a target RNA.

In some embodiments of the present disclosure, the guide sequence has at least 80% sequence identity to the sequence as shown in SEQ ID NO: 45. In some embodiments of the present disclosure, the guide sequence has at least 85%, at least 90%, at least 95% or 100% sequence identity to the sequence as shown in SEQ ID NO: 45. In some embodiments of the present disclosure, the guide sequence has 100% sequence identity to the sequence as shown in SEQ ID NO: 45. In some embodiments of the present disclosure, the guide sequence is the sequence as shown in SEQ ID NO: 45. In some embodiments, the guide sequence is engineered to hybridize with a target RNA.

In some embodiments of the present disclosure, the guide sequence has at least 80% sequence identity to the sequence as shown in SEQ ID NO: 46. In some embodiments of the present disclosure, the guide sequence has at least 85%, at least 90%, at least 95% or 100% sequence identity to the sequence as shown in SEQ ID NO: 46. In some embodiments of the present disclosure, the guide sequence has 100% sequence identity to the sequence as shown in SEQ ID NO: 46. In some embodiments of the present disclosure, the guide sequence is the sequence as shown in SEQ ID NO: 46. In some embodiments, the guide sequence is engineered to hybridize with a target RNA.

In some embodiments of the present disclosure, the guide sequence has at least 80% sequence identity to the sequence as shown in SEQ ID NO: 47. In some embodiments of the present disclosure, the guide sequence has at least 85%, at least 90%, at least 95% or 100% sequence identity to the sequence as shown in SEQ ID NO: 47. In some embodiments of the present disclosure, the guide sequence has 100% sequence identity to the sequence as shown in SEQ ID NO: 47. In some embodiments of the present disclosure, the guide sequence is the sequence as shown in SEQ ID NO: 47. In some embodiments, the guide sequence is engineered to hybridize with a target RNA.

In some embodiments of the present disclosure, the guide sequence has at least 80%, at least 85%, at least 90%, at least 95% or 100% sequence identity to the sequence as shown in SEQ ID NO: 52. In some embodiments of the present disclosure, the guide sequence has 100% sequence identity to the sequence as shown in SEQ ID NO: 52. In some embodiments of the present disclosure, the guide sequence is the sequence as shown in SEQ ID NO: 52. In some embodiments, the guide sequence is engineered to hybridize with a target RNA.

In some embodiments of the present disclosure, the guide sequence has at least 80%, at least 85%, at least 90%, at least 95% or 100% identity to the sequence as shown in SEQ ID NO: 53. In some embodiments of the present disclosure, the guide sequence has 100% sequence identity to the sequence as shown in SEQ ID NO: 53. In some embodiments of the present disclosure, the guide sequence is the sequence as shown in SEQ ID NO: 53. In some embodiments, the guide sequence is engineered to hybridize with a target RNA.

In some embodiments of the present disclosure, the guide sequence has at least 80%, at least 85%, at least 90%, at least 95% or 100% sequence identity to the sequence as shown in SEQ ID NO: 54. In some embodiments of the present disclosure, the guide sequence has 100% sequence identity to the sequence as shown in SEQ ID NO: 54. In some embodiments of the present disclosure, the guide sequence is the sequence as shown in SEQ ID NO: 54. In some embodiments, the guide sequence is engineered to hybridize with a target RNA.

In some embodiments of the present disclosure, the guide sequence has at least 80%, at least 85%, at least 90%, at least 95% or 100% sequence identity to the sequence as shown in SEQ ID NO: 55. In some embodiments of the present disclosure, the guide sequence has 100% sequence identity to the sequence as shown in SEQ ID NO: 55. In some embodiments of the present disclosure, the guide sequence is the sequence as shown in SEQ ID NO: 55. In some embodiments, the guide sequence is engineered to hybridize with a target RNA.

In some embodiments of the present disclosure, the guide sequence has at least 80%, at least 85%, at least 90%, at least 95% or 100% sequence identity to the sequence as shown in SEQ ID NO: 56. In some embodiments of the present disclosure, the guide sequence has 100% sequence identity to the sequence as shown in SEQ ID NO: 56. In some embodiments of the present disclosure, the guide sequence is the sequence as shown in SEQ ID NO: 56. In some embodiments, the guide sequence is engineered to hybridize with a target RNA.

In some embodiments of the present disclosure, the guide sequence has at least 80%, at least 85%, at least 90%, at least 95% or 100% sequence identity to the sequence as shown in SEQ ID NO: 57. In some embodiments of the present disclosure, the guide sequence has 100% sequence identity to the sequence as shown in SEQ ID NO: 57. In some embodiments of the present disclosure, the guide sequence is the sequence as shown in SEQ ID NO: 57. In some embodiments, the guide sequence is engineered to hybridize with a target RNA.

In some embodiments of the present disclosure, the guide sequence has at least 80% sequence identity to the sequence as shown in SEQ ID NO: 58. In some embodiments of the present disclosure, the guide sequence has at least 85%, at least 90%, at least 95% or 100% sequence identity to the sequence as shown in SEQ ID NO: 58. In some embodiments of the present disclosure, the guide sequence has 100% sequence identity to the sequence as shown in SEQ ID NO: 58. In some embodiments of the present disclosure, the guide sequence is the sequence as shown in SEQ ID NO: 58. In some embodiments, the guide sequence is engineered to hybridize with a target RNA.

In some embodiments of the present disclosure, the guide sequence has at least 80% sequence identity to the sequence as shown in SEQ ID NO: 59. In some embodiments of the present disclosure, the guide sequence has at least 85%, at least 90%, at least 95% or 100% sequence identity to the sequence as shown in SEQ ID NO: 59. In some embodiments of the present disclosure, the guide sequence has 100% sequence identity to the sequence as shown in SEQ ID NO: 59. In some embodiments of the present disclosure, the guide sequence is the sequence as shown in SEQ ID NO: 59. In some embodiments, the guide sequence is engineered to hybridize with a target RNA.

In some embodiments of the present disclosure, the guide sequence has at least 80%, at least 85%, at least 90%, at least 95% or 100% sequence identity to the sequence as shown in SEQ ID NO: 60. In some embodiments of the present disclosure, the guide sequence has 100% sequence identity to the sequence as shown in SEQ ID NO: 60. In some embodiments of the present disclosure, the guide sequence is the sequence as shown in SEQ ID NO: 60. In some embodiments, the guide sequence is engineered to hybridize with a target RNA.

In some embodiments of the present disclosure, the guide sequence has at least 80%, at least 85%, at least 90%, at least 95% or 100% sequence identity to the sequence as shown in SEQ ID NO: 64. In some embodiments of the present disclosure, the guide sequence has 100% sequence identity to the sequence as shown in SEQ ID NO: 64. In some embodiments of the present disclosure, the guide sequence is the sequence as shown in SEQ ID NO: 64. In some embodiments, the guide sequence is engineered to hybridize with a target RNA.

In some embodiments of the present disclosure, the guide sequence has at least 80%, at least 85%, at least 90%, at least 95% or 100% sequence identity to the sequence as shown in SEQ ID NO: 65. In some embodiments of the present disclosure, the guide sequence has 100% sequence identity to the sequence as shown in SEQ ID NO: 65. In some embodiments of the present disclosure, the guide sequence is the sequence as shown in SEQ ID NO: 65. In some embodiments, the guide sequence is engineered to hybridize with a target RNA.

In some embodiments of the present disclosure, the guide sequence has at least 80%, at least 85%, at least 90%, at least 95% or 100% sequence identity to the sequence as shown in SEQ ID NO: 73. In some embodiments of the present disclosure, the guide sequence has 100% sequence identity to the sequence as shown in SEQ ID NO: 73. In some embodiments of the present disclosure, the guide sequence is the sequence as shown in SEQ ID NO: 73. In some embodiments, the guide sequence is engineered to hybridize with a target RNA.

In some embodiments of the present disclosure, the guide sequence has at least 80%, at least 85%, at least 90%, at least 95% or 100% sequence identity to the sequence as shown in SEQ ID NO: 216. In some embodiments of the present disclosure, the guide sequence has 100% sequence identity to the sequence as shown in SEQ ID NO: 216. In some embodiments of the present disclosure, the guide sequence is the sequence as shown in SEQ ID NO: 216. In some embodiments, the guide sequence is engineered to hybridize with a target RNA.

In some embodiments of the present disclosure, the guide sequence has at least 80%, at least 85%, at least 90%, at least 95% or 100% sequence identity to the sequence as shown in SEQ ID NO: 219. In some embodiments of the present disclosure, the guide sequence has 100% sequence identity to the sequence as shown in SEQ ID NO: 219. In some embodiments of the present disclosure, the guide sequence is the sequence as shown in SEQ ID NO: 219. In some embodiments, the guide sequence is engineered to hybridize with a target RNA.

In some embodiments of the present disclosure, the guide sequence has at least 80%, at least 85%, at least 90%, at least 95% or 100% sequence identity to the sequence as shown in SEQ ID NO: 220. In some embodiments of the present disclosure, the guide sequence has 100% sequence identity to the sequence as shown in SEQ ID NO: 220. In some embodiments of the present disclosure, the guide sequence is the sequence as shown in SEQ ID NO: 220. In some embodiments, the guide sequence is engineered to hybridize with a target RNA.

In some embodiments of the present disclosure, the guide sequence has at least 80%, at least 85%, at least 90%, at least 95% or 100% sequence identity to the sequence as shown in SEQ ID NO: 223. In some embodiments of the present disclosure, the guide sequence has 100% sequence identity to the sequence as shown in SEQ ID NO: 223. In some embodiments of the present disclosure, the guide sequence is the sequence as shown in SEQ ID NO: 223. In some embodiments, the guide sequence is engineered to hybridize with a target RNA.

In some embodiments of the present disclosure, the guide sequence has at least 80%, at least 85%, at least 90%, at least 95% or 100% sequence identity to the sequence as shown in SEQ ID NO: 227. In some embodiments of the present disclosure, the guide sequence has 100% sequence identity to the sequence as shown in SEQ ID NO: 227. In some embodiments of the present disclosure, the guide sequence is the sequence as shown in SEQ ID NO: 227. In some embodiments, the guide sequence is engineered to hybridize with a target RNA.

In some embodiments of the present disclosure, the guide sequence has at least 80% sequence identity to the sequence as shown in SEQ ID NO: 228. In some embodiments of the present disclosure, the guide sequence has at least 85%, at least 90%, at least 95% or 100% sequence identity to the sequence as shown in SEQ ID NO: 228. In some embodiments of the present disclosure, the guide sequence has 100% sequence identity to the sequence as shown in SEQ ID NO: 228. In some embodiments of the present disclosure, the guide sequence is the sequence as shown in SEQ ID NO: 228. In some embodiments, the guide sequence is engineered to hybridize with a target RNA.

In some embodiments of the present disclosure, the guide sequence has at least 80% sequence identity to the sequence as shown in SEQ ID NO: 229. In some embodiments of the present disclosure, the guide sequence has at least 85%, at least 90%, at least 95% or 100% sequence identity to the sequence as shown in SEQ ID NO: 229. In some embodiments of the present disclosure, the guide sequence has 100% sequence identity to the sequence as shown in SEQ ID NO: 229. In some embodiments of the present disclosure, the guide sequence is the sequence as shown in SEQ ID NO: 229. In some embodiments, the guide sequence is engineered to hybridize with a target RNA.

In some embodiments of the present disclosure, the guide sequence has at least 80% sequence identity to the sequence as shown in SEQ ID NO: 230. In some embodiments of the present disclosure, the guide sequence has at least 85%, at least 90%, at least 95% or 100% sequence identity to the sequence as shown in SEQ ID NO: 230. In some embodiments of the present disclosure, the guide sequence has 100% sequence identity to the sequence as shown in SEQ ID NO: 230. In some embodiments of the present disclosure, the guide sequence is the sequence as shown in SEQ ID NO: 230. In some embodiments, the guide sequence is engineered to hybridize with a target RNA.

gRNA-1 to gRNA-10 can target conserved VEGFA RNA exon regions in humans and mice, gRNA-11 to gRNA-32 can target human VEGFA RNA exon regions, and gRNA-33 to gRNA-45 can target regions near the splicing site of human VEGFARNA. For example, gRNA-36 can target the intron sequence immediately adjacent to the 5' end of exon 2 of VEGFA pre-mRNA, and gRNA-35 can target the splicing site at the 5' end of exon 2 of VEGFA pre-mRNA; gRNA-5-2, gRNA-5-3 and gRNA-5-5 can target exon 1 and exon 2 of mature VEGFA mRNA.

Table 1. Guide sequences of gRNA

| gRNA number | Guide sequence of gRNA | SEQ ID NO |
|---|---|---|
| gRNA-1 | GGTACTCCTGGAAGATGTCCACCAGGGTCT | 9 |
| gRNA-2 | CCACTGCGGCCCCCTCTCCTCTTCCTTC | 10 |
| gRNA-3 | GGCTGGAGCACTGTCTGCGCACA | 11 |
| gRNA-4 | AAGCTCATCTCTCCTATGTGCTGGC | 12 |
| gRNA-5 | TGGGTGCAGCCTGGGACCACTTGGCATGG | 13 |
| gRNA-6 | TCTTTCTTTGGTCTGCATTCACAT | 14 |
| gRNA-7 | GGCCTTGGTGAGGTTTGATCCGCAT | 15 |
| gRNA-8 | ACAAACAAATGCTTTCTCCGCTCTGA | 16 |
| gRNA-9 | GCAGGAACATTTACACGTCTGCGGATCT | 17 |
| gRNA-10 | AGTACGTTCGTTTAACTCAAGCTGCC | 18 |
| gRNA-11 | TGCACCCAAGACAGCAGAAAGTTCA | 40 |
| gRNA-12 | AAGGCTCCAATGCACCCAAGACAGC | 41 |
| gRNA-13 | TGGAGGTAGAGCAGCAAGGCAAGGC | 42 |
| gRNA-14 | CCCTCCTCCTTCTGCCATGGGTGCA | 43 |
| gRNA-15 | CATCCATGAACTTCACCACTTCGTG | 44 |
| gRNA-16 | GTAGCTGCGCTGATAGACATCCATG | 45 |
| gRNA-17 | ATGGCTTGAAGATGTACTCGATCTC | 46 |
| gRNA-18 | CATCAGGGGCACACAGGATGGCTTG | 47 |
| gRNA-19 | AGCAGCCCCCGCATCGCATCAGGGG | 48 |
| gRNA-20 | TTGCAGCAGCCCCCGCATCGCATCA | 49 |
| gRNA-21 | TCGTCATTGCAGCAGCCCCCGCATC | 50 |
| gRNA-22 | ACTCCAGGCCCTCGTCATTGCAGCA | 51 |
| gRNA-23 | CACACACTCCAGGCCCTCGTCATTG | 52 |
| gRNA-24 | TGGGCACACACTCCAGGCCCTCGTC | 53 |
| gRNA-25 | CTCAGTGGGCACACACTCCAGGCCC | 54 |

(continued)

| gRNA number | Guide sequence of gRNA | SEQ ID NO |
|---|---|---|
| gRNA-26 | TGCATGGTGATGTTGGACTCCTCAG | 55 |
| gRNA-27 | ATCTGCATGGTGATGTTGGACTCCT | 56 |
| gRNA-28 | GCATAATCTGCATGGTGATGTTGGA | 57 |
| gRNA-29 | TCCGCATAATCTGCATGGTGATGTT | 58 |
| gRNA-30 | TGTGCTGGCCTTGGTGAGGTTTGAT | 59 |
| gRNA-31 | TTCACATTTGTTGTGCTGTAGGAAG | 60 |
| gRNA-32 | GTCTGCATTCACATTTGTTGTGCTG | 61 |
| gRNA-33 | gggcacgaccgcttacCTTGGCATGG | 62 |
| gRNA-34 | gcgccagcagggcacgaccgcttac | 63 |
| gRNA-35 | TGCAGCCTGGGACCActgaggacaga | 64 |
| gRNA-36 | ctgaggacagaaagagagcaaggcat | 65 |
| gRNA-37 | CCActgaggacagaaagagagcaag | 66 |
| gRNA-38 | cccatccaacagccaggggggactcac | 67 |
| gRNA-39 | TGAACTTCACCActgcatgagaggcg | 68 |
| gRNA-40 | ctgcatgagaggcgatgacacatgag | 69 |
| gRNA-41 | caaagatgcccacCTGCATGGTGATG | 70 |
| gRNA-42 | ttgccccacttcccaaagatgcccac | 71 |
| gRNA-43 | GGTTTGATCCGCATAATctggaaagg | 72 |
| gRNA-44 | gactacatcctcacCTGCATTCACAT | 73 |
| gRNA-45 | taatgaatccgtgactacatcctcac | 74 |
| gRNA-46 | TCGTCATTGCAGCAGCCCCCGCATCGCATC | 75 |
| gRNA-47 | ATAGACATCCATGAACTTCACCACTTCGTG | 76 |
| gRNA-48 | AAGATGTCCACCAGGGTCTCGATTGGATGG | 77 |
| gRNA-49 | CGTCATTGCAGCAGCCCCCGCATCGCATCA | 78 |
| gRNA-50 | TAGACATCCATGAACTTCACCACTTCGTGA | 79 |
| gRNA-51 | TTTGATCCGCATAATCTGCATGGTGATGTT | 80 |
| gRNA-52 | AGACATCCATGAACTTCACCACTTCGTGAT | 81 |
| gRNA-53 | ACATCCATGAACTTCACCACTTCGTGATGA | 82 |
| gRNA-54 | CCCTCGTCATTGCAGCAGCCCCCGCATCGC | 83 |
| gRNA-55 | GCCCTCGTCATTGCAGCAGCCCCCGCATCG | 84 |
| gRNA-56 | ATGTTGGACTCCTCAGTGGGCACACACTCC | 85 |
| gRNA-57 | TGTTGGACTCCTCAGTGGGCACACACTCCA | 86 |
| gRNA-58 | CCAATGCACCCAAGACAGCAGAAAGTTCAT | 87 |
| gRNA-59 | TCCAATGCACCCAAGACAGCAGAAAGTTCA | 88 |
| gRNA-60 | CTCCAATGCACCCAAGACAGCAGAAAGTTC | 89 |
| gRNA-61 | CACACACTCCAGGCCCTCGTCATTGCAGCA | 90 |
| gRNA-62 | GCACACACTCCAGGCCCTCGTCATTGCAGC | 91 |
| gRNA-63 | CACTTCGTGATGATTCTGCCCTCCTCCTTC | 92 |
| gRNA-64 | CCACTTCGTGATGATTCTGCCCTCCTCCTT | 93 |

(continued)

| gRNA number | Guide sequence of gRNA | SEQ ID NO |
|---|---|---|
| gRNA-65 | CCTTGGTGAGGTTTGATCCGCATAATC | 94 |
| gRNA-66 | AAGATGTACTCGATCTCATCAGGGTAC | 95 |
| gRNA-67 | TTTGATCCGCATAATCTGCATGGTGAT | 96 |
| gRNA-68 | TCGTCATTGCAGCAGCCCCCGCATCGC | 97 |
| gRNA-69 | AAGATGTCCACCAGGGTCTCGATTGGA | 98 |
| gRNA-70 | ATGTTGGACTCCTCAGTGGGCACACAC | 99 |
| gRNA-71 | CGTCATTGCAGCAGCCCCCGCATCGCA | 100 |
| gRNA-72 | TAGACATCCATGAACTTCACCACTTCG | 101 |
| gRNA-73 | AGACATCCATGAACTTCACCACTTCGT | 102 |
| gRNA-74 | ACATCCATGAACTTCACCACTTCGTGA | 103 |
| gRNA-75 | GACATCCATGAACTTCACCACTTCGTG | 104 |
| gRNA-76 | GCATTCACATTTGTTGTGCTGTAGGAA | 105 |
| gRNA-77 | ATAGACATCCATGAACTTCACCACTTC | 106 |
| gRNA-78 | GCACACTCCAGGCCCTCGTCATTGC | 107 |
| gRNA-79 | CCCTCGTCATTGCAGCAGCCCCCGCAT | 108 |
| gRNA-80 | CCTTGGTGAGGTTTGATCCGCATAA | 109 |
| gRNA-81 | CACTTCGTGATGATTCTGCCCTCCTCC | 110 |
| gRNA-82 | CCAATGCACCCAAGACAGCAGAAAGTT | 111 |
| gRNA-83 | AGATGTACTCGATCTCATCAGGGTA | 112 |
| gRNA-84 | TCCAATGCACCCAAGACAGCAGAAAGT | 113 |
| gRNA-85 | TTTGATCCGCATAATCTGCATGGTG | 114 |
| gRNA-86 | TGGACTCCTCAGTGGGCACACACTCCA | 115 |
| gRNA-87 | AAGATGTCCACCAGGGTCTCGATTG | 116 |
| gRNA-88 | CGTCATTGCAGCAGCCCCCGCATCG | 117 |
| gRNA-89 | TCGTCATTGCAGCAGCCCCCGCATC | 118 |
| gRNA-90 | ACATCCATGAACTTCACCACTTCGT | 119 |
| gRNA-91 | CATCCATGAACTTCACCACTTCGTG | 120 |
| gRNA-92 | GCATTCACATTTGTTGTGCTGTAGG | 121 |
| gRNA-93 | ATAGACATCCATGAACTTCACCACT | 122 |
| gRNA-94 | GTTGGACTCCTCAGTGGGCACACAC | 123 |
| gRNA-95 | GACATCCATGAACTTCACCACTTCG | 124 |
| gRNA-96 | ATGTTGGACTCCTCAGTGGGCACAC | 125 |
| gRNA-97 | TAGACATCCATGAACTTCACCACTT | 126 |
| gRNA-98 | AGACATCCATGAACTTCACCACTTC | 127 |
| gRNA-99 | TGGACTCCTCAGTGGGCACACACTC | 128 |
| gRNA-100 | CCAATGCACCCAAGACAGCAGAAAG | 129 |
| gRNA-101 | TCCAATGCACCCAAGACAGCAGAAA | 130 |
| gRNA-102 | CCCTCGTCATTGCAGCAGCCCCCGC | 131 |
| gRNA-103 | GACATCCATGAACTTCACCACTTCGTGATG | 132 |

(continued)

| gRNA number | Guide sequence of gRNA | SEQ ID NO |
|---|---|---|
| gRNA-104 | GCATTCACATTTGTTGTGCTGTAGGAAGCT | 133 |
| gRNA-105 | CACCACTTCGTGATGATTCTGCCCTCCTCC | 134 |
| gRNA-106 | CCTTGGTGAGGTTTGATCCGCATAATCTGC | 135 |
| gRNA-107 | CAGGCCCTCGTCATTGCAGCAGCCCCCGCA | 136 |
| gRNA-108 | GCAAGGCTCCAATGCACCCAAGACAGCAGA | 137 |
| gRNA-109 | ACCACTTCGTGATGATTCTGCCCTCCTCCT | 138 |
| gRNA-110 | GCTCCAATGCACCCAAGACAGCAGAAAGTT | 139 |
| gRNA-111 | AAGATGTACTCGATCTCATCAGGGTACTCC | 140 |
| gRNA-112 | CCTCGTCATTGCAGCAGCCCCCGCATCGCA | 141 |
| gRNA-113 | GTTGGACTCCTCAGTGGGCACACACTCCAG | 142 |
| gRNA-114 | CGCATCGCATCAGGGGCACACAGGATGGCT | 143 |
| gRNA-115 | GTAGCTGCGCTGATAGACATCCATGAACTT | 144 |
| gRNA-116 | GAAGATGTCCACCAGGGTCTCGATTGGATG | 145 |
| gRNA-117 | AGGCTCCAATGCACCCAAGACAGCAGAAAG | 146 |
| gRNA-118 | GGCAAGGCTCCAATGCACCCAAGACAGCAG | 147 |
| gRNA-119 | GCCCTCGTCATTGCAGCAGCCCCCGCA | 148 |
| gRNA-120 | CGCATCGCATCAGGGGCACACAGGATG | 149 |
| gRNA-121 | CCACTTCGTGATGATTCTGCCCTCCTC | 150 |
| gRNA-122 | ACTTCGTGATGATTCTGCCCTCCTCCT | 151 |
| gRNA-123 | CTCCAATGCACCCAAGACAGCAGAAAG | 152 |
| gRNA-124 | CCGCATCGCATCAGGGGCACACAGGAT | 153 |
| gRNA-125 | CATCCATGAACTTCACCACTTCGTGAT | 154 |
| gRNA-126 | AGATGTACTCGATCTCATCAGGGTACT | 155 |
| gRNA-127 | CACACACTCCAGGCCCTCGTCATTGCA | 156 |
| gRNA-128 | TTGGACTCCTCAGTGGGCACACACTCC | 157 |
| gRNA-129 | TGTTGGACTCCTCAGTGGGCACACACT | 158 |
| gRNA-130 | GTAGCTGCGCTGATAGACATCCATGAA | 159 |
| gRNA-131 | GTTGGACTCCTCAGTGGGCACACACTC | 160 |
| gRNA-132 | CACCACTTCGTGATGATTCTGCCCTCC | 161 |
| gRNA-133 | TAGCTGCGCTGATAGACATCCATGAAC | 162 |
| gRNA-134 | GCTCCAATGCACCCAAGACAGCAGAAA | 163 |
| gRNA-135 | GGACTCCTCAGTGGGCACACACTCCAG | 164 |
| gRNA-136 | ATCTGCATGGTGATGTTGGACTCCTCA | 165 |
| gRNA-137 | AGTAGCTGCGCTGATAGACATCCATGA | 166 |
| gRNA-138 | ACCACTTCGTGATGATTCTGCCCTCCT | 167 |
| gRNA-139 | GAAGATGTCCACCAGGGTCTCGATTGG | 168 |
| gRNA-140 | AATGCACCCAAGACAGCAGAAAGTTCA | 169 |
| gRNA-141 | ATGCACCCAAGACAGCAGAAAGTTCAT | 170 |
| gRNA-142 | GCTTGAAGATGTACTCGATCTCATCAG | 171 |

(continued)

| gRNA number | Guide sequence of gRNA | SEQ ID NO |
|---|---|---|
| gRNA-143 | CTGCATTCACATTTGTTGTGCTGTAGG | 172 |
| gRNA-144 | TCTGCATGGTGATGTTGGACTCCTCAG | 173 |
| gRNA-145 | CAGGCCCTCGTCATTGCAGCAGCCCCC | 174 |
| gRNA-146 | CTTCGTGATGATTCTGCCCTCCTCCTT | 175 |
| gRNA-147 | CTGCATGGTGATGTTGGACTCCTCAGT | 176 |
| gRNA-148 | GCAAGGCTCCAATGCACCCAAGACAGC | 177 |
| gRNA-149 | GGCTCCAATGCACCCAAGACAGCAGAA | 178 |
| gRNA-150 | GCCCTCGTCATTGCAGCAGCCCCCG | 179 |
| gRNA-151 | GAAGATGTCCACCAGGGTCTCGATT | 180 |
| gRNA-152 | CGCATCGCATCAGGGGCACACAGGA | 181 |
| gRNA-153 | GCACACACTCCAGGCCCTCGTCATT | 182 |
| gRNA-154 | CACTTCGTGATGATTCTGCCCTCCT | 183 |
| gRNA-155 | CCACTTCGTGATGATTCTGCCCTCC | 184 |
| gRNA-156 | AAGATGTACTCGATCTCATCAGGGT | 185 |
| gRNA-157 | ATTTGTTGTGCTGTAGGAAGCTCAT | 186 |
| gRNA-158 | TTGGACTCCTCAGTGGGCACACACT | 187 |
| gRNA-159 | CACACACTCCAGGCCCTCGTCATTG | 188 |
| gRNA-160 | CTCCAATGCACCCAAGACAGCAGAA | 189 |
| gRNA-161 | TGTTGGACTCCTCAGTGGGCACACA | 190 |
| gRNA-162 | CTGCATTCACATTTGTTGTGCTGTA | 191 |
| gRNA-163 | ACTTCGTGATGATTCTGCCCTCCTC | 192 |
| gRNA-164 | GTAGCTGCGCTGATAGACATCCATG | 193 |
| gRNA-165 | AATGCACCCAAGACAGCAGAAAGTT | 194 |
| gRNA-166 | CACCACTTCGTGATGATTCTGCCCT | 195 |
| gRNA-167 | CCTCGTCATTGCAGCAGCCCCCGCA | 196 |
| gRNA-168 | CAATGCACCCAAGACAGCAGAAAGT | 197 |
| gRNA-169 | GCATCGCATCAGGGGCACACAGGAT | 198 |
| gRNA-170 | CTGCATGGTGATGTTGGACTCCTCA | 199 |
| gRNA-171 | GGACTCCTCAGTGGGCACACACTCC | 200 |
| gRNA-172 | TAGCTGCGCTGATAGACATCCATGA | 201 |
| gRNA-173 | ACCACTTCGTGATGATTCTGCCCTC | 202 |
| gRNA-174 | GCTCCAATGCACCCAAGACAGCAGA | 203 |
| gRNA-175 | AGTAGCTGCGCTGATAGACATCCAT | 204 |
| gRNA-176 | CATGGTGATGTTGGACTCCTCAGTG | 205 |
| gRNA-177 | CTTCGTGATGATTCTGCCCTCCTCC | 206 |
| gRNA-178 | ATCTGCATGGTGATGTTGGACTCCT | 207 |
| gRNA-179 | CAGGCCCTCGTCATTGCAGCAGCCC | 208 |
| gRNA-180 | TCTGCATGGTGATGTTGGACTCCTC | 209 |
| gRNA-181 | TGCATGGTGATGTTGGACTCCTCAG | 210 |

(continued)

| gRNA number | Guide sequence of gRNA | SEQ ID NO |
|---|---|---|
| gRNA-182 | TAGAGCAGCAAGGCAAGGCTCCAAT | 211 |
| gRNA-183 | ATGCACCCAAGACAGCAGAAAGTTC | 212 |
| gRNA-184 | CCGCATCGCATCAGGGGCACACAGG | 213 |
| gRNA-185 | GCTGGCCTTGGTGAGGTTTGATCCG | 214 |
| gRNA-186 | GTGCTGGCCTTGGTGAGGTTTGATC | 215 |
| gRNA-187 | ATGTGCTGGCCTTGGTGAGGTTTGA | 216 |
| gRNA-188 | CTATGTGCTGGCCTTGGTGAGGTTT | 217 |
| gRNA-189 | TCCTATGTGCTGGCCTTGGTGAGGT | 218 |
| gRNA-190 | TCTCCTATGTGCTGGCCTTGGTGAG | 219 |
| gRNA-191 | TCTCTCCTATGTGCTGGCCTTGGTG | 220 |
| gRNA-192 | CATCTCTCCTATGTGCTGGCCTTGG | 221 |
| gRNA-193 | TCATCTCTCCTATGTGCTGGCCTTG | 222 |
| gRNA-194 | CTCATCTCTCCTATGTGCTGGCCTT | 223 |
| gRNA-195 | GCTCATCTCTCCTATGTGCTGGCCT | 224 |
| gRNA-196 | TGCTGGCCTTGGTGAGGTTTGATCC | 225 |
| gRNA-197 | TATGTGCTGGCCTTGGTGAGGTTTG | 226 |
| gRNA-5-2 | CAGCCTGGGACCACTTGGCATGGTGG | 227 |
| gRNA-5-3 | GCAGCCTGGGACCACTTGGCATGGT | 228 |
| gRNA-5-4 | TGGGTGCAGCCTGGGACCACT | 229 |
| gRNA-5-5 | AGCCTGGGACCACTTGGCATGGTGGAGG | 230 |

In some embodiments of the present disclosure, the guide sequence is engineered to hybridize with a target RNA. In some embodiments of the present disclosure, the guide sequence is engineered to hybridize with the target RNA with no more than 6, no more than 5, no more than 4, no more than 3, no more than 2, or no more than 1 nucleotide mismatch.

In some embodiments of the present disclosure, the guide RNA is capable of forming a complex with an RNA-guided nuclease and guiding the sequence-specific binding of the complex to a target RNA.

In some embodiments of the present disclosure, the guide RNA is capable of forming a complex with an RNA-guided nuclease and guiding the complex to bind to and cleave a target RNA.

In some embodiments of the present disclosure, the RNA-guided nuclease is a Cas protein.

In some embodiments of the present disclosure, the guide RNA is capable of forming a CRISPR complex with the Cas protein and guiding the sequence-specific binding of the CRISPR complex to a target RNA.

In some embodiments of the present disclosure, the guide RNA is capable of forming a CRISPR complex with the Cas protein and guiding the CRISPR complex to bind to and cleave a target RNA.

In some embodiments of the present disclosure, the RNA-guided nuclease is a Cas13 protein.

In some embodiments of the present disclosure, the RNA-guided nuclease is a Cas13a protein, a Cas13b protein, a Cas13c protein, or a Cas13d protein.

In some embodiments of the present disclosure, the RNA-guided nuclease is a Cas13d protein.

In some embodiments of the present disclosure, the Cas protein comprises an amino acid sequence having ≥50% sequence identity to the sequence as shown in any one of SEQ ID NOs: 1-3. In some embodiments of the present disclosure, the Cas protein comprises an amino acid sequence having ≥60%, ≥65%, ≥70%, ≥75%, ≥80%, ≥85%, ≥90%, ≥95%, ≥96%, ≥97%, ≥98%, ≥99%, ≥99.1%, ≥99.2%, ≥99.3%, ≥99.4%, ≥99.5%, ≥99.6%, ≥99.7% or ≥99.8% sequence identity to the sequence as shown in any one of SEQ ID NOs: 1-3.

In some embodiments of the present disclosure, the Cas protein comprises a sequence having 100% sequence identity to the sequence as shown in any one of SEQ ID NOs: 1-3.

In some embodiments of the present disclosure, the Cas protein comprises the sequence as shown in any one of SEQ ID NOs: 1-3.

In some embodiments of the present disclosure, the Cas protein comprises a sequence having ≥50% sequence identity to the sequence as shown in SEQ ID NO: 3. In some embodiments of the present disclosure, the Cas protein comprises a sequence having ≥60%, ≥65%, ≥70%, ≥75%, ≥80%, ≥85%, ≥90%, ≥95%, ≥96%, ≥97%, ≥98%, ≥99%, ≥99.1%, ≥99.2%, ≥99.3%, ≥99.4%, ≥99.5%, ≥99.6%, ≥99.7% or ≥99.8% sequence identity to the sequence as shown in SEQ ID NO: 3.

In some embodiments of the present disclosure, the Cas protein comprises a sequence having 100% sequence identity to the sequence as shown in SEQ ID NO: 3.

In some embodiments of the present disclosure, the Cas protein comprises the sequence as shown in SEQ ID NO: 3.

In some embodiments of the present disclosure, the Cas protein comprises a sequence having ≥50% sequence identity to CasRx. In some embodiments of the present disclosure, the Cas protein comprises a sequence having ≥60%, ≥65%, ≥70%, ≥75%, ≥80%, ≥85%, ≥90%, ≥95%, ≥96%, ≥97%, ≥98%, ≥99% or ≥99.5% sequence identity to the sequence as shown in any one of SEQ ID NOs: 1-3.

In some embodiments of the present disclosure, the Cas protein comprises a sequence having 100% sequence identity to CasRx.

In some embodiments of the present disclosure, the Cas protein comprises the sequence of CasRx.

In some embodiments of the present disclosure, the RNA-guided nuclease comprises a nuclease portion and a fused homologous or heterologous domain.

In some embodiments of the present disclosure, the RNA-guided nuclease comprises any one or more of the following: a subcellular localization signal, a deaminase domain, a translation activation domain, a translation repression domain, an RNA methylation domain, an RNA demethylation domain, a nuclease domain, a splicing factor domain, a reporter domain, a reporter tag, an affinity domain and an affinity tag.

In some embodiments of the present disclosure, the Cas protein comprises any one or more of the following: a subcellular localization signal, a deaminase domain, a translation activation domain, a translation repression domain, an RNA methylation domain, an RNA demethylation domain, a nuclease domain, a splicing factor domain, a reporter domain, a reporter tag, an affinity domain and an affinity tag.

In some embodiments of the present disclosure, the subcellular localization signal is selected from a nuclear localization signal and a nuclear export signal.

In some embodiments of the present disclosure, the target RNA is VEGFA RNA. In some embodiments of the present disclosure, the target RNA is human VEGFA RNA. In some embodiments of the present disclosure, the target RNA is VEGFA pre-mRNA. In some embodiments of the present disclosure, the target RNA is mature VEGFA mRNA.

In some embodiments of the present disclosure, the guide RNA comprises a guide sequence and a direct repeat sequence. In some embodiments of the present disclosure, the guide RNA comprises a guide sequence and a direct repeat sequence, and the direct repeat sequence interacts with an RNA-guided nuclease.

In some embodiments of the present disclosure, the complex reduces the level of the target RNA in an animal (such as in a human).

In some embodiments of the present disclosure, the complex reduces the level of the target RNA in a cell, such as the level of the target RNA in a cell expressing the target RNA.

In some embodiments of the present disclosure, the complex reduces the level of the target RNA in the cell by at least 5%, at least 10%, at least 15%, at least 20%, at least 25%, at least 30%, at least 35%, at least 40%, at least 50%, at least 60%, at least 70%, at least 80%, at least 90%, at least 95%, at least 96%, at least 97%, at least 98% or at least 99%. The reduction in the level of the target RNA can be tested using conventional methods in the art; including but not limited to the qPCR method described in the examples, and untreated cells or cells treated with a gene editing system targeting a non-mammalian genome can be used as negative controls to calculate the target RNA knockdown level of the experimental group compared with the negative control.

In some embodiments of the present disclosure, the number of off-target genes when the complex binds to and cleaves the target RNA is less than 40, less than 35, less than 30, less than 25, less than 20, less than 19, less than 18, less than 17, less than 16, less than 15, less than 14, less than 13, less than 12, less than 11, less than 10, less than 9, less than 8, less than 7, less than 6, less than 5, less than 4, less than 3, less than 2 or less than 1. The number of off-target genes can be determined by conventional methods in the art. In some embodiments, the number of off-target genes is determined by taking the intersection of the set of differentially expressed genes determined by RNA sequencing with the set of off-target genes predicted by the program. Non-limiting examples, such as the method predicted by the program, include using the EMBOSS-water program to predict in the whole genome and whole cDNA sequence of the target species (Homo sapiens/Mus musculus), setting the parameters to gap_extend=0.5 & gap_extend=10, using the sense and antisense strands of the gRNA guide sequence for comparison, filtering the prediction results, and obtaining predicted potential target genes (including on-target genes and off-target genes).

In some embodiments of the present disclosure, the complex reduces the level of the target RNA-encoded protein in an animal (such as in a human).

In some embodiments of the present disclosure, the complex reduces the level of the target RNA-encoded protein in a cell. In some embodiments of the present disclosure, the target RNA-encoded protein is a VEGFA protein. In some embodiments of the present disclosure, the complex reduces the level of the VEGFA protein in a cell.

In some embodiments of the present disclosure, the complex reduces the level of VEGFA protein in the cell by at least 5%, at least 10%, at least 15%, at least 20%, at least 25%, at least 30%, at least 35%, at least 40%, at least 50%, at least 60%, at least 70%, at least 80%, at least 90%, at least 95%, at least 96%, at least 97%, at least 98% or at least 99%. The reduction in the level of the target RNA-encoded protein can be tested using conventional methods in the art; including but not limited to ELISA and Western Blotting, and untreated cells or cells treated with a gene editing system targeting a non-mammalian genome can be used as negative controls to calculate the knockdown level of the target RNA-encoded protein in the experimental group compared with the negative control.

The second aspect of the present disclosure provides an isolated nucleic acid encoding the guide RNA according to the present disclosure.

The third aspect of the present disclosure provides a vector comprising a polynucleotide sequence encoding the guide RNA according to the present disclosure, and a regulatory sequence for regulating the expression of the guide RNA.

In some embodiments of the present disclosure, the vector is an adeno-associated virus vector.

In some embodiments of the present disclosure, the regulatory sequence is promoter and/or enhancer sequences. In some embodiments of the present disclosure, the regulatory sequence is a promoter sequence. In some embodiments of the present disclosure, the regulatory sequence is promoter and enhancer sequences.

In some embodiments of the present disclosure, the regulatory sequence is selected from: a native promoter, a ubiquitous promoter, a CAG promoter, a CBA promoter, a CBh promoter, a CMV promoter and a tissue-specific promoter.

In some embodiments of the present disclosure, the regulatory sequence is a promoter, and the promoter is selected from: an RPE65 promoter, a VDM2 promoter, an Mlc1 promoter, a GFAP promoter and an ICAM-2 promoter.

In some embodiments of the present disclosure, the regulatory sequence is a U6 promoter.

In some embodiments of the present disclosure, the promoter is an eye-specific promoter.

In some embodiments of the present disclosure, the promoter is a CBh promoter.

In some embodiments of the present disclosure, the regulatory sequence comprises an HRE enhancer element.

In some embodiments of the present disclosure, the regulatory sequence comprises an NRS element and an HRE enhancer element in tandem.

The fourth aspect of the present disclosure provides a gene editing system, comprising:

(a) the guide RNA according to the present disclosure or a polynucleotide sequence encoding the guide RNA; and
(b) an RNA-guided nuclease or a polynucleotide sequence encoding the RNA-guided nuclease;

the guide RNA is capable of forming a complex with the nuclease and guiding the sequence-specific binding of the complex to a target RNA.

In some embodiments of the present disclosure, the guide RNA is capable of forming a complex with the nuclease and guiding the complex to bind to and cleave a target RNA.

In some embodiments of the present disclosure, the guide sequence is engineered to hybridize with a target RNA. In some embodiments of the present disclosure, the guide sequence is engineered to hybridize with the target RNA with no more than 6, no more than 5, no more than 4, no more than 3, no more than 2, or no more than 1 nucleotide mismatch.

In some embodiments of the present disclosure, the polynucleotide sequence encoding the RNA-guided nuclease is linked to a regulatory sequence, and the regulatory sequence is used to regulate the expression of the RNA-guided nuclease.

In some embodiments of the present disclosure, the polynucleotide sequence encoding the guide RNA is linked to a regulatory sequence, and the regulatory sequence is used to regulate the expression of the guide RNA.

In some embodiments of the present disclosure, the regulatory sequence that regulates the expression of the RNA-guided nuclease is the same as or different from the regulatory sequence that regulates the expression of the guide RNA.

In some embodiments of the present disclosure, the regulatory sequence is promoter and/or enhancer sequences. In some embodiments of the present disclosure, the regulatory sequence is a promoter sequence. In some embodiments of the present disclosure, the regulatory sequence is promoter and enhancer sequences.

In some embodiments of the present disclosure, the regulatory sequence is selected from: a native promoter, a ubiquitous promoter, a CAG promoter, a CBA promoter, a CBh promoter, a CMV promoter and a tissue-specific promoter.

In some embodiments of the present disclosure, the regulatory sequence is a promoter, and the promoter is selected from: an RPE65 promoter, a VDM2 promoter, an Mlc1 promoter, a GFAP promoter and an ICAM-2 promoter.

In some embodiments of the present disclosure, the regulatory sequence is a U6 promoter.

In some embodiments of the present disclosure, the promoter is an eye-specific promoter.

In some embodiments of the present disclosure, the promoter is a CBh promoter.

In some embodiments of the present disclosure, the regulatory sequence comprises an HRE enhancer element (hypoxia response element). In some embodiments, the regulatory sequence comprises at least 2, at least 3, at least 4, at least 5, at least 6, at least 7, at least 8 or at least 9 HRE enhancer elements in tandem. In some embodiments, the regulatory sequence comprises 2, 3, 4, 5, 6, 7, 8 or 9 HRE enhancer elements in tandem.

In some embodiments of the present disclosure, the regulatory sequence comprises an NRS element (neuron restrictive silencer) from the human synapsin gene. In some embodiments, the regulatory sequence comprises an NRS element and an HRE enhancer element. In some embodiments, the regulatory sequence comprises an NRS element and an HRE enhancer element in tandem. To enhance the regulatory effect, the NRS element and HRE enhancer element in tandem can be repeated multiple times. In some embodiments, the regulatory sequence comprises an NRS element and an HRE enhancer element in tandem repeated at least 2 times, at least 3 times, at least 4 times, at least 5 times or at least 6 times. In some embodiments, the regulatory sequence comprises an NRS element and an HRE enhancer element in tandem repeated 2 times, 3 times, 4 times, 5 times or 6 times.

In some embodiments of the present disclosure, the HRE enhancer element sequence is TGTCACGTCCTG CACGACGTA (SEQ ID NO: 233) or a reverse complement sequence thereof.

In some embodiments of the present disclosure, the NRS element sequence is TTCAGCACCGCGGACAGT GCC (SEQ ID NO: 234) or a reverse complement sequence thereof.

In some embodiments of the present disclosure, the regulatory sequence comprises repeated TGTCACGTC CTGCACGACGTA (SEQ ID NO: 233) or a reverse complement sequence thereof.

In some embodiments of the present disclosure, the regulatory sequence comprises TTCAGCACCGCGGA CAGTGCCTGTCACGTCCTGCACGACGTA (SEQ ID NO: 235) in tandem or a reverse complement sequence thereof.

In some embodiments of the present disclosure, the gene editing system is a CRISPR-Cas system.

In some embodiments of the present disclosure, the RNA-guided nuclease is a Cas protein.

In some embodiments of the present disclosure, the guide RNA is capable of forming a CRISPR complex with the Cas protein and guiding the sequence-specific binding of the CRISPR complex to a target RNA.

In some embodiments of the present disclosure, the guide RNA is capable of forming a CRISPR complex with the Cas protein and guiding the CRISPR complex to bind to and cleave a target RNA.

In some embodiments of the present disclosure, the RNA-guided nuclease is a Cas13 protein.

In some embodiments of the present disclosure, the RNA-guided nuclease is a Cas13a protein, a Cas13b protein, a Cas13c protein or a Cas13d protein.

In some embodiments of the present disclosure, the RNA-guided nuclease is a Cas13d protein.

In some embodiments of the present disclosure, the Cas protein comprises an amino acid sequence having ≥50% sequence identity to the sequence as shown in any one of SEQ ID NOs: 1-3. In some embodiments of the present disclosure, the Cas protein comprises an amino acid sequence having ≥60%, ≥65%, ≥70%, ≥75%, ≥80%, ≥85%, ≥90%, ≥95%, ≥96%, ≥97%, ≥98%, ≥99%, ≥99.1%, ≥99.2%, ≥99.3%, ≥99.4%, ≥99.5%, ≥99.6%, ≥99.7% or ≥99.8% sequence identity to the sequence as shown in any one of SEQ ID NOs: 1-3.

In some embodiments of the present disclosure, the Cas protein comprises a sequence having 100% sequence identity to the sequence as shown in any one of SEQ ID NOs: 1-3.

In some embodiments of the present disclosure, the Cas protein comprises the sequence as shown in any one of SEQ ID NOs: 1-3.

In some embodiments of the present disclosure, the Cas protein comprises a sequence having ≥50% sequence identity to the sequence as shown in SEQ ID NO: 3. In some embodiments of the present disclosure, the Cas protein comprises a sequence having ≥60%, ≥65%, ≥70%, ≥75%, ≥80%, ≥85%, ≥90%, ≥95%, ≥96%, ≥97%, ≥98%, ≥99%, ≥99.1%, ≥99.2%, ≥99.3%, ≥99.4%, ≥99.5%, ≥99.6%, ≥99.7% or ≥99.8% sequence identity to the sequence as shown in SEQ ID NO: 3.

In some embodiments of the present disclosure, the Cas protein comprises a sequence having 100% sequence identity to the sequence as shown in SEQ ID NO: 3.

In some embodiments of the present disclosure, the Cas protein comprises the sequence as shown in SEQ ID NO: 3.

In some embodiments of the present disclosure, the Cas protein comprises a sequence having ≥50% sequence identity to CasRx. In some embodiments of the present disclosure, the Cas protein comprises a sequence having ≥60%, ≥65%, ≥70%, ≥75%, ≥80%, ≥85%, ≥90%, ≥95%, ≥96%, ≥97%, ≥98%, ≥99% or ≥99.5% sequence identity to the sequence as shown in any one of SEQ ID NOs: 1-3.

In some embodiments of the present disclosure, the Cas protein comprises a sequence having 100% sequence identity to CasRx.

In some embodiments of the present disclosure, the Cas protein comprises the sequence of CasRx.

In some embodiments of the present disclosure, the gene editing system is a CRISPR-Cas system, comprising:

(a) the guide RNA according to the present disclosure or a polynucleotide sequence encoding the guide RNA; and

(b) a Cas protein or a polynucleotide sequence encoding the Cas13 protein;

the guide RNA is capable of forming a complex with the Cas13 protein and guiding the sequence-specific binding of the complex to a target RNA.

In some embodiments of the present disclosure, the gene editing system comprises:

(a) the guide RNA according to the present disclosure or a polynucleotide sequence encoding the guide RNA; and

(b) a Cas13 protein or a polynucleotide sequence encoding the Cas13 protein;

the guide RNA is capable of forming a complex with the Cas13 protein and guiding the complex to bind to and cleave a target RNA.

In some embodiments of the present disclosure, the polynucleotide sequence encoding the guide RNA is linked to a first regulatory sequence, and the first regulatory sequence is used to regulate the expression of the guide RNA; the polynucleotide sequence encoding the RNA-guided nuclease is linked to a second regulatory sequence, and the second regulatory sequence is used to regulate the expression of the RNA-guided nuclease.

In some embodiments of the present disclosure, the RNA-guided nuclease comprises a nuclease portion and a fused homologous or heterologous domain.

In some embodiments of the present disclosure, the RNA-guided nuclease comprises any one or more of the following: a subcellular localization signal, a deaminase domain, a translation activation domain, a translation repression domain, an RNA methylation domain, an RNA demethylation domain, a nuclease domain, a splicing factor domain, a reporter domain, a reporter tag, an affinity domain and an affinity tag.

In some embodiments of the present disclosure, the Cas protein comprises any one or more of the following: a subcellular localization signal, a deaminase domain, a translation activation domain, a translation repression domain, an RNA methylation domain, an RNA demethylation domain, a nuclease domain, a splicing factor domain, a reporter domain, a reporter tag, an affinity domain and an affinity tag.

In some embodiments of the present disclosure, the subcellular localization signal is selected from a nuclear localization signal and a nuclear export signal.

The gene editing system described herein can be introduced into a cell (or a cell-free system) in a variety of non-limiting ways: (i) as an mRNA encoding an RNA-guided nuclease and a guide RNA, (ii) as part of a single vector or plasmid, or divided into multiple vectors or plasmids, (iii) as a separate RNA-guided nuclease and a guide RNA, or (iv) as an RNP complex of an RNA-guided nuclease and a guide RNA.

In some embodiments of the present disclosure, the target RNA is VEGFA RNA. In some embodiments of the present disclosure, the target RNA is VEGFA pre-mRNA. In some embodiments of the present disclosure, the target RNA is mature VEGFA mRNA.

In some embodiments of the present disclosure, the complex reduces the level of the target RNA in an animal (such as in a human).

In some embodiments of the present disclosure, the complex reduces the level of the target RNA in a cell, such as the level of the target RNA in a cell expressing the target RNA.

In some embodiments of the present disclosure, the complex reduces the level of the target RNA in the cell by at least 5%, at least 10%, at least 15%, at least 20%, at least 25%, at least 30%, at least 35%, at least 40%, at least 50%, at least 60%, at least 70%, at least 80%, at least 90%, at least 95%, at least 96%, at least 97%, at least 98% or at least 99%. That is, the editing efficiency of the gene editing system is at least 5%, at least 10%, at least 15%, at least 20%, at least 25%, at least 30%, at least 35%, at least 40%, at least 50%, at least 60%, at least 70%, at least 80%, at least 90%, at least 95%, at least 96%, at least 97%, at least 98% or at least 99%. The reduction in the level of the target RNA can be tested using conventional methods in the art; including but not limited to the qPCR method described in the examples, and untreated cells or cells treated with a gene editing system targeting a non-mammalian genome can be used as negative controls to calculate the target RNA knockdown level of the experimental group compared with the negative control.

In some embodiments of the present disclosure, the number of off-target genes when the complex binds to and cleaves the target RNA is less than 40, less than 35, less than 30, less than 25, less than 20, less than 19, less than 18, less than 17, less than 16, less than 15, less than 14, less than 13, less than 12, less than 11, less than 10, less than 9, less than 8, less than 7, less than 6, less than 5, less than 4, less than 3, less than 2 or less than 1. The number of off-target genes can be determined by conventional methods in the art. In some embodiments, the number of off-target genes is determined by taking the intersection of the set of differentially expressed genes determined by RNA sequencing with the set of off-target genes predicted by the program. Non-limiting examples, such as the method predicted by the program, include using the EMBOSS-water program to predict in the whole genome and whole cDNA sequence of the target species (Homo sapiens/Mus musculus), setting the parameters to gap _extend=0.5 & gap_extend=10, using the sense and antisense strands of the gRNA guide sequence for comparison, filtering the prediction results, and obtaining predicted potential target genes (including on-target genes and off-target genes).

In some embodiments of the present disclosure, the complex reduces the level of the target RNA-encoded protein in an animal (such as in a human).

In some embodiments of the present disclosure, the complex reduces the level of the target RNA-encoded protein in a cell. In some embodiments of the present disclosure, the target RNA-encoded protein is a VEGFA protein. In some embodiments of the present disclosure, the complex reduces the level of the VEGFA protein in a cell.

In some embodiments of the present disclosure, the complex reduces the level of VEGFA protein in the cell by at least 5%, at least 10%, at least 15%, at least 20%, at least 25%, at least 30%, at least 35%, at least 40%, at least 50%, at least 60%, at least 70%, at least 80%, at least 90%, at least 95%, at least 96%, at least 97%, at least 98% or at least 99%. The reduction in the level of the target RNA-encoded protein can be tested using conventional methods in the art; including but not limited to ELISA and Western Blotting, and untreated cells or cells treated with a gene editing system targeting a non-mammalian genome can be used as negative controls to calculate the knockdown level of the target RNA-encoded protein in the experimental group compared with the negative control.

The fifth aspect of the present disclosure provides a vector system comprising the gene editing system according to the present disclosure, wherein the vector system comprises a polynucleotide sequence encoding a guide RNA and a first regulatory sequence for regulating the expression of the guide RNA; and a polynucleotide sequence encoding an RNA-guided nuclease and a second regulatory sequence for regulating the expression of the RNA-guided nuclease.

In some embodiments of the present disclosure, the vector system comprises one or more vectors.

In some embodiments of the present disclosure, the vector system comprises only one vector.

In some embodiments of the present disclosure, the vector system comprises more than one vector, wherein the polynucleotide sequence encoding the guide RNA and the first regulatory sequence regulating the expression of the guide RNA are located on a first vector, and the polynucleotide sequence encoding the RNA-guided nuclease and the second regulatory sequence regulating the expression of the RNA-guided nuclease are located on a second vector.

In some embodiments of the present disclosure, the first regulatory sequence and/or the second regulatory sequence are promoter and/or enhancer sequences. In some embodiments of the present disclosure, the first regulatory sequence or the second regulatory sequence is a promoter sequence. In some embodiments of the present disclosure, the first regulatory sequence and the second regulatory sequence are promoter and enhancer sequences.

The sixth aspect of the present disclosure provides an adeno-associated virus vector comprising the gene editing system according to the present disclosure, wherein the adeno-associated virus vector comprises a DNA encoding the RNA-guided nuclease and guide RNA according to the present disclosure.

The seventh aspect of the present disclosure provides a lipid nanoparticle comprising the gene editing system according to the present disclosure, wherein the lipid nanoparticle comprises the guide RNA according to the present disclosure and an mRNA encoding the RNA-guided nuclease according to the present disclosure.

The eighth aspect of the present disclosure provides a lentiviral vector comprising the gene editing system according to the present disclosure, wherein the lentiviral vector comprises the guide RNA according to the present disclosure and an mRNA encoding the RNA-guided nuclease according to the present disclosure; optionally, the lentiviral vector is pseudotyped with an envelope protein; optionally, the mRNA encoding the RNA-guided nuclease is linked to an aptamer sequence.

The ninth aspect of the present disclosure provides a ribonucleoprotein complex comprising the gene editing system according to the present disclosure, wherein the ribonucleoprotein complex is formed by the guide RNA and the RNA-guided nuclease according to the present disclosure.

The tenth aspect of the present disclosure provides a virus-like particle comprising the gene editing system according to the present disclosure, wherein the virus-like particle comprises the ribonucleoprotein complex formed by the guide RNA and the RNA-guided nuclease according to the present disclosure; optionally, the RNA-guided nuclease is fused to a gag protein.

The eleventh aspect of the present disclosure provides a eukaryotic cell comprising the gene editing system according to the present disclosure; optionally, the eukaryotic cell is a mammalian cell.

The twelfth aspect of the present disclosure provides a pharmaceutical composition comprising the gene editing system according to the present disclosure, the vector system according to the present disclosure, the adeno-associated virus vector according to the present disclosure, the lipid nanoparticle according to the present disclosure, the lentiviral vector according to the present disclosure, the ribonucleoprotein complex according to the present disclosure, the virus-like particle according to the present disclosure, or the eukaryotic cell according to the present disclosure.

In some embodiments of the present disclosure, the pharmaceutical composition further comprises a pharmaceutically acceptable excipient.

The thirteenth aspect of the present disclosure provides a use of the guide RNA according to the present disclosure, the isolated nucleic acid according to the present disclosure, the vector according to the present disclosure, the gene editing system according to the present disclosure, the vector system according to the present disclosure, the adeno-associated virus vector according to the present disclosure, the lipid nanoparticle according to the present disclosure, the lentiviral vector according to the present disclosure, the ribonucleoprotein complex according to the present disclosure, the eukaryotic cell according to the present disclosure, the pharmaceutical composition according to the present disclosure, and/or the virus-like particle according to the present disclosure in any one of the following or in the manufacture of a reagent to achieve any one of the following solutions:

cleaving or nicking one or more target RNA molecules, activating or upregulating one or more target RNA molecules, activating or repressing the translation of one or more target RNA molecules, inactivating one or more target RNA molecules, visualizing, labeling or detecting one or more target RNA molecules, binding to one or more target RNA molecules, transporting one or more target RNA molecules, and masking one or more target RNA molecules.

In some embodiments of the present disclosure, the present disclosure provides a use of the guide RNA according to the present disclosure, the isolated nucleic acid according to the present disclosure, the vector according to the present disclosure, the gene editing system according to the present disclosure, the vector system according to the present disclosure, the adeno-associated virus vector according to the present disclosure, the lipid nanoparticle according to the present disclosure, the lentiviral vector according to the present disclosure, the ribonucleoprotein complex according to the present disclosure, and the virus-like particle according to the present disclosure in any one of the following or in the manufacture of a reagent to achieve any one of the following solutions:

cleaving one or more target RNA molecules and binding to one or more target RNA molecules.

In some embodiments of the present disclosure, the target RNA is VEGFA RNA.

For example, it can be understood by those skilled in the art that the RNA-guided nuclease can be fused or associated with one or more homologous or heterologous domains (*e.g.*, through fusion proteins and linker peptides). For example, a Cas13 mutant that completely or partially loses nuclease activity is fused with a homologous or heterologous domain. These homologous or heterologous domains can have various activities, such as methylase activity, demethylase activity, deaminase activity, translation activation activity, translation repression activity, RNA cleavage activity, nucleic acid binding activity, base editing activity, and switching activity (such as light induction). The homologous or heterologous domains include, but are not limited to, localization signals (*e.g.*, nuclear localization signal NLS or nuclear export signal NES), labels or detection labels (*e.g.*, fluorescent dyes such as FITC or DAPI), targeting moieties, antigenic determinant tags (*e.g.*, Hismyc, V5, FLAG, HA, VSV-G or Trx), deaminases or deamination domains (*e.g.*, ADAR1, ADAR2, APOBEC, AID or TAD), methylases, demethylases, ssRNA cleavage activity domains, dsRNA cleavage activity domains, DNA or RNA ligases, or any combination thereof. For example, the Cas13 protein can be fused with a deaminase and combined with a gRNA to target the target RNA to achieve single-base editing of the target RNA molecule. For example, the homologous or heterologous domain can be a detectable label. When the CRISPR-CAS complex contacts or binds to the target nucleic acid, the conjugate containing the Cas13 nuclease cleaves or modifies the target nucleic acid, and the presence of the target nucleic acid in the sample to be tested is analyzed by observing the presence of the detectable label. The detectable label is such as a fluorescent group, a chromogenic agent, an imaging agent or a radioactive isotope.

The fourteenth aspect of the present disclosure provides a method for diagnosing, treating or preventing a disease or disorder associated with a target RNA, comprising administering an effective amount of the guide RNA according to the present disclosure, the isolated nucleic acid according to the present disclosure, the vector according to the present disclosure, the gene editing system according to the present disclosure, the vector system according to the present disclosure, the adeno-associated virus vector according to the present disclosure, the lipid nanoparticle according to the present disclosure, the lentiviral vector according to the present disclosure, the ribonucleoprotein complex according to the present disclosure, the virus-like particle according to the present disclosure, or the pharmaceutical composition according to the present disclosure to a sample of a subject in need thereof or to a subject in need thereof.

In some embodiments of the present disclosure, the disease or disorder associated with the target RNA refers to a disease or disorder caused by abnormal expression of the target RNA.

In some embodiments of the present disclosure, the disease or disorder associated with the target RNA includes but is not limited to age related macular degeneration.

In some embodiments of the present disclosure, the disease or disorder associated with the target RNA includes but is not limited to dry age related macular degeneration. In some embodiments of the present disclosure, the disease or disorder associated with the target RNA includes but is not limited to wet age related macular degeneration.

In some embodiments of the present disclosure, the target RNA is VEGFA RNA.

The fifteenth aspect of the present disclosure provides a use of the guide RNA according to the present disclosure, the isolated nucleic acid according to the present disclosure, the vector according to the present disclosure, the gene editing system according to the present disclosure, the vector system according to the present disclosure, the adeno-associated virus vector according to the present disclosure, the lipid nanoparticle according to the present disclosure, the lentiviral vector according to the present disclosure, the ribonucleoprotein complex according to the present disclosure, and the virus-like particle according to the present disclosure in the manufacture of a medicament for diagnosing, treating or preventing a disease or disorder associated with a target RNA.

In some embodiments of the present disclosure, the disease or disorder associated with the target RNA refers to a disease or disorder caused by abnormal expression of the target RNA.

In some embodiments of the present disclosure, the disease or disorder associated with the target RNA includes but is not limited to age related macular degeneration.

In some embodiments of the present disclosure, the disease or disorder associated with the target RNA includes but is not limited to dry age related macular degeneration. In some embodiments of the present disclosure, the disease or disorder associated with the target RNA includes but is not limited to wet age related macular degeneration.

In some embodiments of the present disclosure, the target RNA is VEGFA RNA.

On the basis of being in accordance with the common knowledge in the art, the above preferred conditions can be arbitrarily combined to obtain preferred embodiments of the present disclosure.

BRIEF DESCRIPTION OF THE DRAWINGS

FIG. 1 is a schematic diagram of the editing efficiency results of different combinations of Cas13 and gRNA in cells.

FIG. 2 is a schematic diagram of the relative expression level results of VEGFA RNA.

FIG. 3 is a schematic diagram of the editing efficiency results of different tool combinations.

FIG. 4A is a schematic diagram of the editing efficiency comparison results of gRNA-5 and gRNA-6 at the RNA level.

FIG. 4B is a schematic diagram of the editing efficiency comparison results of gRNA-4 and gRNA-6 at the RNA level.

FIG. 4C is a schematic diagram of the knockdown efficiency comparison results at the protein level.

FIG. 5 is a schematic diagram of the editing efficiency results in mice.

FIG. 6 is a schematic diagram of the editing efficiency results of some gRNAs from gRNA-11 to gRNA-32 at the RNA level.

FIG. 7 is a schematic diagram of the editing efficiency results of gRNA-11 to gRNA-32 at the protein level.

FIG. 8 is a schematic diagram of the editing efficiency results of the region near the splicing site targeting VEGFA RNA.

FIGs. 9A and 9B are the editing effects of the adjacent regions of the target sequence targeting gRNA-30; FIG. 9A shows the specific location and corresponding editing efficiency of each target sequence, and FIG. 9B shows the editing efficiency presented in the form of mean + standard deviation.

FIG. 10 shows the distribution of reads in the genome range of the target VEGFA using IGV software after editing cells with C13-2-gRNA5 and CasRx-X-dual. The figure shows the test results of 3 batches.

FIG. 11 shows the design principles of gRNA-5-2 to gRNA-5-5.

FIG. 12 shows the distribution of reads of the target VEGFA genome range as displayed by IGV software after editing the cells with C13-2-gRNA.

FIG. 13 shows that gRNA-4 and gRNA-5 significantly inhibit neovascularization. The white dotted circular selection box in the figure represents the laser photocoagulation spots, and the neovascularization areas are stained with IB4-488 7 days after modeling. There are 10 mice in each group. Both eyes of each mouse are injected with AAV drugs in the subretinal cavity. All eyes underwent laser modeling 3 weeks after successful administration are selected. After 7 days of modeling, the samples are collected, fixed, stained, and photographed for statistical results, bar = 200 $\mu$m.

FIG. 14 shows that the combination of CasRx and the gRNA of the present disclosure in Example 12 also has a high editing efficiency.

FIG. 15 shows the effect of inhibiting neovascularization in non-human primate (NHP) cynomolgus monkeys. The drug groups C13-2-gRNA-30 and C13-2-gRNA-5 can significantly inhibit the generation of grade IV spots, that is, significantly inhibit neovascularization, and the effect is sustained for a long time.

DETAILED DESCRIPTION OF THE PREFERRED EMBODIMENT

The terms "guide sequence" and "targeting domain" can be used interchangeably.

When referring to the "guide sequence", the "t" in the sequence can be used interchangeably with "u".

When referring to the RNA sequence, the "t" in the sequence can be used interchangeably with "u".

When referring to the "direct repeat sequence", the "t" in the sequence can be used interchangeably with "u".

**Definition section:**

As used herein, the terms gene editing system guide RNA, guide RNA and gRNA can be used interchangeably. The term guide RNA is used to refer to a molecule in a gene editing system that forms a complex with an RNA-guided nuclease and guides the sequence-specific binding of the complex to a target sequence. The guide RNA comprises a guide sequence that can hybridize with a target sequence. When the RNA-guided nuclease is a Cas protein, especially a Cas13 protein, the guide RNA generally comprises a direct repeat sequence linked to the guide sequence.

As used herein, the term "guide sequence" can be used interchangeably with "targeting domain" and refers to a continuous nucleotide sequence in the gRNA that has partial or complete complementarity with a target sequence in the target RNA and can hybridize with a target sequence in the target RNA through base pairing promoted by the RNA-guided nuclease. The complete complementarity between the guide sequence and the target sequence described in the present disclosure is not necessary, as long as there is sufficient complementarity to cause hybridization and promote the formation of a gene editing complex.

Suitable direct repeat (DR) sequences can be present in the CRISPR locus structure of prokaryotes (such as bacteria and archaea) and obtained by experimental screening; they can also be obtained by sequence modification or optimization on this basis, non-limiting examples such as the deletion, replacement or addition of 1, 2, 3, 4 or more complementary base pairs in the complementary double-stranded region of the secondary structure of the DR sequence, and the deletion, replacement or addition of nucleotides on the loop of the stem-loop structure of the secondary structure of the DR sequence (for example, an aptamer sequence can be inserted into the loop). The size of the direct repeat sequence is usually tens of nucleotides, and some of its fragments are reverse complementary to each other, which means that a secondary structure is formed inside the RNA molecule, such as a stem-loop structure (commonly known as a hairpin structure), and other fragments are unstructured. When the RNA-guided nuclease is a Cas protein, the direct repeat sequence is a constant part of the gRNA molecule, which contains a strong secondary structure, which facilitates the interaction between the Cas protein and the gRNA molecule.

The term "hybridization" or "hybridizing" refers to the process by which fully or partially complementary polynucleotide chains come together under suitable hybridization conditions to form a double-stranded structure or region, including the binding between nucleic acids caused by hydrogen bonds. As used herein, the term hybridization includes situations in which the double-stranded structure or region contains one or more bulges or mismatches. The strength of hybridization and hybridization (*i.e.,* the strength of binding between nucleic acids) is affected by factors such as the degree of complementarity between the nucleic acids, the stringency of the conditions involved, and the Tm of the hybrid formed. Although hydrogen bonds are generally formed between adenine and thymine, adenine and uracil, or, cytosine and guanine, other non-classical base pairs can also form hydrogen bonds. It can be expected that modified nucleotides can form hydrogen bonds that allow or promote hybridization in a non-classical manner.

As used herein, the term target RNA refers to a polynucleotide containing a target sequence, which means a specific sequence or its reverse complement sequence that one wishes to bind, target or modify using a gene editing system. For example, it may be a completely mature mRNA molecule or a pre-mRNA molecule.

As used herein, the term target sequence refers to a short sequence in a target RNA molecule that is complementary (completely complementary or partially complementary) to the guide sequence of a gRNA molecule. The gene editing complex is specifically localized to the target sequence through the guide sequence and performs the corresponding function at or near this location. The length of the target sequence is often tens of nt (nucleotides), for example, about 10 nt, about 20 nt, about 30 nt, about 40 nt, about 50 nt or about 60 nt.

As used herein, the term cleavage (cleaving) refers to breaking the covalent bond (*e.g.,* covalent phosphodiester bond) in the ribosyl phosphodiester backbone of a polynucleotide.

The ability of the guide RNA to guide the sequence-specific binding of the gene editing complex to the target RNA can be evaluated by any suitable assay. For example, the components of the gene editing system sufficient to form a CRISPR complex, including the guide RNA to be tested, can be provided to a host cell with the corresponding target RNA molecule, such as by transfection of a vector encoding the components of the CRISPR complex, and then the preferential cleavage within the target sequence is assessed. Similarly, cleavage of a target RNA sequence can be assessed in a test tube by providing a target RNA, components of a CRISPR complex, including a guide RNA to be tested and a control guide RNA different from the guide RNA to be tested, and comparing the ability to bind to the target RNA or the rate of cleavage of the target RNA between the guide RNA to be tested and the control guide RNA. The ability of the guide RNA to guide the complex to cleave the target RNA can also be assessed by the assays described above.

The term RNA-guided nuclease refers to a polypeptide that binds to a specific target RNA sequence in a sequence-specific manner, and the polypeptide is guided to a target RNA by a guide RNA that is complexed with the polypeptide and hybridized to a target sequence on the target RNA. Cleavage of target sequences by RNA-guided nucleases can lead to strand breaks. Although RNA-guided nucleases can cleave a target sequence upon binding, the term RNA-guided nuclease also includes nuclease-inactive RNA-guided nucleases that are able to bind to but not cleave a target sequence. The RNA-guided nucleases of the present disclosure include, but are not limited to, wild-type RNA-guided nucleases (*e.g.,* C13-2 and CasRx), variants thereof (*e.g.,* mutants with complete loss of cleavage activity, mutants with partial loss of cleavage activity, mutants with increased cleavage activity, mutants with reduced off-target effects, mutants with reduced collateral effects), and functional fragments thereof or fusion proteins thereof.

As used herein, the term Cas protein is a CRISPR-associated (Cas) polypeptide or protein that, when complexed or functionally combined with one or more guide RNAs, can be guided to a target sequence in a target RNA, and can sometimes subsequently bind to or cleave the target RNA. The term Cas protein also includes nuclease-inactivated Cas proteins that can bind to but not cleave the target sequence. The Cas proteins of the present disclosure include, but are not limited to, wild-type Cas proteins (*e.g.,* C13-2 and CasRx), variants thereof (*e.g.,* mutants with complete loss of cleavage activity, mutants with partial loss of cleavage activity, mutants with increased cleavage activity, mutants with reduced off-target effects, mutants with reduced collateral effects), and functional fragments thereof or fusion proteins thereof.

As used herein, when referring to nucleotide sequences/DNA/RNA encoding proteins, RNAs or CRISPR complexes, the coding sequences may be codon-optimized. For example, the coding sequence is codon-optimized for expression in a eukaryotic cell environment, the coding sequence is codon-optimized for expression in a mammalian cell environment, or the coding sequence is codon-optimized for expression in a human cell environment.

As used herein, the term "sequence identity" (identity or percent identity) is used to refer to the matching of sequences between two polypeptides or between two nucleic acids. When a position in two compared sequences is occupied by the same base or amino acid monomer subunit *(e.g.,* a position in each of two DNA molecules is occupied by adenine, or a position in each of two polypeptides is occupied by lysine), then the molecules are identical at that position. The "percent sequence identity" (percent identity) between two sequences is a function of the number of matching positions shared by the two sequences divided by the number of compared positions $\times$ 100%. For example, if 6 out of 10 positions in two sequences match, then the two sequences have 60% sequence identity. Typically, a comparison is made when two sequences are aligned to produce maximum sequence identity. Such alignment can be performed using published and commercially available alignment algorithms and programs, such as but not limited to Clustal $\Omega$, MAFFT, Probcons, T-Coffee, Probalign, BLAST, which can be reasonably selected by a person skilled in the art. A person skilled in the art can determine suitable parameters for comparing sequences, including, for example, any algorithms needed to achieve a superior comparison or optimal comparison for the full length of the compared sequences, as well as any algorithms needed to achieve a superior comparison or optimal comparison locally for the compared sequences. When the Cas13 protein of the present disclosure is fused with other domains, the sequence identity of the Cas13 protein portion is compared.

As used herein, the term regulatory sequence is intended to include a promoter, an enhancer, an internal ribosome entry site (IRES), and other expression control elements (*e.g.,* a transcription termination signal, such as a polyadenylation signal and a poly(U) sequence). Regulatory sequences include those elements that guide the continuous expression of a nucleotide sequence in many types of host cells and those elements that guide the expression of a nucleotide sequence only in certain host cells (*e.g.*, tissue-specific regulatory sequences). Tissue-specific promoters can guide expression primarily in a desired tissue of interest such as muscle, neuron, bone, skin, blood, a specific organ (*e.g.,* liver and pancreas), or a specific cell type (*e.g.,* neuronal cells and lymphocytes). Regulatory sequences can also guide expression in a time-dependent manner such as a cell cycle-dependent or developmental stage-dependent manner, which may or may not be tissue-specific or cell type-specific. The term regulatory sequence also encompasses enhancer elements such as WPRE, a CMV enhancer, an SV40 enhancer, an HRE enhancer element, and an intron sequence between exons 2 and 3 of rabbit $\beta$-globin.

As used herein, the term promoter has the meaning commonly recognized in the art.

As used herein, the term enhancer has the meaning commonly recognized in the art.

Those skilled in the art understand that the design of expression vectors may depend on factors such as the choice of host cells to be transformed and the desired expression levels. The vector can be introduced into a host cell to produce the RNA-guided nuclease and/or guide RNA of the present disclosure.

As used herein, the term pharmaceutically acceptable excipient refers to a diluent, adjuvant, pharmaceutical carrier or other excipient administered together with the active ingredient. Its selection depends on the use and the intended method of administration. The excipient should not be incompatible with the active ingredient, for example, producing any undesirable biological effect or interacting with any other component of the pharmaceutical composition in a harmful manner. The pharmaceutical composition can be prepared by known methods in the art of pharmaceutical preparation.

**Guide RNA**

In some embodiments of the present disclosure, the guide RNA is capable of forming a complex (also referred to as a gene editing complex) with an RNA-guided nuclease and guiding the sequence-specific binding of the complex to a target RNA.

In some embodiments of the present disclosure, the guide RNA is capable of forming a complex with an RNA-guided nuclease and guiding the complex to bind to and cleave the target RNA.

In some embodiments of the present disclosure, the complex reduces the level of the target RNA in an animal (such as in a human).

In some embodiments of the present disclosure, the complex reduces the level of the target RNA in a cell, such as the level of the target RNA in a cell expressing the target RNA.

In some embodiments of the present disclosure, the complex reduces the level of the target RNA in the cell by at least 5%, at least 10%, at least 15%, at least 20%, at least 25%, at least 30%, at least 35%, at least 40%, at least 50%, at least 60%, at least 70%, at least 80%, at least 90%, at least 95%, at least 96%, at least 97%, at least 98% or at least 99%. The reduction in the level of the target RNA can be tested using conventional methods in the art; including but not limited to the qPCR method described in the examples, and untreated cells or cells treated with a gene editing system targeting a non-mammalian genome can be used as negative controls to calculate the target RNA knockdown level of the experimental group compared with the negative control.

In some embodiments of the present disclosure, the number of off-target genes when the complex binds to and cleaves the target RNA is less than 40, less than 35, less than 30, less than 25, less than 20, less than 19, less than 18, less than 17, less than 16, less than 15, less than 14, less than 13, less than 12, less than 11, less than 10, less than 9, less than 8, less than 7, less than 6, less than 5, less than 4, less than 3, less than 2 or less than 1. The number of off-target genes can be determined by conventional methods in the art. In some embodiments, the number of off-target genes is determined by taking the intersection of the set of differentially expressed genes determined by RNA sequencing with the set of off-target genes predicted by the program. Non-limiting examples, such as the method predicted by the program, include using the EMBOSS-water program to predict in the whole genome and whole cDNA sequence of the target species (Homo sapiens/Mus musculus), setting the parameters to gap _extend=0.5 & gap_extend=10, using the sense and antisense strands of the gRNA guide sequence for comparison, filtering the prediction results, and obtaining predicted potential target genes (including on-target genes and off-target genes), from which the off-target gene set is obtained by removing on-target genes.

In some embodiments of the present disclosure, the complex reduces the level of the target RNA-encoded protein in an animal (such as in a human).

In some embodiments of the present disclosure, the complex reduces the level of the target RNA-encoded protein in a cell. In some embodiments of the present disclosure, the target RNA-encoded protein is a VEGFA protein. In some embodiments of the present disclosure, the complex reduces the level of the VEGFA protein in a cell.

In some embodiments of the present disclosure, the complex reduces the level of VEGFA protein in the cell by at least 5%, at least 10%, at least 15%, at least 20%, at least 25%, at least 30%, at least 35%, at least 40%, at least 50%, at least 60%, at least 70%, at least 80%, at least 90%, at least 95%, at least 96%, at least 97%, at least 98% or at least 99%. The reduction in the level of the target RNA-encoded protein can be tested using conventional methods in the art; including but not limited to ELISA and Western Blotting, and untreated cells or cells treated with a gene editing system targeting a non-mammalian genome can be used as negative controls to calculate the knockdown level of the target RNA-encoded protein in the experimental group compared with the negative control.

In some embodiments of the present disclosure, the RNA-guided nuclease is a Cas protein. In some embodiments, the cleavage activity of the RNA-guided nuclease is completely or partially lost.

In some embodiments of the present disclosure, the RNA-guided nuclease is a Cas protein, and the guide RNA comprises a guide sequence and a direct repeat sequence.

In some embodiments of the present disclosure, the Cas protein is a Cas13 protein.

In some embodiments of the present disclosure, the Cas protein is a Cas13a protein, a Cas13b protein, a Cas13c protein or a Cas13d protein. In some embodiments of the present disclosure, the Cas protein is a Cas13a protein, a Cas13b protein, a Cas13c protein or a Cas13d protein with mutations in the catalytic center RxxxxH motif of both HEPN domains, and the cleavage activity of these Cas proteins is completely or partially lost. In some embodiments, the Cas protein is a CasRx protein. In some embodiments, the Cas protein is a dCasRx protein with mutations in both HEPN domains (R239A and H244A of HEPN-1 and R858A and H863A of HEPN-2).

In some embodiments of the present disclosure, the Cas protein comprises a sequence having ≥50% sequence identity to the sequence as shown in any one of SEQ ID NOs: 1-3. In some embodiments of the present disclosure, the Cas protein comprises a sequence having ≥60%, ≥65%, ≥70%, ≥75%, ≥80%, ≥85%, ≥90%, ≥95%, ≥96%, ≥97%, ≥98%, ≥99%, ≥99.1%, ≥99.2%, ≥99.3%, ≥99.4%, ≥99.5%, ≥99.6%, ≥99.7% or ≥99.8% sequence identity to the sequence as shown in any one of SEQ ID NOs: 1-3.

In some embodiments of the present disclosure, the Cas protein comprises a sequence having 100% sequence identity to the sequence as shown in any one of SEQ ID NOs: 1-3.

In some embodiments of the present disclosure, the Cas protein comprises the sequence as shown in any one of SEQ ID NOs: 1-3.

In some embodiments of the present disclosure, the Cas protein comprises a sequence having ≥50% sequence identity to the sequence as shown in SEQ ID NO: 3. In some embodiments of the present disclosure, the Cas protein comprises a sequence having ≥60%, ≥65%, ≥70%, ≥75%, ≥80%, ≥85%, ≥90%, ≥95%, ≥96%, ≥97%, ≥98%, ≥99%, ≥99.1%, ≥99.2%, ≥99.3%, ≥99.4%, ≥99.5%, ≥99.6%, ≥99.7% or ≥99.8% sequence identity to the sequence as shown in SEQ ID NO: 3.

In some embodiments of the present disclosure, the Cas protein comprises a sequence having 100% sequence identity to the sequence as shown in SEQ ID NO: 3.

In some embodiments of the present disclosure, the Cas protein comprises the sequence as shown in SEQ ID NO: 3.

In some embodiments of the present disclosure, the Cas protein comprises a sequence having ≥50% sequence identity to CasRx. In some embodiments of the present disclosure, the Cas protein comprises a sequence having ≥60%, ≥65%, ≥70%, ≥75%, ≥80%, ≥85%, ≥90%, ≥95%, ≥96%, ≥97%, ≥98%, ≥99%, ≥99.1%, ≥99.2%, ≥99.3%, ≥99.4%, ≥99.5%, ≥99.6%, ≥99.7% or ≥99.8% sequence identity to the sequence as shown in any one of SEQ ID NOs: 1-3.

In some embodiments of the present disclosure, the Cas protein comprises a sequence having 100% sequence identity to CasRx.

In some embodiments of the present disclosure, the Cas protein comprises the sequence of CasRx.

In some embodiments of the present disclosure, the RNA-guided nuclease comprises a nuclease portion and a fused homologous or heterologous domain.

In some embodiments of the present disclosure, the RNA-guided nuclease comprises any one or more of the following: a subcellular localization signal, a deaminase domain, a translation activation domain, a translation repression domain, an RNA methylation domain, an RNA demethylation domain, a nuclease domain, a splicing factor domain, a reporter domain, a reporter tag, an affinity domain and an affinity tag.

In some embodiments of the present disclosure, the guide sequence comprises 20-40, 20-35, 20-30 or 25-30 nucleotides.

In some embodiments of the present disclosure, the guide sequence hybridizes with the target RNA with no more than 6, no more than 5, no more than 4, no more than 3, no more than 2 or no more than 1 nucleotide mismatch.

In some embodiments of the present disclosure, the guide sequence has 100% sequence identity to the target RNA sequence, *i.e.,* completely complementary.

In some embodiments of the present disclosure, the guide RNA comprises a guide sequence and a direct repeat sequence. In some embodiments of the present disclosure, the guide RNA comprises a guide sequence and a direct repeat sequence, and the direct repeat sequence interacts with the RNA-guided nuclease.

In some embodiments of the present disclosure, the guide sequence is located at the 3' end or the 5' end of the direct repeat sequence. In some embodiments of the present disclosure, the guide sequence is located at the 3' end of the direct repeat sequence. In some embodiments of the present disclosure, the guide sequence is located at the 5' end of the direct repeat sequence.

In some embodiments of the present disclosure, the direct repeat sequence comprises a sequence having ≥70%, ≥75%, ≥80%, ≥85%, ≥90%, ≥95%, ≥96%, ≥97% or ≥98% sequence identity to the sequence as shown in any one of SEQ ID NOs: 19-21.

In some embodiments of the present disclosure, the direct repeat sequence comprises a sequence having 100% sequence identity to the sequence as shown in any one of SEQ ID NOs: 19-21.

In some embodiments of the present disclosure, the direct repeat sequence comprises the sequence as shown in any one of SEQ ID NOs: 19-21. In some embodiments of the present disclosure, the direct repeat sequence consists of the sequence as shown in any one of SEQ ID NOs: 19-21. In some embodiments of the present disclosure, the direct repeat sequence consists of the sequence as shown in SEQ ID NO: 21.

In some embodiments of the present disclosure, the direct repeat sequence comprises a sequence having ≥70%, ≥75%, ≥80%, ≥85%, ≥90%, ≥95%, ≥96%, ≥97% or ≥98% sequence identity to the direct repeat sequence corresponding to CasRx in the examples of the present disclosure.

In some embodiments of the present disclosure, the direct repeat sequence comprises a sequence having 100% sequence identity to the direct repeat sequence corresponding to CasRx in the examples of the present disclosure.

In some embodiments of the present disclosure, the direct repeat sequence comprises the direct repeat sequence corresponding to CasRx in the examples of the present disclosure.

In some embodiments of the present disclosure, the guide RNA comprises an aptamer sequence.

In some embodiments of the present disclosure, the aptamer sequence is inserted into a loop of the stem-loop structure of the secondary structure of the direct repeat sequence of the guide RNA.

In some embodiments of the present disclosure, the guide RNA comprises a modified nucleotide. Such modifications include, but are not limited to, 2'-O-methyl modification, 2'-O-methyl-3'-thiophosphate modification or 2'-O-methyl-3'-thio PACE modification. In some embodiments of the present disclosure, the guide RNA comprises a modified nucleotide, and the modified nucleotide is selected from a deoxyribonucleotide and a locked nucleic acid (LNA). In some embodiments, the guide RNA comprises at least one chemically modified nucleotide. In some embodiments, the guide RNA is a hybrid RNA-DNA guide, that is, some RNA nucleotides in the guide RNA are replaced by DNA nucleotides. In some embodiments, the guide RNA is a hybrid RNA-LNA (locked nucleic acid) guide, that is, some RNA nucleotides in the guide RNA are replaced by LNA nucleotides.

In some embodiments of the present disclosure, the target RNA is located in the nucleus and/or cytoplasm of a eukaryotic cell.

**Target RNA**

The gene editing system and composition described in the present disclosure can be used to target one or more target RNA molecules, such as target RNA molecules present in a biological sample. In some embodiments, the target RNA is pre-mRNA or mRNA (mature mRNA).

In some embodiments of the present disclosure, the target RNA is VEGFA RNA. In some embodiments of the present disclosure, the target RNA is human VEGFA RNA.

In some embodiments of the present disclosure, the target RNA is VEGFA RNA or a fragment thereof; optionally, the VEGFA RNA is derived from a human.

In some embodiments of the present disclosure, the target RNA is VEGFA mRNA or a fragment thereof; optionally, the VEGFA mRNA is derived from a human.

In some embodiments of the present disclosure, the target RNA is VEGFA pre-mRNA or a fragment thereof; optionally, the VEGFA pre-mRNA is derived from a human.

In some embodiments of the present disclosure, the target RNA is located in the nucleus or cytoplasm of a cell.

In some embodiments of the present disclosure, the expression level of VEGFA RNA can be increased using the gene editing system according to the present disclosure; for example, an RNA-guided nuclease fused to a translation activation domain is used to contact VEGFA RNA under gRNA guidance and increase its translation level. In some embodiments, the expression level of VEGFA RNA can be reduced using the gene editing system according to the present disclosure; for example, using an RNA-guided nuclease, the VEGFA RNA is contacted and cleaved under gRNA guidance. In some embodiments, the expression level of VEGFA RNA in a cell can be reduced by using the gene editing system according to the present disclosure.

**RNA-guided nuclease**

In some embodiments of the present disclosure, the RNA-guided nuclease is a Cas protein.

In some embodiments of the present disclosure, the RNA-guided nuclease is selected from wild-type RNA-guided nucleases (including but not limited to CasRx, C 13-2, *etc.*), variants thereof (including but not limited to mutants with complete loss of cleavage activity, mutants with partial loss of cleavage activity, mutants with improved cleavage activity or mutants with reduced off-target/collateral effects), or functional fragments thereof (*e.g.*, fragments that only retain RNA binding domain or fragments with the HEPN domain deleted).

In some embodiments of the present disclosure, the RNA-guided nuclease comprises a nuclease portion and a fused homologous or heterologous domain.

In some embodiments of the present disclosure, the RNA-guided nuclease comprises any one or more of the following fusion domains: a subcellular localization signal, a deaminase domain, a translation activation domain, a translation repression domain, an RNA methylation domain, an RNA demethylation domain, a nuclease domain, a splicing factor domain, a reporter tag and an affinity tag.

In some embodiments of the present disclosure, the RNA-guided nuclease comprises a subcellular localization signal.

In some embodiments of the present disclosure, the RNA-guided nuclease comprises a subcellular localization signal and a deaminase domain.

In some embodiments of the present disclosure, the subcellular localization signal is selected from a nuclear localization signal and a nuclear export signal.

In some embodiments of the present disclosure, the deaminase domain is selected from: cytidine deaminase and adenosine deaminase.

In some embodiments of the present disclosure, the deaminase domain is selected from: PPR protein (Pentatricopeptide repeat), ADAR family protein and APOBEC family protein.

In some embodiments of the present disclosure, the translation activation domain is selected from: eIF4E and other translation initiation factors, yeast poly (A)-binding proteins and domains of GLD2.

In some embodiments of the present disclosure, the translation repression domain is selected from: Pumilio protein, deadenylase (such as deadenylase CAF 1) and Argonaute protein.

In some embodiments of the present disclosure, the nuclease domain is selected from: FokI, PIN endonuclease domain, NYN domain, SMR domain from SOT1, and RNase domain from *Staphylococcus* nuclease.

In some embodiments of the present disclosure, the methylase domain is selected from: m6A methyltransferase.

In some embodiments of the present disclosure, the demethylation domain is selected from: RNA demethylase ALKBH5.

In some embodiments of the present disclosure, the regulatory splicing domain is selected from: SRSF1, hnRNP A1 and RBM4.

In some embodiments of the present disclosure, the RNA-guided nuclease is covalently linked to the fusion domain through or without a linker sequence; that is, the RNA-guided nuclease is directly covalently linked to the fusion domain (excluding the linker sequence), or can be covalently linked through a linker sequence. Typically, the linker sequence consists of 1-100, 1-50, 1-30, 1-20, 1-10 or 1-5 amino acids.

**Cas protein**

In some embodiments of the present disclosure, the Cas protein is a Cas13 protein.

In some embodiments of the present disclosure, the Cas protein is a Cas13a protein, a Cas13b protein, a Cas13c protein or a Cas13d protein. In some embodiments of the present disclosure, the RNA-guided nuclease is a Cas13d protein.

In some embodiments of the present disclosure, the Cas protein is a Cas13a protein, a Cas13b protein, a Cas13c protein or a Cas13d protein with mutations in both HEPN domains, and the cleavage activity of these Cas proteins is completely or partially lost. In some embodiments, the Cas protein comprises the amino acid sequence of a CasRx protein. In some embodiments, the Cas protein is a CasRx protein. In some embodiments, the Cas protein is a dCasRx protein with mutations in both HEPN domains (R239A and H244A of HEPN-1 and R858A and H863A of HEPN-2).

In some embodiments of the present disclosure, the Cas protein comprises a sequence having ≥50% sequence identity to the sequence as shown in any one of SEQ ID NOs: 1-3. In some embodiments of the present disclosure, the Cas protein comprises a sequence having ≥60%, ≥65%, ≥70%, ≥75%, ≥80%, ≥85%, ≥90%, ≥95%, ≥96%, ≥97%, ≥98%, ≥99%, ≥99.1%, ≥99.2%, ≥99.3%, ≥99.4%, ≥99.5%, ≥99.6%, ≥99.7% or ≥99.8% sequence identity to the sequence as shown in any one of SEQ ID NOs: 1-3.

In some embodiments of the present disclosure, the Cas protein comprises a sequence having 100% sequence identity to the sequence as shown in any one of SEQ ID NOs: 1-3.

In some embodiments of the present disclosure, the Cas protein comprises the sequence as shown in any one of SEQ ID NOs: 1-3.

In some embodiments of the present disclosure, the Cas protein comprises a sequence having ≥50% sequence identity to the sequence as shown in SEQ ID NO: 3. In some embodiments of the present disclosure, the Cas protein comprises a sequence having ≥60%, ≥65%, ≥70%, ≥75%, ≥80%, ≥85%, ≥90%, ≥95%, ≥96%, ≥97%, ≥98%, ≥99%, ≥99.1%, ≥99.2%, ≥99.3%, ≥99.4%, ≥99.5%, ≥99.6%, ≥99.7% or ≥99.8% sequence identity to the sequence as shown in SEQ ID NO: 3.

In some embodiments of the present disclosure, the Cas protein comprises a sequence having ≥50%, ≥60%, ≥65%, ≥70%, ≥75%, ≥80%, ≥85%, ≥90%, ≥95%, ≥96%, ≥97%, ≥98%, ≥99%, ≥99.1%, ≥99.2%, ≥99.3%, ≥99.4%, ≥99.5%, ≥99.6%, ≥99.7% or ≥99.8% sequence identity to the sequence as shown in SEQ ID NO: 3, and the Cas protein:

(a) mutates to other residues at the positions corresponding to residues R210 and H215 in the sequence as shown in SEQ ID NO: 3;
(b) mutates to other residues at the positions corresponding to residues R785 and H790 in the sequence as shown in SEQ ID NO: 3;
(c) mutates to other residues at the positions corresponding to residues R210, H215, R785 and H790 in the sequence as shown in SEQ ID NO: 3; or
(d) mutates to other residues at the positions corresponding to residues R210, H215, R750A, H755A, R785 and H790 in the sequence as shown in SEQ ID NO: 3.

In some embodiments of the present disclosure, the Cas protein comprises a sequence having ≥50%, ≥60%, ≥65%, ≥70%, ≥75%, ≥80%, ≥85%, ≥90%, ≥95%, ≥96%, ≥97%, ≥98%, ≥99%, ≥99.1%, ≥99.2%, ≥99.3%, ≥99.4%, ≥99.5%, ≥99.6%, ≥99.7% or ≥99.8% sequence identity to the sequence as shown in SEQ ID NO: 3, and the Cas protein:

(a) mutates to A at the positions corresponding to residues R210 and H215 in the sequence as shown in SEQ ID NO: 3;
(b) mutates to A at the positions corresponding to residues R785 and H790 in the sequence as shown in SEQ ID NO: 3;
(c) mutates to A at the positions corresponding to residues R210, H215, R785 and H790 in the sequence as shown in SEQ ID NO: 3; or
(d) mutates to A at the positions corresponding to residues R210, H215, R750A, H755A, R785 and H790 in the sequence as shown in SEQ ID NO: 3.

In some embodiments of the present disclosure, the Cas protein comprises a sequence having 100% sequence identity to the sequence as shown in SEQ ID NO: 3.

In some embodiments of the present disclosure, the Cas protein comprises the sequence as shown in SEQ ID NO: 3.

In some embodiments of the present disclosure, the Cas protein comprises a sequence having ≥50% sequence identity to CasRx. In some embodiments of the present disclosure, the Cas protein comprises a sequence having ≥60%, ≥65%, ≥70%, ≥75%, ≥80%, ≥85%, ≥90%, ≥95%, ≥96%, ≥97%, ≥98%, >99% or ≥99.5% sequence identity to the sequence as shown in any one of SEQ ID NOs: 1-3.

In some embodiments of the present disclosure, the Cas protein comprises a sequence having 100% sequence identity to CasRx.

In some embodiments of the present disclosure, the Cas protein comprises the sequence of CasRx.

In some embodiments of the present disclosure, the Cas protein is a Cas13b protein. In some embodiments of the present disclosure, the Cas13b protein is a Cas13X protein (also known as Cas13e) or a Cas13Y protein (also known as Cas13f). In some embodiments of the present disclosure, the Casl3 protein has ≥80%, ≥85%, ≥90%, ≥95%, ≥98%, ≥99% or 100% sequence identity to any one of Cas13e.1-Cas13e.8. In some embodiments of the present disclosure, the Cas13 protein has ≥80%, ≥85%, ≥90%, ≥95%, ≥98%, ≥99% or 100% sequence identity to any one of Cas13f.1-Casl3f.7.

In some embodiments of the present disclosure, the Cas protein comprises a Cas protein portion and a fused homologous or heterologous domain.

In some embodiments of the present disclosure, the Cas protein comprises any one or more of the following fusion domains: a subcellular localization signal, a deaminase domain, a translation activation domain, a translation repression domain, an RNA methylation domain, an RNA demethylation domain, a nuclease domain, a splicing factor domain, a reporter domain, a reporter tag, an affinity domain and an affinity tag.

In some embodiments of the present disclosure, the Cas protein comprises a subcellular localization signal.

In some embodiments of the present disclosure, the Cas protein comprises a subcellular localization signal and a deaminase domain.

In some embodiments of the present disclosure, the subcellular localization signal is selected from a nuclear localization signal and a nuclear export signal.

In some embodiments of the present disclosure, the Cas protein is covalently linked to the fusion domain through or without a linker sequence; that is the Cas protein is directly covalently linked to the fusion domain (excluding the linker sequence), or can be covalently linked through a linker sequence. Typically, the linker sequence consists of 1-100, 1-50, 1-30, 1-20, 1-10 or 1-5 amino acids.

**Nucleotide sequence encoding guide RNA or RNA-guided nuclease**

In some embodiments, the nucleotide sequence encoding the RNA-guided nuclease is a plasmid. In some embodiments, the nucleotide sequence encoding the RNA-guided nuclease is part of a viral vector genome, such as a DNA genome of an AAV vector flanked by ITRs. In some embodiments, the nucleotide sequence encoding the RNA-guided nuclease is mRNA.

In some embodiments of the present disclosure, the nucleotide sequence encoding the guide RNA or RNA-guided nuclease is codon-optimized.

In some embodiments of the present disclosure, the nucleotide sequence encoding the guide RNA is DNA. In some embodiments, the sequence of the DNA is codon-optimized.

In some embodiments of the present disclosure, the nucleotide sequence encoding the RNA-guided nuclease is DNA or mRNA. In some embodiments, the sequence of the DNA is codon-optimized. In some embodiments, the sequence of the mRNA is codon-optimized.

In some embodiments of the present disclosure, codon optimization is performed for expression in a desired cell type. In some embodiments, codon optimization is performed for expression in a eukaryotic cell environment. In some embodiments, codon optimization is performed for expression in a mammalian cell environment. In some embodiments, codon optimization is performed for expression in a human cell environment.

In general, codon optimization refers to modifying a nucleic acid sequence to enhance expression in a host cell of interest by replacing at least one codon (*e.g.,* about 1, 2, 3, 4, 5, 10, 15, 20, 25, 50 or more codons) of the original sequence with a codon that is more frequently used in the genes of the host cell while maintaining the original amino acid sequence. Computer programs or algorithms for codon optimization are also available, for example, the following tools or algorithms can be used for codon optimization: ExpOptimizer, Codon OptimWiz, NGTM Codon, Codon optimization, Synthetic Gene Designer, DNAWorks, *etc.*

**Vector or vector system**

The vector may comprise any type of nucleotides, including but not limited to DNA and RNA, may be single-stranded or double-stranded, may be partially obtained from natural sources, and may comprise natural, non-natural or modified nucleotides. Suitable vectors include those designed for expression, such as plasmids and viruses.

In some embodiments, the recombinant vector comprises regulatory sequences, such as transcription and translation start codons and stop codons, and the regulatory sequences are specific to the type of host cell (*e.g.,* bacteria, fungi, plants or animals) into which the vector is to be introduced, as the case may be.

In some embodiments, the recombinant vector optionally comprises a gene vector element (nucleic acid), such as a selectable marker region, a lactose operon, a CMV promoter, a CAG promoter, a tac promoter, a T7 RNA polymerase promoter, an SP6 RNA polymerase promoter, an SV40 promoter, an IRES sequence, a WPRE element, an ITR sequence, a FLAG tag coding region, a c-myc tag coding region, a polyHis tag coding region, a HA tag coding region, a MBP tag coding region, a GST tag coding region, a ployA coding region, an SV40 polyadenylation signal, an SV40 replication origin, a Col E1 replication origin, a loxP site or a Cre recombinase coding region.

**Regulatory sequence**

In some embodiments of the present disclosure, the regulatory sequence comprises one or more pol III promoters (*e.g.*, 1, 2, 3, 4, 5 or more pol III promoters), one or more pol II promoters (*e.g.,* 1, 2, 3, 4, 5 or more pol II promoters), one or more pol I promoters (*e.g.,* 1, 2, 3, 4, 5 or more pol I promoters), or a combination thereof.

In some embodiments of the present disclosure, the regulatory sequence comprises an HRE enhancer element (hypoxia response element). In some embodiments, the regulatory sequence comprises at least 2, at least 3, at least 4, at least 5, at least 6, at least 7, at least 8 or at least 9 HRE enhancer elements in tandem. In some embodiments, the regulatory sequence comprises 2, 3, 4, 5, 6, 7, 8 or 9 HRE enhancer elements in tandem.

In some embodiments of the present disclosure, the regulatory sequence comprises an NRS element (neuron restrictive silencer) from the human synapsin gene. In some embodiments, the regulatory sequence comprises an NRS element and an HRE enhancer element. In some embodiments, the regulatory sequence comprises an NRS element and an HRE enhancer element in tandem. To enhance the regulatory effect, the NRS element and HRE enhancer element in tandem can be repeated multiple times. In some embodiments, the regulatory sequence comprises an NRS element and an HRE enhancer element in tandem repeated at least 2 times, at least 3 times, at least 4 times, at least 5 times or at least 6 times. In some embodiments, the regulatory sequence comprises an NRS element and an HRE enhancer element in tandem repeated 2 times, 3 times, 4 times, 5 times or 6 times.

In some embodiments of the present disclosure, the HRE enhancer element sequence is TGTCACGTCCTG CACGACGTA (SEQ ID NO: 233) or a reverse complement sequence thereof.

In some embodiments of the present disclosure, the NRS element sequence is TTCAGCACCGCGGACAGT GCC (SEQ ID NO: 234) or a reverse complement sequence thereof.

In some embodiments of the present disclosure, the regulatory sequence comprises repeated TGTCACGTC CTGCACGACGTA (SEQ ID NO: 233) or a reverse complement sequence thereof.

In some embodiments of the present disclosure, the regulatory sequence comprises TTCAGCACCGCGGA CAGTGCCTGTCACGTCCTGCACGACGTA (SEQ ID NO: 235) in tandem or a reverse complement sequence thereof.

**Promoter**

In some embodiments of the present disclosure, the vector comprises a pol III promoter (*e.g.,* U6 and H1 promoters), a pol II promoter (*e.g.,* retroviral Rous sarcoma virus (RSV) LTR promoter (optionally with an RSV enhancer), a cytomegalovirus (CMV) promoter (optionally with a CMV enhancer), an SV40 promoter, a dihydrofolate reductase promoter, a β-actin promoter, a phosphoglycerate kinase (PGK) promoter or an EF1α promoter), or a pol III promoter and a pol II promoter.

In some embodiments of the present disclosure, the promoter is a constitutive promoter, which is continuously active and is not regulated by external signals or molecules. Suitable constitutive promoters include but are not limited to CMV, RSV, SV40, EF1α, CAG, CBh and β-actin promoters. In some embodiments, the promoter is an inducible promoter regulated by an external signal or molecule *(e.g.,* a transcription factor).

In some embodiments of the present disclosure, the promoter is selected from: RPE65, VDM2, Mlc1, GFAP and ICAM-2 promoters.

In some embodiments of the present disclosure, the promoter is a tissue-specific promoter, which can be used to drive tissue-specific expression of the guide RNA or gene editing system according to the present disclosure.

In some embodiments of the present disclosure, the promoter is an eye-specific promoter.

In some embodiments of the present disclosure, the eye-specific promoter is selected from: a retinoschisis protein promoter, a K12 promoter, a rhodopsin promoter, a rod cell-specific promoter, a cone cell-specific promoter, a rhodopsin kinase promoter, a GRK1 promoter, an interphotoreceptor retinoid-binding protein proximal (IRBP) promoter and an opsin promoter (*e.g.*, a red opsin promoter or a blue opsin promoter).

In some embodiments of the present disclosure, the promoter is a chicken β-actin (CB) promoter. The chicken β-actin promoter can be a short chicken β-actin promoter or a long chicken β-actin promoter. In some embodiments, the promoter (*e.g.,* a chicken β-actin promoter) comprises an enhancer sequence, such as a cytomegalovirus (CMV) enhancer sequence. The CMV enhancer sequence can be a short CMV enhancer sequence or a long CMV enhancer sequence. In some embodiments, the promoter comprises a long CMV enhancer sequence and a long chicken β-actin promoter. In some embodiments, the promoter comprises a short CMV enhancer sequence and a short chicken β-actin promoter. However, those skilled in the art know that a short CMV enhancer can be used with a long CB promoter, and a long CMV enhancer can be used with a short CB promoter. In some embodiments of the present disclosure, the promoter is a CBh promoter.

Suitable muscle-specific promoters include, but are not limited to, CK8, MHCK7, a myoglobin promoter (Mb), a desmin promoter, a muscle creatine kinase promoter (MCK) and variants thereof, and an SPc5-12 synthetic promoter. Suitable immune cell-specific promoters include, but are not limited to, a B29 promoter (B cells), a CD14 promoter (monocytes), a CD43 promoter (leukocytes and platelets), a CD68 (macrophages), and an SV40/CD43 promoter (leukocytes and platelets). Suitable blood cell-specific promoters include, but are not limited to, a CD43 promoter (leukocytes and platelets), a CD45 promoter (hematopoietic cells), an INF-β (hematopoietic cells), a WASP promoter (hematopoietic cells), an SV40/CD43 promoter (leukocytes and platelets), and an SV40/CD45 promoter (hematopoietic cells). Suitable pancreas-specific promoters include, but are not limited to, an elastase-1 promoter. Suitable endothelial cell-specific promoters include, but are not limited to, a Fit-1 promoter and an ICAM-2 promoter. Suitable neuronal tissue/cell-specific promoters include, but are not limited to, a GFAP promoter (astrocytes), an SYN1 promoter (neurons), and an NSE/RU5' promoter (mature neurons). Neuronal tissue/cell-specific promoters may be a GFAP promoter or an SYN1 promoter. Suitable kidney-specific promoters include, but are not limited to, an NphsI promoter (podocytes). Suitable bone-specific promoters include, but are not limited to, an OG-2 promoter (osteoblasts and odontoblasts). Suitable lung-specific promoters include, but are not limited to, an SP-B promoter (lung). Suitable liver-specific promoters include, but are not limited to, an SV40/Alb promoter. Suitable heart-specific promoters include, but are not limited to, α-MHC.

## Enhancer

In some embodiments of the present disclosure, the enhancer is selected from WPRE, a CMV enhancer, an SV40 enhancer, an HRE enhancer element, and an intron sequence between exons 2 and 3 of rabbit β-globin.

In some embodiments of the present disclosure, the enhancer is located upstream of the promoter element; however, it may also be located downstream or within the coding sequence regulated by the promoter and maintain its function. Thus, an enhancer or a portion thereof may be present in an RNA sequence transcribed from a coding sequence.

In some embodiments of the present disclosure, the enhancer may be located in 100, 200, 300, 400, 500 or more base pairs upstream or downstream of a coding sequence regulated by a promoter.

In some embodiments of the present disclosure, the enhancer increases the expression of the coding sequence to a level higher than the increased expression provided by the promoter.

## Adeno-associated virus vector (AAV vector)

In some embodiments of the present disclosure, the AAV vector comprises an ssDNA genome comprising a coding sequence for an RNA-guided nuclease and guide RNA flanked by ITRs.

In some embodiments of the present disclosure, the guide RNA or gene editing system described herein is encapsulated in an AAV vector, for example, encapsulated in an AAV1, AAV2, AAV3, AAV4, AAV5, AAV6, AAV7, AAV8, AAV9, AAV10, AAV11, AAV12, AAV13, AAV PHP.B, AAV PHP.B2, AAV PHP.B3, AAV PHP.A, AAV PHP.eB, AAV PHP.eS, AAV2.7m8, AAV8.7m8, AAV ShH10, AAVrh10 or AAVrh74 capsid.

In some embodiments of the present disclosure, the guide RNA or gene editing system described herein is encapsulated in an AAV2, AAV5, AAV6, AAV8 or AAV9 capsid.

In some embodiments of the present disclosure, the AAV vector described herein is selected from: AAV2/2, AAV2/3, AAV2/4, AAV2/5, AAV2/6, AAV2/7, AAV2/8, AAV2/9, AAV2/10, AAV2/11, AAV2/12, AAV2/13, AAV2/PHP.B, AAV2/PHP.B2, AAV2/PHP.B3, AAV2/PHP.A, AAV2/PHP.eB, AAV2/PHP.eS, AAV2/2.7m8, AAV2/8.7m8, AAV2/ShH10, AAV2/rh10 and AAV2/rh74.

In some embodiments of the present disclosure, the AAV vector described herein is selected from: AAV2/2, AAV2/5, AAV2/6, AAV2/8 and AAV2/9.

In some embodiments, the gene editing system described herein is encapsulated in an AAV vector comprising an engineered capsid with tissue tropism, such as an engineered capsid with ocular tissue tropism.

## Lipid nanoparticle

In some embodiments of the present disclosure, in addition to the RNA payload (mRNA encoding the RNA-guided nuclease and the guide RNA), the lipid nanoparticle (LNP) further comprises four components: a cationic or ionizable lipid, cholesterol, a helper lipid and a PEG-lipid. In some embodiments, the cationic or ionizable lipid comprises cKK-E12, C12-200, ALC-0315, DLin-MC3-DMA, DLin-KC2-DMA, FTT5, Moderna SM-102 and Intellia LP01. In some embodiments, the PEG-lipid comprises PEG-2000-C-DMG, PEG-2000-DMG or ALC-0159. In some embodiments, the helper lipid comprises DSPC.

**Lentiviral vector**

In some embodiments of the present disclosure, the lentiviral vector is pseudotyped with homologous or heterologous envelope proteins, such as VSV-G. In some embodiments, the mRNA encoding the RNA-guided nuclease is linked to an aptamer sequence.

**Aptamer/aptamer sequence**

In some embodiments, the guide polynucleotide further comprises an aptamer sequence. In some embodiments, the aptamer sequence is inserted into a loop of the guide polynucleotide. In some embodiments, the aptamer sequence is attached to the end of the guide polynucleotide.
In some embodiments, the aptamer sequence comprises an MS2 aptamer sequence, a PP7 aptamer sequence or a Qβ aptamer sequence.

**Adaptor protein**

In some embodiments, the gene editing system further comprises a fusion protein comprising an adaptor protein and a fusion domain, or a nucleic acid encoding the fusion protein, wherein the adaptor protein is capable of binding to the aptamer sequence.
In some embodiments, the adaptor protein comprises an MS2 bacteriophage coat protein (MCP), a PP7 bacteriophage coat protein (PCP) or a Qβ bacteriophage coat protein (QCP). In some embodiments, the fusion domain comprises a cytosine deaminase domain, an adenosine deaminase domain, a translation activation domain, a translation repression domain, an RNA methylation domain, an RNA demethylation domain, a nuclease domain, a splicing factor domain, or an affinity or reporter tag or domain.

**RNP complex (ribonucleoprotein complex)**

In some embodiments of the present disclosure, the RNP complex (ribonucleoprotein complex) can be delivered to eukaryotic cells, mammalian cells or human cells by microinjection or electroporation. In some embodiments, the ribonucleoprotein complex can be encapsulated in virus-like particles and delivered to mammals or human subjects *in vivo.*

**Virus-like particle**

In some embodiments of the present disclosure, the engineered virus-like particles (VLPs) are pseudotyped with homologous or heterologous envelope proteins, such as VSV-G. In some embodiments, the RNA-guided nuclease is fused to a gag protein (*e.g.*, MLVgag) via a cleavable linker, wherein cleavage of the linker in the target cell exposes an NLS located between the linker and the RNA-guided nuclease. In some embodiments, the fusion protein comprises (*e.g.*, from 5' to 3') a gag protein (*e.g.*, MLVgag), one or more NESs, a cleavable linker, one or more NLSs and an RNA-guided nuclease. In some embodiments, the RNA-guided nuclease is fused to a first dimerization domain capable of dimerizing or heterodimerizing with a second dimerization domain fused to a membrane protein, wherein the presence of a ligand promotes dimerization and enriches the RNA-guided nuclease or fusion protein into VLPs.

**Eukaryotic cell**

In some embodiments of the present disclosure, the eukaryotic cell is a mammalian cell or a human cell. In some embodiments, the eukaryotic cells are primary eukaryotic cells, stem cells, tumor/cancer cells, circulating tumor cells (CTCs), blood cells (*e.g.,* T cells, B cells, NK cells or Tregs), hematopoietic stem cells, specialized immune cells (*e.g.*, tumor-infiltrating lymphocytes or tumor-suppressing lymphocytes), or stromal cells in the tumor microenvironment (*e.g.*, cancer-associated fibroblasts). In some embodiments, the cells are brain or neuronal cells of the central or peripheral nervous system (*e.g.*, neurons, astrocytes, microglia, retinal ganglion cells or rod/cone cells).

**Disease or disorder associated with target RNA**

In some embodiments of the present disclosure, the disease or disorder associated with the target RNA refers to a disease or disorder caused by abnormal expression of the target RNA. In some embodiments of the present disclosure, the disease or disorder associated with the target RNA refers to a disease or disorder caused by abnormally high expression of the target RNA. In some embodiments of the present disclosure, the disease or disorder associated with the target RNA refers to a disease or disorder caused by abnormally low expression of the target RNA.

In some embodiments of the present disclosure, the disease or disorder associated with the target RNA includes but is not limited to age related macular degeneration.

In some embodiments of the present disclosure, the disease or disorder associated with the target RNA includes but is not limited to dry age related macular degeneration. In some embodiments of the present disclosure, the disease or disorder associated with the target RNA includes but is not limited to wet age related macular degeneration.

In some embodiments of the present disclosure, the target RNA is VEGFA RNA.

In some embodiments of the present disclosure, the target RNA is mammalian VEGFA RNA.

In some embodiments of the present disclosure, the target RNA is non-human primate VEGFA RNA.

In some embodiments of the present disclosure, the target RNA is human VEGFA RNA.

In some embodiments, the pharmaceutical composition is delivered to a human subject *in vivo.* The pharmaceutical composition may be delivered by any effective route, and a therapeutically effective amount of the pharmaceutical composition can be delivered to a subject in need thereof. Exemplary routes of administration include, but are not limited to, intravenous infusion, intravenous injection, intraperitoneal injection, intramuscular injection, intratumoral injection, subcutaneous injection, intradermal injection, intraventricular injection, intravascular injection, intracerebellar injection, intraocular injection, subretinal injection, intravitreal injection, intracameral injection, intratympanic injection, intranasal administration and inhalation.

In some embodiments of the present disclosure, a therapeutically effective amount of the gene editing system or pharmaceutical composition according to the present disclosure is delivered to a subject in need thereof using the delivery method disclosed herein, which can inhibit the expression or activity of VEGFA RNA or its encoded protein in ocular tissue cells, reduce neovascularization, and thereby diagnosing, preventing and/or treating macular degeneration diseases, such as neovascular age related macular degeneration.

The present disclosure is further illustrated by way of specific examples below, but the present disclosure is not limited to the scope of the described examples. The experimental methods for which specific conditions are not indicated in the following examples are selected according to conventional methods and conditions, or according to the product instructions.

**Examples**

**Example 1: CRISPR gene editing method for downregulating vegfa gene transcription level**

The restriction endonucleases involved in this example were purchased from NEB. The small-scale plasmid DNA extraction kit, 2×Accurate Taq master Mix and the genomic DNA extraction kit were purchased from Accurate Biotech (Hunan). OPTI-MEM, T4 DNA ligase and Lipofectamine 2000 transfection reagent were purchased from Thermo. The primers used for gRNA, PCR and sequencing were synthesized by conventional methods.

I. Source of Cas13 protein and plasmid acquisition

This example involves a variety of Cas13 proteins to verify the effect of targeting VEGFA, including Cas13m.3 (not belonging to the Cas13d subtype), Cas13m.6 (not belonging to the Cas13d subtype), and C13-2 (also known as CasRfg.4, belonging to the Cas13d subtype) recently discovered by the applicant. These three Cas13 proteins have amino acid sequences of Cas13m.3 (SEQ ID NO: 1), Cas13m.6 (SEQ ID NO: 2) and C13-2 (SEQ ID NO: 3), respectively.

Cas13m.3-BsaI plasmid (sequence as shown in SEQ ID NO: 4), Cas13m.6-BsaI plasmid (sequence as shown in SEQ ID NO: 5) and C13-2-BsaI plasmid (sequence as shown in SEQ ID NO: 6) were prepared by the laboratory outsourcing service company, which contained the coding sequence of different Cas13 proteins optimized by codons (CMV-driven expression) and the coding sequence of the direct repeat sequence (U6-driven expression).

II. Construction of recombinant plasmid

1. gRNA design

First, the guide sequence of gRNA was determined, which targeted the RNA sequence (target RNA) after transcription of the VEGFA gene. The guide sequence of gRNA was the reverse complement sequence of the target sequence on the target RNA molecule. gRNA was designed with a guide sequence length of 21-29 nt, as shown in Table 1 below, and Cas13m.3-BsaI (also known as Cas13m.3-V), Cas13m.6-BsaI (also known as Cas13m.6-V) and C13-2-BsaI (also known as C13-2-V) vectors were used as negative controls (Cas13m.3-BsaI and Cas13m.6-BsaI vectors contained gRNA coding sequences, and their guide sequences were ggagaccacggcaggtctca (SEQ ID NO: 7), which were theoretically disordered sequences and would not target the mammalian genome; C13-2-BsaI vectors contained gRNA coding sequences, and their guide sequences were ggagaccacggcaggtctca (SEQ ID NO: 7), which would not theoretically target the mammalian genome).

Table 1. Guide sequence of gRNA

| Number | Guide sequence of gRNA |
|---|---|
| gRNA-1 | GGTACTCCTGGAAGATGTCCACCAGGGTCT(SEQ ID NO: 9) |
| gRNA-2 | CCACTGCGGCCCCCTCTCCTCTTCCTTC(SEQ ID NO: 10) |
| gRNA-3 | GGCTGGAGCACTGTCTGCGCACA(SEQ ID NO: 11) |
| gRNA-4 | AAGCTCATCTCTCCTATGTGCTGGC(SEQ ID NO: 12) |
| gRNA-5 | TGGGTGCAGCCTGGGACCACTTGGCATGG(SEQ ID NO: 13) |
| gRNA-6 | TCTTTCTTTGGTCTGCATTCACAT(SEQ ID NO: 14) |
| gRNA-7 | GGCCTTGGTGAGGTTTGATCCGCAT(SEQ ID NO: 15) |
| gRNA-8 | ACAAACAAATGCTTTCTCCGCTCTGA(SEQ ID NO: 16) |
| gRNA-9 | GCAGGAACATTTACACGTCTGCGGATCT(SEQ ID NO: 17) |
| gRNA-10 | AGTACGTTCGTTTAACTCAAGCTGCC(SEQ ID NO:18) |

2. Construction of plasmid

All gRNA recombinant plasmids were constructed by annealing gRNA forward and reverse primers, and Cas13m.3-BsaI, Cas13m.6-BsaI and C13-2-BsaI plasmids were digested by BsaI. Then, the sense and antisense strands of the DNA sequence corresponding to the guide sequence of gRNA were annealed and ligated to the Cas13m.3-BsaI, Cas13m.6-BsaI and C13-2-BsaI vectors respectively by T4 ligase. The direct repeat sequences of gRNA encoded by Cas13m.3, Cas13m.6 and C13-2 vectors were:

5'-GTTGTAGAAGCCGTTCATTCGGGACGGTATGACAAC-3' (SEQ ID NO: 19),
5'-GTTGTAGAAGCCTATCGTTAGGATAGGTATGACAAC-3' (SEQ ID NO: 20),
5'-GGAAGATAACTCTACAAACCTGTAGGGTTCTGAGAC-3' (SEQ ID NO: 21).

The sense and antisense strands of the DNA sequence corresponding to the guide sequence of gRNA were synthesized by conventional methods. Corresponding to the Cas13m.3-BsaI and Cas13m.6-BsaI vectors, cacc was added to the 5'-end of the sense strand (when the 5'-end of the guide sequence was not g, caccg was added), and caac was added to the 5'-end of the antisense strand (when the 5'-end of the guide sequence was not g, c was added to the 3'-end of the antisense strand). Corresponding to the C13-2-BsaI vector, agac was added to the 5'-end of the sense strand, and aaaa was added to the 5'-end of the antisense strand.

According to the following reaction system, 2 $\mu$L of the sense strand and the antisense strand of the DNA sequence corresponding to the above gRNA target sequence were each taken, mixed, added with 2 $\mu$L of NEB 10x cuttersmart buffer and 14 $\mu$L of H$_2$O, and incubated at 95°C in a PCR instrument for 5 minutes, then immediately taken out and incubated on ice for 5 minutes to anneal and form double-stranded DNA with sticky ends.

According to the reaction system in Table 2, the digested plasmid, the annealed primer and DNA Ligation Kit Ver.2.1 (TAKARA, 6022Q) were sequentially added into a PCR instrument and incubated at 16°C for 1 hour to complete the connection between the annealed product and the linearized backbone to obtain Cas13m.3, Cas13m.6, C13-2 plasmids targeting VEGFA RNA (Cas13m.3-gRNA-n, Cas13m.6-gRNA-n, C13-2-gRNA-n, n was a number corresponding to a different guide sequence), which could express Cas13 protein and gRNA targeting VEGFA RNA (including a specific guide sequence and the direct repeat sequence corresponding to the Cas13 protein).

Table 2. Reaction system

| Total reaction volume | 6 μL |
|---|---|
| 2× Solution I | 3 μL |
| Annealed primer | 2 μL |
| Digested plasmid | 1 μL |

3. Plasmid transformation

In the clean bench, all reaction products ligated by T4 ligase were quickly added to a tube (50 μL) of competent cells of *Escherichia coli* Stbl3, and then incubated on ice for 30 minutes. The competent cells were immersed in a water bath at 42°C and heat shocked for 90 seconds, and then put back on ice and incubated for 2 minutes. In the clean bench, 400 μL of LB medium without antibiotics was added, and then the bacterial suspension was placed in a bacterial shaker, cultured and recovered at 37°C and 200 rpm for 45 minutes. During the recovery period, the biochemical incubator was opened and an LB agar plate containing an appropriate amount of ampicillin was placed in the incubator to allow it to dry. The bacterial suspension was centrifuged at 12000 rpm for 1 minute at room temperature, most of the supernatant was removed by aspiration, and about 50 μL was retained and the pellet was fully resuspended. The bacterial suspension was aspirated and dripped to the edge of an LB agar plate containing ampicillin, then streaked across the plate using a pipette tip. The plate was then inverted and placed into the biochemical incubator for an additional 16 hours of cultivation.

4. Identification of positive clones

1-10 μL pipette tips were used within the clean bench to individually pick seven monoclones, which were then transferred into 50 μL of LB medium containing ampicillin. The bacteria were mixed thoroughly with the LB medium by pipetting up and down several times. 2 μL of the bacterial suspension was added to the colony PCR reaction mixture, thoroughly mixed, and briefly centrifuged to collect the liquid at the bottom of the tube, followed by PCR reaction. The remaining bacterial suspension was placed in a biochemical incubator for continued cultivation. After PCR amplification, agarose gel electrophoresis was performed, and clones with correct electrophoresis band size, singleness, and normal brightness were regarded as positive clones. 10 μL of bacterial suspension was sent for Sanger sequencing. The remaining bacterial suspension was used for plasmid extraction. The operation procedure followed the instructions of the Accurate Plasmid Extraction Kit (Accurate Biotech, AG21001). Finally, the plasmid was eluted with 50 μL of Elution Buffer. According to the operation method of Nanodrop double-stranded DNA determination, the concentration of plasmid DNA was determined. One positive clone was selected from each plasmid, and 5-10 μL was provided for Sanger sequencing to screen for plasmids with completely correct sequencing.

III. Detection of editing efficiency

1. Detection of editing efficiency at mRNA levels

The HEK293T cells were transfected in strict accordance with the procedure of Life Tech's Lipofectamine 2000 (Thermo Fisher 11668019) reagent. One day before transfection, 293T cells in good condition were seeded at $1 \times 10^5$ cells per well in a 24-well plate. On the day of transfection, 500 ng of the recombinant Cas13 plasmid targeting VEGFA RNA was respectively added to 50 μL of OPTI-MEM (Thermo, 2120588) in the corresponding volumes, while 1 μL of Lipofectamine 2000 was respectively added to 50 μL of OPTI-MEM medium. After mixing thoroughly, the mixture was left at room temperature for 5 minutes. Subsequently, the diluted plasmid DNA was mixed with Lipofectamine 2000 thoroughly, and left at room temperature for 15 minutes. Then, 100 μL of the plasmid DNA complex was added to each well of the 24-well plate containing cells, and the culture plate was gently shaken to ensure uniform mixing (the negative control group was transfected with Cas13m.3-BsaI, Cas13m.6-BsaI and C13-2-BsaI plasmids). Incubation was carried out at 37°C for 72 hours.

Cells were collected 72 hours after transfection, and total RNA was extracted using the Accurate SteadyPure Universal RNA Extraction Kit AG21017, followed by mRNA concentration measurement via Nanodrop. The mRNA products were reverse transcribed using the Evo M-MLV Mix Kit with gDNA Clean for qPCR AG11728 reverse transcription kit. The reverse transcription products were detected using the SYBR Green Premix Pro Taq HS qPCR Kit (Low Rox Plus) AG11720 qPCR kit.

The primers used for qPCR are shown in Table 3, wherein gVegfa-F1/Vegfa-R1 were used to detect the editing efficiency of gRNA-5, while qVegfa-F2/qVegfa-R2 were used to test the editing efficiencies of all other gRNAs, including gRNA-4 and gRNA-7.

Table 3. qPCR primer sequences

| Primer number | Sequence | Use | SEQ ID |
|---|---|---|---|
| qGAPDHF1 | CCATGGGGAAGGTGAAGGTC | For detecting internal reference genes in qPCR experiments | 22 |
| qGAPDHR1 | GAAGGGGTCATTGATGGCAA C | For detecting internal reference genes in qPCR experiments | 23 |
| qVegfa-F1 | ACCTCCACCATGCCAAGTGG | For detecting *Vegfa* RNA levels | 24 |
| qVegfa-R1 | CAGGGTCTCGATTGGATGGC | For detecting *Vegfa* RNA levels | 25 |
| qVegfa-F2 | CAATGACGAGGGCCTGGAGT | For detecting *Vegfa* RNA levels | 26 |
| qVegfa-R2 | TCTTTGGTCTGCATTCACAT | For detecting *Vegfa* RNA levels | 27 |

The reaction system was configured according to the instructions of the SYBR Green Premix Pro Taq HS qPCR Kit (Low Rox Plus) AG11720, and detection was performed using the QuantStudio™ 5 Real-Time PCR System, 96-well.

2. Calculation method

This experiment used the relative quantitative method, namely the $2^{-\triangle\triangle Ct}$ method, to calculate the changes in target RNA. The calculation method is as follows:

$$\triangle Ct = Ct\ (Vegfa) - Ct\ (GAPDH);$$

$\triangle\triangle Ct = \triangle Ct$ (group to be verified) - $\triangle Ct$ (each negative control group);

$$2^{-\triangle\triangle Ct} = 2^{\wedge}(-\triangle\triangle Ct).$$

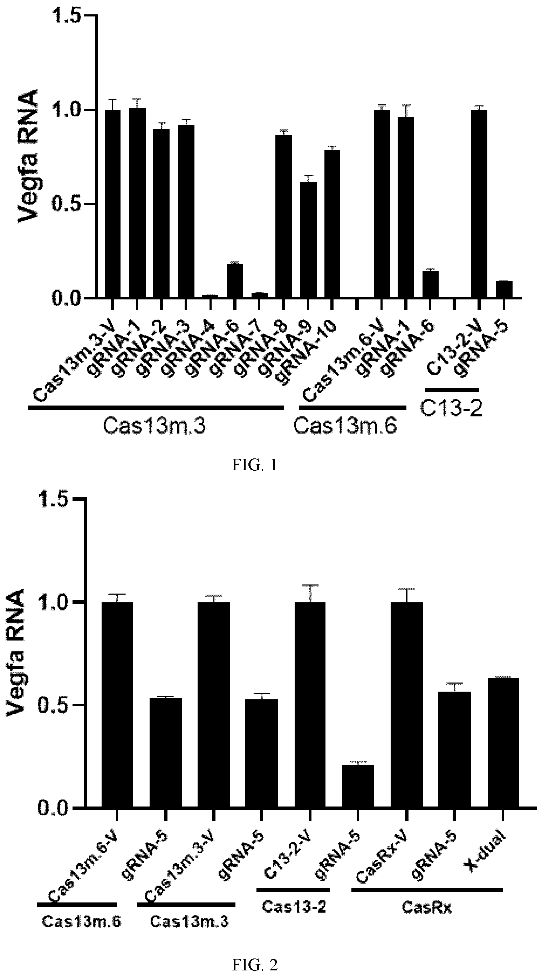

In this example, the biological experiments were independently repeated three times. The relative RNA expression levels of *Vegfa* were calculated according to the above method. The result data were taken as the average value of the three tests, as shown in the following Table 4 and FIG. 1.

Table 4. Editing efficiency of different combinations of Cas13 and gRNA

| Group (transfection with different plasmids) | *VEGFA* RNA level (%) | Editing efficiency (%) |
|---|---|---|
| Cas13m.3-V (Cas13m.3-BsaI) | 100.0 | 0 |
| Cas13m.3-gRNA-1 | 100.9 | 0 |
| Cas 13m.3-gRNA-2 | 89.9 | 10.1 |
| Cas 13m.3-gRNA-3 | 91.8 | 8.2 |
| Cas 13m.3-gRNA-4 | 1.6 | 98.4 ** |
| Cas 13m.3-gRNA-6 | 18.3 | 81.7 ** |
| Cas 13m.3-gRNA-7 | 2.7 | 97.3 ** |
| Cas 13m.3-gRNA-8 | 86.5 | 13.5 * |
| Cas 13m.3-gRNA-9 | 61.7 | 38.3 ** |
| Cas13m.3-gRNA-10 | 78.8 | 21.2 ** |
| Cas13m.6-V (Cas13m.6-BsaI) | 100.0 | 0.0 |
| Cas13m.6-gRNA-1 | 96.3 | 3.7 |
| Cas 13m.6-gRNA-6 | 14.2 | 85.8 ** |
| C13-2-V (C13-2-BsaI) | 100 | 0 |

(continued)

| Group (transfection with different plasmids) | *VEGFA* RNA level (%) | Editing efficiency (%) |
|---|---|---|
| C13-2-gRNA-5 | 9 | 91 ** |
| Note: "**" indicates p<0.01 compared with the corresponding negative control group, and "*" indicates p<0.05 compared with the corresponding negative control group. | | |

As can be seen from the above table, gRNA-2 to gRNA-10 designed by the inventor can edit *VEGFA* RNA. Compared with gRNA-1, the editing efficiency of gRNA-2 to gRNA-10 is improved, with the highest efficiency observed in gRNA-4, gRNA-5, gRNA-6 and gRNA-7, followed by gRNA-9 and gRNA-10.

**Example 2: Gene editing using different combinations of Cas13 proteins and gRNA-5**

Construction of overexpression vector

Plasmids Cas13m.3-gRNA-5, Cas13m.6-gRNA-5 and C13-2-gRNA-5 targeting VEGFA RNA were constructed using the method described in Example 1. These plasmids were capable of expressing Cas13 protein and gRNA-5 targeting VEGFA RNA (containing the guide sequence as shown in SEQ ID NO: 13, as well as the corresponding direct repeat sequence of Cas13 protein). Cas13m.3-BsaI, Cas13m.6-BsaI and C13-2-BsaI vectors of Example 1 were used as negative controls.

The prior art (CN112143701A, CN112430597A) reports a CasRx+gRNA combination (gRNAs containing different guide sequences or their tandem combinations: X-1, X-2, X-dual [*i.e.,* X-1+X-2], RJ-2, RJ-5, RJ-8, RJ-5+RJ-8 [*i.e.,* RJ-5+8]) targeting VEGFA RNA. The guide sequences are shown in Table 5.

CasRx and its corresponding gRNA plasmids (purchased from Miaoling, pXR001: EF1a-CasRx-2A-EGFP and pXR004: CasRx pre-gRNA cloning backbone) were ordered and modified to obtain pAAV-CasRx-gRNA plasmid (also known as CasRx-V, SEQ ID NO: 28). Subsequently, following the method described in Example 1, the annealed products of the forward and reverse primers (primers with restriction enzyme cutting sites, *i.e.,* the sequences in Table 5 below were modified by adding AAAC to the 5' end to form the forward primer, and the reverse complement sequence of the sequences in Table 5 was modified by adding cttg to the 5' end to form the reverse primer) corresponding to the prior art control gRNA and the guide sequence of the gRNA-5 of the present disclosure were linked into the BpiI-digested backbone of pAAV-CasRx-gRNA plasmid. The resulting construct was transformed into competent *E. coli,* cultured in a culture medium containing the appropriate antibiotic, and subjected to colony PCR reaction. Positive clones were screened via electrophoresis, and plasmids were extracted. Sanger sequencing was performed for verification, thereby obtaining CasRx plasmid targeting VEGFA RNA, which could be used to express CasRx and gRNA (such as X-1 gRNA and/or X-2 gRNA).

Table 5. gRNA related sequences

| Name | gRNA related sequences | SEQ ID |
|---|---|---|
| Guide sequence of RJ-2 | GCAGCCTGGGACCACTTGGCATGGTGGAG | 29 |
| Guide sequence of RJ-5 | CCTGGAAGATGTCCACCAGGGTCTCGATTG | 30 |
| Guide sequence of RJ-8 | ATGTTGGACTCCTCAGTGGGCACACACTCC | 31 |
| RJ-5+RJ-8 in tandem | CCTGGAAGATGTCCACCAGGGTCTCGATTGcaag taaaccccctaccaactggtcggggtttgaaacATGTTGGACTCCT CAGTGGGCACACACTCC | 32 |
| Guide sequence of X-1 | GTGCTGTAGGAAGCTCATCTCTCCTATGTG | 33 |
| Guide sequence of X-2 | GGTACTCCTGGAAGATGTCCACCAGGGTCT | 34 |
| X-dual (X-1+X-2 in tandem) | GGTACTCCTGGAAGATGTCCACCAGGGTCTcaag taaaccccctaccaactggtcggggtttgaaacGTGCTGTAGGAA GCTCATCTCTCCTATGTG | 35 |

The plasmid was transfected into HEK293T cells using the same method as described in Example 1. The negative control group was transfected with CasRx-V plasmid.

Total RNA was extracted 72 hours after transfection and reverse transcribed into cDNA. The editing efficiency at the mRNA level was detected using qPCR technology, following the same method as described in Example 1. The primers involved are shown in Table 6 below. In this example, the biological experiments were independently repeated three times. The detection results are shown in FIG. 2 and Tables 7-8 below, with the result data being the average of three tests. It can be seen that the editing efficiencies of the combination of different Cas13 proteins and gRNA-5 are all higher than that of the control group CasRx-X-dual (dual gRNA).

Table 6. qPCR primer sequences

| Primer number | Use | SEQ ID |
|---|---|---|
| qGAPDHF1 | For detecting internal reference genes in qPCR experiments | 22 |
| qGAPDHR1 | For detecting internal reference genes in qPCR experiments | 23 |
| qVegfa-F1 | For detecting Vegfa RNA levels | 24 |
| qVegfa-R1 | For detecting Vegfa RNA levels | 25 |

Table 7. Editing efficiency of combinations of different tools and gRNA-5

| Group (transfection with different plasmids) | VEGFA RNA level (%) | Editing efficiency (%) |
|---|---|---|
| Cas13m.6-V (Cas13m.6-BsaI) | 100.0 | 0.0 |
| Cas13m.6-gRNA-5 | 53.4 | 46.6 ** |
| Cas13m.3-V (Cas13m.3-BsaI) | 100.0 | 0.0 |
| Cas 13m.3-gRNA-5 | 52.8 | 47.2 ** |
| C13-2-V (C13-2-BsaI) | 100.0 | 0.0 |
| C13-2-gRNA-5 | 20.8 | 79.2 ** |
| CasRx-V (pAAV-CasRx-gRNA) | 100.0 | 0.0 |
| CasRx-gRNA-5 | 56.7 | 43.3 ** |
| CasRx-X-dual | 62.9 | 37.1 ** |
| Note: "**" indicates p<0.01 compared with the corresponding negative control group. | | |

Table 8. P value of the difference test between combinations of different Cas13 and gRNA in this example (t test)

| Cas13-gRNA combination | CasRx-X-dual | CasRx-gRNA-5 |
|---|---|---|
| Cas13m.6-gRNA-5 | <0.001 | 0.23 |
| Cas 13m.3-gRNA-5 | <0.01 | 0.24 |
| C13-2-gRNA-5 | <0.001 | <0.001 |

**Example 3: Gene editing using combinations of different Cas13 proteins with gRNA-4, gRNA-6 and gRNA-7**

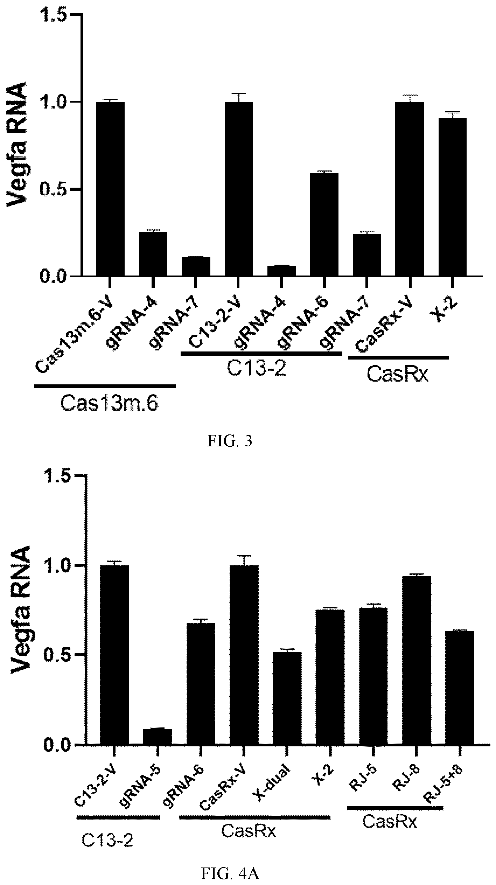

The plasmids of combinations of gRNA-4, gRNA-6 and gRNA-7 with different Cas13 proteins (Cas13m.6 and C13-2) were prepared using the method described in Example 1. These plasmids were capable of expressing Cas13 and gRNA (containing specific guide sequences as well as direct repeat sequences corresponding to the Cas13 protein). The CasRx-V plasmid and CasRx-X-2 plasmid (which can be used to express CasRx and X-2 gRNA) were prepared using the method described in Example 2. The plasmid was transfected into 293T cells, and then the total RNA of the cells was extracted 72 hours after transfection and reverse transcribed into cDNA. The qPCR detection method was the same as the method in Example 1. The primer sequences used in this example are shown in Table 9. The biological experiments were independently repeated three times, and the detection results are shown in FIG. 3 and Tables 10-11 below, with the result data being the average of three tests. The combination of gRNA-4 and gRNA-7 with different Cas13 proteins can significantly downregulate VEGFA RNA levels, with editing efficiencies significantly better than that of the control CasRx-X-2.

Table 9. Primer sequences

| Primer number | Use | SEQ ID |
|---|---|---|
| qGAPDHF1 | For detecting internal reference genes in qPCR experiments | 22 |
| qGAPDHR1 | For detecting internal reference genes in qPCR experiments | 23 |
| qVegfa-F2 | For detecting *Vegfa* RNA levels | 26 |
| qVegfa-R2 | For detecting *Vegfa* RNA levels | 27 |

Table 10. Editing efficiency of different tool combinations

| Group (transfection with different plasmids) | *VEGFA* mRNA level (%) | Editing efficiency (%) |
|---|---|---|
| Cas13m.6-V (Cas13m.6-BsaI) | 100.0 | 0.0 |
| Cas13.m6-gRNA-4 | 25.7 | 74.3 ** |
| Cas13.m6-gRNA-7 | 11.1 | 88.9 ** |
| C13-2-V (C13-2-BsaI) | 100.0 | 0.0 |
| C13-2-gRNA-4 | 6.0 | 94.0 ** |
| C13-2-gRNA-6 | 59.2 | 40.8 ** |
| C13-2-gRNA-7 | 24.5 | 75.5 ** |
| CasRx-V | 100.0 | 0.0 |
| CasRx-X-2 | 90.8 | 9.2 * |
| Note: "**" indicates p<0.01 compared with the corresponding negative control group, and "*" indicates p<0.05 compared with the corresponding negative control group. | | |

Table 11. P value of the difference test between combinations of different Cas 13 and gRNA in this example (t test)

| Cas13-gRNA combination | CasRx-X-2 |
|---|---|
| Cas13.m6-gRNA-4 | <0.001 |
| Cas13.m6-gRNA-7 | <0.001 |
| C13-2-gRNA-4 | <0.001 |
| C13-2-gRNA-6 | <0.001 |
| C13-2-gRNA-7 | <0.001 |

**Example 4: Comparison of editing efficiency at RNA and protein levels with the prior art**

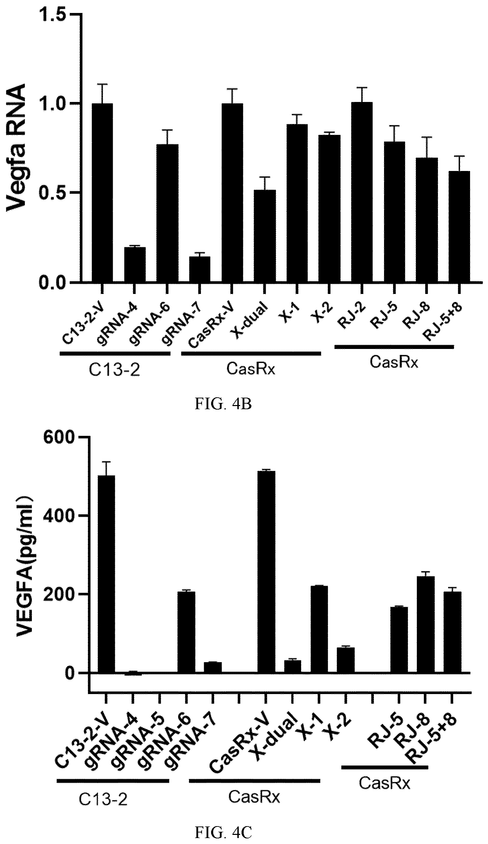

The Cas13-gRNA plasmid designed by the inventor and the CasRx-gRNA plasmid of the prior art were transfected in the same batch to compare the editing efficiency. The plasmids used in this example (for expressing Cas13 protein and gRNA [single or paired gRNA]) were prepared using the methods described in Example 1 and Example 2. The plasmid was transfected into 293T cells, and total RNA was extracted 72 hours after transfection. The cDNA was obtained through reverse transcription, and qPCR detection methods were the same as those in Example 1, and the primers are shown in Table 12. The biological experiments were independently repeated three times, and the detection results are shown in FIGs. 4A-4B and Tables 13-16, with the result data being the average of three tests. The primers used in Table 13 and FIG. 4A were qGAPDHF1/qGAPDHR1 and qVegfa-F1/qVegfa-R1, with the P-values for intergroup difference tests shown in Table 14. The primers used in Table 15 and FIG. 4B were qGAPDHF1/qGAPDHR1 and qVegfa-F2/qVegfa-R2, with the P-values for intergroup difference tests shown in Table 16. The combination of CasRx and dual gRNA in the prior art had higher editing efficiency than the combination of CasRx and single gRNA. Compared with the combination of CasRx and single gRNA and the combination of CasRx and dual gRNA in the prior art, the combination of C13-2 with gRNA-4, gRNA-5 and gRNA-7 significantly improved the editing efficiency of VEGFA RNA levels.

While collecting cell pellets to extract total RNA, cell supernatants were collected for ELISA to detect changes in the level of native VEGFA protein. The Human VEGF-A (Vascular Endothelial Cell Growth Factor A) ELISA Kit from Elascience was used, strictly adhering to the kit's operating procedures. Standards with different concentrations were set, and samples were processed. Final data were read at 450 nm using a microplate reader (MultiskanFC from Thermo Fisher). The corresponding formula between sample concentration and OD value was calculated based on the OD value of the standard. The experiment was repeated twice. The results are shown in Table 17 and FIG. 4C, showing that compared with the prior art (e.g., CasRx-RJ-5, CasRx-RJ-8 and CasRx-RJ-5+8), the downregulation levels of the VEGFA protein edited by the combinations of C13-2 with gRNA-4, gRNA-5 and gRNA-7 are significantly increased.

Table 12. Primer sequences

| Primer number | Use | SEQ ID |
|---|---|---|
| qGAPDHF1 | For detecting internal reference genes in qPCR experiments | 22 |
| qGAPDHR1 | For detecting internal reference genes in qPCR experiments | 23 |
| qVegfa-F1 | For detecting *Vegfa* RNA levels | 24 |
| qVegfa-R1 | For detecting *Vegfa* RNA levels | 25 |
| qVegfa-F2 | For detecting *Vegfa* RNA levels | 26 |
| qVegfa-R2 | For detecting *Vegfa* RNA levels | 27 |

Table 13. Comparison of editing efficiency at RNA levels (FIG. 4A)

| Group (transfection with different plasmids) | *VEGFA* RNA level (%) | Editing efficiency (%) |
|---|---|---|
| C13-2-V | 100.0 | 0.0 |
| C13-2-gRNA-5 | 9.1 | 90.9 ** |
| C13-2-gRNA-6 | 67.7 | 32.3 ** |
| CasRx-V | 100.0 | 0.0 |
| CasRx-X-dual | 51.7 | 48.3 ** |
| CasRx-RJ-5 | 76.4 | 23.6 ** |
| CasRx-RJ-8 | 94.1 | 5.9 |
| CasRx-RJ-5+8 | 63.1 | 36.9 ** |
| Note: "**" indicates $p<0.01$ compared with the corresponding negative control group, and "*" indicates $p<0.05$ compared with the corresponding negative control group. | | |

Table 14. P value of mRNA level difference test between combinations of different Cas13 and gRNA (t test)

| Cas13-gRNA combination | CasRx-X-dual | CasRx-RJ-5+8 |
|---|---|---|
| C13-2-gRNA5 | <0.001 | <0.001 |

Table 15. Comparison of editing efficiency at RNA levels (FIG. 4B)

| Group (transfection with different plasmids) | *VEGFA* RNA level (%) | Editing efficiency (%) |
|---|---|---|
| C13-2-V | 100.0 | 0.0 |
| C13-2-gRNA-4 | 19.9 | 80.1 ** |
| C13-2-gRNA-6 | 77.1 | 22.9 * |
| C13-2-gRNA-7 | 14.5 | 85.5 ** |
| CasRx-V | 100.0 | 0.0 |
| CasRx-X-dual | 51.6 | 48.4 ** |
| CasRx-X-1 | 88.4 | 11.6 |
| CasRx-X-2 | 82.4 | 17.6 ** |
| RJ-2 | 100.9 | 0 |
| RJ-5 | 78.7 | 21.3 |

(continued)

| Group (transfection with different plasmids) | *VEGFA* RNA level (%) | Editing efficiency (%) |
|---|---|---|
| RJ-8 | 69.7 | 30.3 |
| CasRx-RJ-5+8 | 62.3 | 37.7 ** |
| Note: "**" indicates p<0.01 compared with the corresponding negative control group, and "*" indicates p<0.05 compared with the corresponding negative control group. | | |

Table 16. P value of mRNA level difference test between combinations of different Cas13 and gRNA (t test)

| Cas13-gRNA combination | CasRx-X-dual | CasRx-RJ-5+8 |
|---|---|---|
| C13-2-gRNA4 | <0.01 | <0.001 |
| C13-2-gRNA7 | <0.01 | <0.001 |

Table 17. Comparison of knockdown efficiency at protein levels (FIG. 4C)

| Group (transfection with different plasmids) | *VEGFA* protein level (%) | Protein downregulation efficiency (%) |
|---|---|---|
| C13-2-V | 100.0 | 0.0 |
| C13-2-gRNA4 | 0.8 | 99.2 ** |
| C13-2-gRNA5 | 0.0 | 100.0 ** |
| C13-2-gRNA6 | 41.1 | 58.9 ** |
| C13-2-gRNA7 | 5.5 | 94.5 ** |
| CasRx-V | 100.0 | 0.0 |
| CasRx-X-dual | 6.3 | 93.7 ** |
| CasRx-X-1 | 43.0 | 57.0 ** |
| CasRx-X-2 | 12.8 | 87.2 ** |
| CasRx-RJ-5 | 32.6 | 67.4 ** |
| CasRx-RJ-8 | 47.9 | 52.1 ** |
| CasRx-RJ-5+8 | 40.2 | 59.8 ** |
| Note: The VEGFA protein levels of the negative control C13-2-V and CasRx-V groups are all set as 100%, and the data of other groups are calculated based on their respective negative controls. "**" indicates p<0.01 compared with the corresponding negative control group. | | |

**Example 5: Editing in mice using AAV vectors**

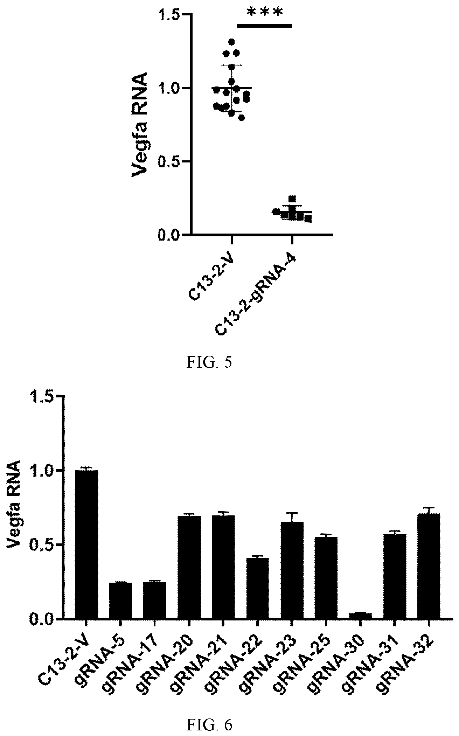

The C13-2-gRNA-4 plasmid constructed in Example 1 was outsourced for packaging into AAV8 WT virus (for the expression of C13-2 and gRNA-4). Three plasmids were transfected into 293T cells to produce AAV, and AAV was obtained by gradient centrifugation for purification. The mice were anesthetized by intraperitoneal injection of 2.5% tribromoethanol (100 $\mu$L-200 $\mu$L/10 g). Compound tropicamide eye drops (Mydrin-P) were sequentially dripped to the eyes to dilate the pupils. After the pupils were fully dilated, proparacaine hydrochloride eye drops (Alcon) were dripped for local anesthesia. One minute after anesthesia, the eye drops were wiped off, and the medical sodium hyaluronate gel of Yishukang was evenly applied to the mouse's eyes. Under an ophthalmic microscope, a 30G sharp needle was first used to puncture the sclera near the corneal edge, followed by the use of a WPI 10 $\mu$L injection system (Nanofil-10 $\mu$L) connected to a 35G flat needle containing the drug. AAV8-C13-2-gRNA-4 was subretinally injected into the mouse's eye (the negative control group was injected with the WT serotype virus AAV8-C13-2-V, which was packaged from the above C13-2-BsaI plasmid and could express C13-2 and gRNA that did not target the mammalian genome). Finally, tobramycin-dexamethasone ointment was applied to the eyes, and the mice were kept warm at 37°C. After the mice were awakened, the mice were returned to their cages for further rearing and observation. The mice were euthanized by cervical dislocation 4 weeks after injection, and their eyeballs were enucleated. The retinas were rapidly and gently dissected, then immediately immersed in Trizol. Total RNA extraction was performed strictly following Trizol (Thermo Fisher). After RNA extraction, cDNA was obtained via reverse transcription using the same method as described in Example 1. The expression changes and editing efficiency of VEGFA in mice were assessed by qPCR using mouse VEGFA-specific primers. The results are shown in FIG. 5 and Table 19 below. After 4 weeks of AAV8-C13-2-gRNA-4 injection, VEGFA transcription levels are significantly downregulated, with an editing efficiency as high as 84.5%. The primers required for qPCR are shown in Table 18 below.

Table 18. qPCR primers

| Primer number | Primer sequence | SEQ ID NO |
|---|---|---|
| qmVA-F | CTTCAAGCCGTCCTGTGTGC | 36 |
| qmVA-R | GCTCATCTCTCCTATGTGCT | 37 |
| mGAPDH-F | GCTGAGTATGTCGTGGAGTCTA | 38 |
| mGAPDH-R | GTGGTTCACACCCATCACAA | 39 |

Table 19. Editing efficiency of C13-2-gRNA4 in mice

| Sample number | *VEGFA* mRNA level (%) | Editing efficiency (%) | P value |
|---|---|---|---|
| C13-2-V (C13-2-BsaI) | 100.0 | 0.0 | - |
| C13-2-gRNA-4 | 15.5 | 84.5 | <0.0001 |

**Example 6: Off-target detection**

A total of seven plasmids, C13-2-V, C13-2-gRNA-4, C13-2-gRNA-5, C13-2-gRNA-6, C13-2-gRNA-7, CasRx-V and CasRx-X-dual, were constructed according to the methods described in Example 1 and Example 2, and transfected into 293T cells, with three replicate wells being transfected for each plasmid.

After 72 hours of transfection, total RNA was extracted from the cells and sent to the sequencing company. All samples uniformly used 1 $\mu$g for ribosomal RNA removal, followed by cDNA synthesis via RT reverse transcription. The cDNA was randomly fragmented, subjected to end repair and A-tailing, and then PCR-amplified to construct the library. The library was quality-controlled before being sequenced on the DNBSEQ (BGI Genomics) platform with a PE150 read length. The raw sequencing data were aligned to the human reference genome using the STAR software after quality control and filtering. Gene expression levels were quantified by StringTie2, followed by significance of difference analysis using DESeq2 software (compared to their respective negative control groups). Padj (corrected P value) ≤0.05 and expression fold ratio ≥2 (log2(fold change) >1) were used as significant difference criteria, and genes that met this condition were identified as differentially expressed genes (DEGs).

The number of differentially expressed genes (DEGs) in each sample group is shown in Table 20, wherein the number of differentially expressed genes in each C13-2 experimental group is significantly less than that in the CasRx-X-dual group; compared with the CasRx-X-dual group, the editing of the combination of C13-2 and each gRNA in the table causes less perturbation to the transcriptome, and therefore is safer.

Table 20. Differentially expressed genes

| Group | Negative control | Number of downregulated DEGs | Number of upregulated DEGs | Total number of upregulated or downregulated DEGs |
|---|---|---|---|---|
| C13-2-gRNA-4 | C13-2-V | 8 | 7 | 15 |
| C13-2-gRNA-5 | C13-2-V | 10 | 42 | 52 |
| C13-2-gRNA-6 | C13-2-V | 13 | 8 | 21 |
| C13-2-gRNA-7 | C13-2-V | 9 | 26 | 35 |
| CasRx-X-dual | CasRx-V | 245 | 248 | 493 |

The differentially expressed genes were then intersected with the predicted targeted gene sets.

Off-target prediction method: Prediction was performed in the whole genome and whole cDNA sequences of the target species (Homo sapiens) by the EMBOSS-water program, setting the parameters gap_open=10.0;gap_extend=0.5, and using the sense and antisense strands of the gRNA guide sequences for comparison, taking the minimum matching bases of not less than 18 bases after comparison, and the genes corresponding to transcripts with no more than 6 bases of mismatch+gap were taken as predicted target genes.

It can be seen from the number after taking the intersection in Table 21 that compared with CasRx-X-dual, the number of off-target genes in the combination of C13-2 and each gRNA in the table is significantly reduced, so the off-target safety is higher.

Table 21. Predicted target genes and their intersection with differentially expressed genes

| Group | Total number of predicted target genes | Number of upregulated DEGs intersecting with predicted target genes | Number of downregulated DEGs intersecting with predicted target genes * | Number of DEGs intersecting with predicted target genes |
|---|---|---|---|---|
| C13-2-gRNA-4 | 1391 | 2 | 0 | 2 |
| C13-2-gRNA-5 | 3516 | 11 | 2 | 13 |
| C13-2-gRNA-6 | 513 | 0 | 3 | 3 |
| C13-2-gRNA-7 | 613 | 1 | 0 | 1 |
| CasRx-X-dual | 2407 | 21 | 31 | 52 |
| Note: * indicates that the target gene VEGFA has been deleted. | | | | |

As can be seen from the above examples, gRNA-4, gRNA-5 and gRNA-7 are simultaneously superior to the prior art in terms of editing efficiency and off-target safety, thus achieving unexpected technical effects.

**Example 7: Editing efficiency of some gRNAs at RNA and protein levels**

The sense and antisense chains of the DNA sequences corresponding to the guide sequences of gRNA-11 to gRNA-45 were synthesized using the method of Example 1 (the sense chain was obtained by adding agac to the 5'-end of the guide sequence, and the antisense chain was obtained by adding aaaa to the 5'-end of the reverse complement sequence of the guide sequence), and the annealed product was ligated with the linearized C13-2-BsaI plasmid digestion product via T4 ligase; after plasmid transformation and positive clone identification, C13-2 and plasmid vectors of each gRNA targeting VEGFA were obtained; the plasmid was transfected into HEK293T cells, and total RNA was extracted 72 h after transfection and reverse transcribed into cDNA, and the editing efficiency was detected by qPCR.

The primers used for qPCR are shown in Table 22 below, wherein qVegfa-F1/qVegfa-R1 are used to detect the editing efficiency of gRNA-5, 17, 20, 21, 22 and 23; qVegfa-F2/qVegfa-R2 are used to test the editing efficiency of gRNA-25, 26, 27, 28, 29, 30, 31 and 32.

Table 22. qPCR primer sequences

| Primer number | Sequence | Use | SEQ ID NO |
|---|---|---|---|
| qGAPDHF1 | TCCAAAATCAAGTGGGGCGA | For detecting internal reference genes in qPCR experiments | 231 |
| qGAPDHR1 | TGATGACCCTTTTGGCTCCC | For detecting internal reference genes in qPCR experiments | 232 |
| qVegfa-F1 | ACCTCCACCATGCCAAGTGG | For detecting *Vegfa* RNA levels | 24 |
| qVegfa-R1 | CAGGGTCTCGATTGGATGGC | For detecting *Vegfa* RNA levels | 25 |
| qVegfa-F2 | CAATGACGAGGGCCTGGAGT | For detecting *Vegfa* RNA levels | 26 |

(continued)

| Primer number | Sequence | Use | SEQ ID NO |
|---|---|---|---|
| qVegfa-R2 | TCTTTGGTCTGCATTCACAT | For detecting *Vegfa* RNA levels | 27 |

The relative quantitative method, namely the $2^{-\triangle\triangle Ct}$ method, was used to calculate the changes in target RNA. The biological experiments were independently repeated three times with the result data being the average of three tests. As shown in Table 23 and FIG. 6.

Table 23. Editing efficiency of different gRNAs

| Sample number | *Vegfa* RNA level after editing (%) | Editing efficiency (%) |
|---|---|---|
| C13-2-V (C13-2-BsaI) | 100% | 0% |
| gRNA-5 | 24% | 76% |
| gRNA-17 | 25% | 75% |
| gRNA-20 | 69% | 31% |
| gRNA-21 | 70% | 30% |
| gRNA-22 | 41% | 59% |
| gRNA-23 | 65% | 35% |
| gRNA-25 | 55% | 45% |
| gRNA-30 | 4% | 96% |
| gRNA-31 | 57% | 43% |
| gRNA-32 | 71% | 29% |

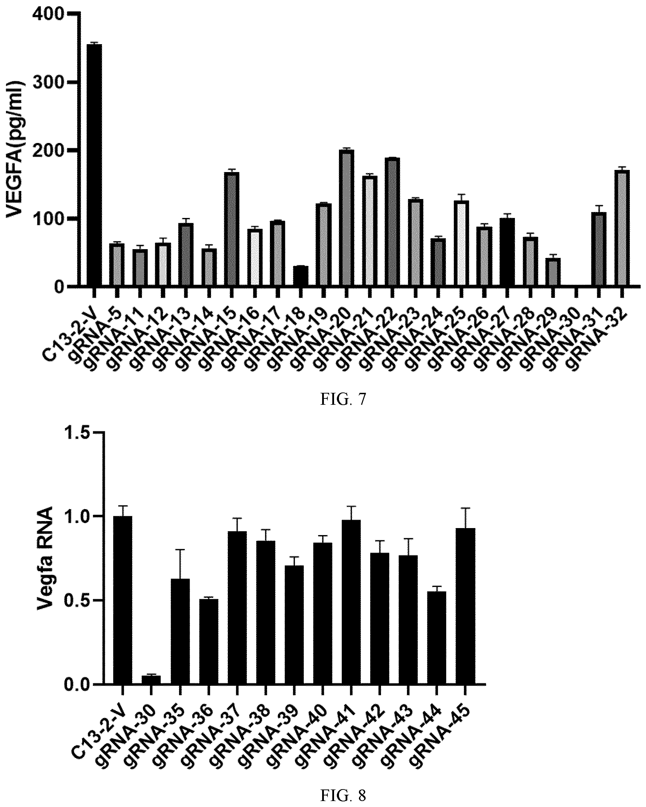

After 72 hours of plasmid transfection, the cell pellet was used for RNA extraction and qPCR detection of RNA levels, while the cell supernatant was used to detect changes in protein levels. The cells were observed to be relatively uniform in growth and cell density, and then an equal volume of cell culture medium was collected and centrifuged at 14000 rpm for 15 min at 4°C to remove dead cells and cell debris. Finally, an equal volume of cell supernatant was taken for Elisa detection. The procedure of the Human VEGFA ELISA kit (E-EL-H0111c) of Elabscience was strictly followed, and two replicate wells were set for each sample. Different concentration standards were set and the samples were treated. The final data were read at 450 nm using a microplate reader (MultiskanFC, Thermo Fisher). A standard curve was constructed based on the concentrations and OD values of the standards, and the concentration corresponding to each sample's OD value was calculated using the corresponding formula. The test results are shown in Table 24 below and FIG. 7. As can be seen from Table 24, compared with the negative control C13-2-V group, the VEGFA protein level is significantly downregulated after editing with gRNA-30, and the VEGFA protein level is also significantly downregulated after editing with gRNA-5, 11, 12, 14, 18, 24, 26, 27, 28 and 29, which is consistent with the trend of downregulation at the RNA level. The VEGFA protein level in the gRNA-30 group after editing is significantly lower than that in the other groups (P<0.01). The VEGFA protein level in the gRNA-18 group after editing is significantly lower than that in the gRNA-5 group (P<0.01). Therefore, an unexpected technical effect is achieved.

Table 24. Protein downregulation after editing with different gRNAs

| Sample number | Total amount of VEGFA protein after editing (pg/mL) | VEGFA protein downregulation rate after editing (%) |
|---|---|---|
| C13-2-V (C13-2-BsaI) | 355.73 | 0.00 |
| gRNA-5 | 64.00 | 82.01 |
| gRNA-11 | 55.73 | 84.33 |
| gRNA-12 | 64.70 | 81.81 |
| gRNA-13 | 93.73 | 73.65 |
| gRNA-14 | 56.77 | 84.04 |

(continued)

| Sample number | Total amount of VEGFA protein after editing (pg/mL) | VEGFA protein downregulation rate after editing (%) |
|---|---|---|
| gRNA-15 | 168.63 | 52.60 |
| gRNA-16 | 84.63 | 76.21 |
| gRNA-17 | 96.43 | 72.89 |
| gRNA-18 | 30.63 | 91.39 |
| gRNA-19 | 122.80 | 65.48 |
| gRNA-20 | 200.63 | 43.60 |
| gRNA-21 | 162.73 | 54.25 |
| gRNA-22 | 189.67 | 46.68 |
| gRNA-23 | 128.57 | 63.86 |
| gRNA-24 | 71.67 | 79.85 |
| gRNA-25 | 126.67 | 64.39 |
| gRNA-26 | 88.07 | 75.24 |
| gRNA-27 | 100.87 | 71.65 |
| gRNA-28 | 72.77 | 79.54 |
| gRNA-29 | 43.00 | 87.91 |
| gRNA-30 | 0.30 | 99.92 |
| gRNA-31 | 109.57 | 69.20 |
| gRNA-32 | 171.67 | 51.74 |

## Example 8: Targeting regions near VEGFA RNA splicing sites

The vector was constructed using the method of Example 1 and edited in HEK293T cells. The results are shown in FIG. 8 and Table 25.

gRNA-30 still exhibits the highest editing efficiency; the editing efficiency of gRNA-35, gRNA-36 and gRNA-44 targeting regions near splicing sites is relatively high, while the editing efficiency of other gRNAs is relatively low.

Table 25. RNA downregulation after editing with different gRNAs

| Sample number | *Vegfa* RNA level after editing (%) | Editing efficiency (%) |
|---|---|---|
| C13-2-V (C13-2-BsaI) | 100% | 0% |
| gRNA-30 | 5% | 95% |
| gRNA-35 | 63% | 37% |
| gRNA-36 | 51% | 49% |
| gRNA-37 | 91% | 9% |
| gRNA-38 | 86% | 14% |
| gRNA-39 | 71% | 29% |
| gRNA-40 | 84% | 16% |
| gRNA-41 | 98% | 2% |
| gRNA-42 | 78% | 22% |
| gRNA-43 | 77% | 23% |
| gRNA-44 | 55% | 45% |

(continued)

| Sample number | *Vegfa* RNA level after editing (%) | Editing efficiency (%) |
|---|---|---|
| gRNA-45 | 93% | 7% |

## Example 9: Targeted editing of adjacent regions of the gRNA-30 target sequence

Multiple gRNAs adjacent to the target sequence of gRNA-30 were selected to test editing efficiency.

Using the method of Example 1, the sense and antisense strands of the DNA sequences corresponding to the guide sequences of gRNA-185 to gRNA-196 were synthesized, and the annealed product was ligated with the linearized C13-2-BsaI digestion vector via T4 ligase; after plasmid transformation and positive clone identification, a plasmid vector was obtained, which can express C13-2 and each gRNA. The plasmid was transfected into HEK293T cells, and total RNA was extracted and reverse transcribed into cDNA 72 h after transfection, and the editing efficiency was detected by qPCR.

The following primers are used:

qGAPDHF 1: TCCAAAATCAAGTGGGGCGA (SEQ ID NO: 231)
qGAPDHR1: TGATGACCCTTTTGGCTCCC (SEQ ID NO: 232)
qVEGFA-F2: CAATGACGAGGGCCTGGAGT (SEQ ID NO: 26)
qVEGFA-R2: TCTTTGGTCTGCATTCACAT (SEQ ID NO: 27)

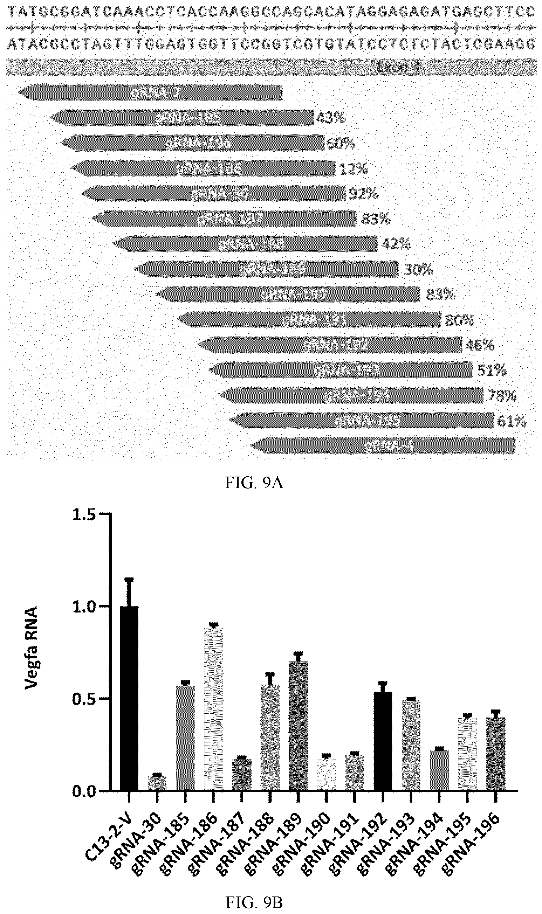

The results are shown in FIG. 9A, FIG. 9B and Table 26. The editing efficiency of gRNA-30 targeting the VEGFA exon 4 region is the highest, which is significantly higher than that of gRNA-185 to gRNA-196 in all experimental groups (P<0.01). Although the editing efficiency of gRNA-187, which differs by only one base, is also relatively high, it is significantly lower than that of gRNA-30 (P<0.01). The editing efficiency of gRNA-186, which differed by only one base, is even only 12%. While the editing efficiencies of gRNA-190, gRNA-191 and gRNA-194 exceed 70%, but still significantly lower than gRNA-30 (P<0.01). Therefore, gRNA-30 achieves unexpected technical effects.

Table 26. Editing efficiency of gRNA-30 and nearby gRNAs

| Sample number | VEGFA mRNA level (%) | Editing efficiency (%) |
|---|---|---|
| C13-2-V (C13-2-BsaI) | 100 | 0 |
| gRNA-30 | 8 | 92 |
| gRNA-185 | 57 | 43 |
| gRNA-186 | 88 | 12 |
| gRNA-187 | 17 | 83 |
| gRNA-188 | 58 | 42 |
| gRNA-189 | 70 | 30 |
| gRNA-190 | 17 | 83 |
| gRNA-191 | 20 | 80 |
| gRNA-192 | 54 | 46 |
| gRNA-193 | 49 | 51 |
| gRNA-194 | 22 | 78 |
| gRNA-195 | 39 | 61 |
| gRNA-196 | 40 | 60 |

## Example 10: Reducing intron enrichment

The CRISPR-Cas13 system was targeted at the VEGFA RNA exon region, and whether novel transcripts were generated, thereby affecting the safety of *in vivo* therapy, remained a subject worthy of further investigation. Therefore, plasmids expressing the CRISPR-Cas13 system (Cas13 and gRNA) were transfected into HEK293T cells, followed by RNA-seq sequencing to analyze changes at the transcriptome level.

The same methods as in Example 1 and Example 2 were used to construct plasmids. The plasmids were transfected into HEK 293T cells, and total RNA was extracted. Gene sequencing companies were commissioned to build libraries through RNA-seq using the method of removing ribosomal RNA, and sequenced with a DNBSEQ-T7 sequencer from BGI Genomics. The data were analyzed using STAR and kallisto. All valid reads of the RNA-seq sequencing data were mapped to the human reference genome using software, and then the IGV software was used to view the distribution of reads in the genome range of the target VEGFA.

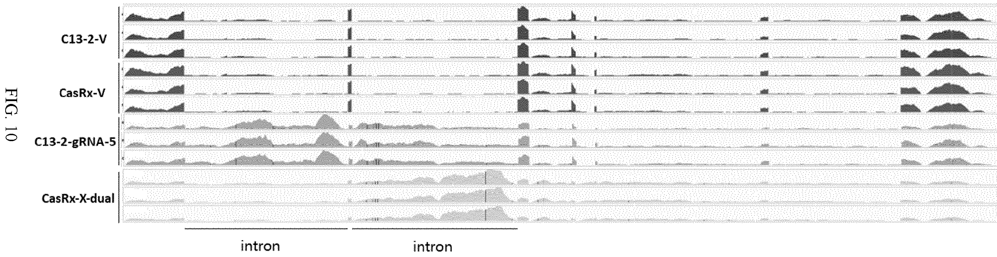

As shown in FIG. 10, in the C13-2-gRNA-5 group and the CasRx-X-dual group after editing, a large number of reads were enriched at introns, and long sequences suspected to be novel transcripts (containing multiple exon and intron sequences) were spliced and obtained. Although the reads at these introns did not generate new transcripts that could encode functional proteins, they might also regulate *in vivo* growth and development in the form of long non-coding RNAs, presenting certain safety concerns.

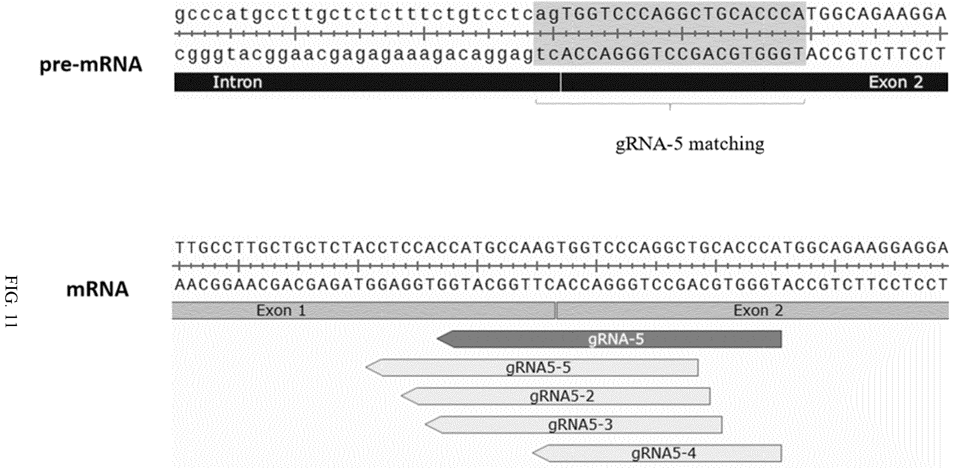

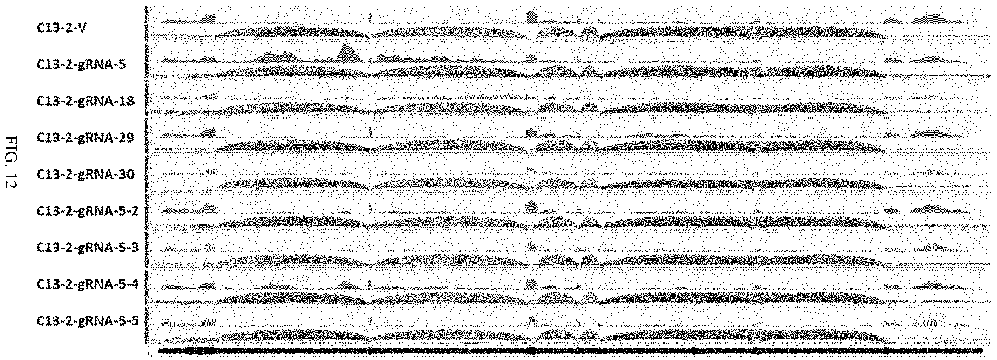

It was speculated that the 21-nt continuous nucleotide sequence of gRNA-5 was complementary to the VEGFA pre-mRNA, which might have led to the binding of the complex of C13-2 and gRNA-5 to the pre-mRNA, subsequently causing a new splicing mode of pre-mRNA, leading to the enrichment of introns. To solve this problem, multiple gRNAs were designed near gRNA-5, with their guide sequences evenly distributed across the two exon sequences, aiming to reduce the binding of the C13-2-gRNA complex to pre-mRNA. As a result, gRNA-5-2, gRNA-5-3, gRNA-5-5 and gRNA-5-4 as a control were obtained. As shown in FIG. 11. The designed gRNA was combined with C13-2 for editing, and the RNA-seq results are shown in FIG. 12. gRNA-5-2, gRNA-5-3 and gRNA-5-5 did not cause enrichment of reads at introns. At the same time, gRNA-29 and gRNA-30 did not cause enrichment of reads at introns. gRNA-5-4 caused intron enrichment. The degree of intron enrichment caused by gRNA-18 was low. These results revealed that the safety of gRNA-30, gRNA-29, gRNA-5-2, gRNA-5-3 and gRNA-5-5 was better than that of gRNA-5. The safety of the combination of gRNA-30, gRNA-29, gRNA-5-2, gRNA-5-3 and gRNA-5-5 with C13-2 was better than that of CasRx-X-dual in the prior art. Therefore, gRNA-30, gRNA-29, gRNA-5-2, gRNA-5-3, gRNA-5-5 and gRNA-18 achieved unexpected technical effects.

In addition, the method of Example 1 was used to prepare plasmids, transfect cells, and perform qPCR detection. The primer sequences used are shown in Table 27. The biological experiments were independently repeated three times, and the test results show that the editing efficiency of the combination of gRNA-5-2, gRNA-5-3, gRNA-5-4 and gRNA-5-5 with C13-2 is higher than 50%.

Table 27. qPCR primer sequences

| Primer number | Sequence | Use | SEQ ID NO |
|---|---|---|---|
| qGAPDHF1 | TCCAAAATCAAGTGGGGCGA | For detecting internal reference genes in qPCR experiments | 231 |
| qGAPDHR1 | TGATGACCCTTTTGGCTCCC | For detecting internal reference genes in qPCR experiments | 232 |
| qVEGFA-F1 | ACCTCCACCATGCCAAGTGG | For detecting *VEGFA* RNA levels | 24 |
| qVEGFA-R1 | CAGGGTCTCGATTGGATGGC | For detecting *VEGFA* RNA levels | 25 |

**Example 11: Significant inhibition of neovascularization in mice by targeting VEGFA**

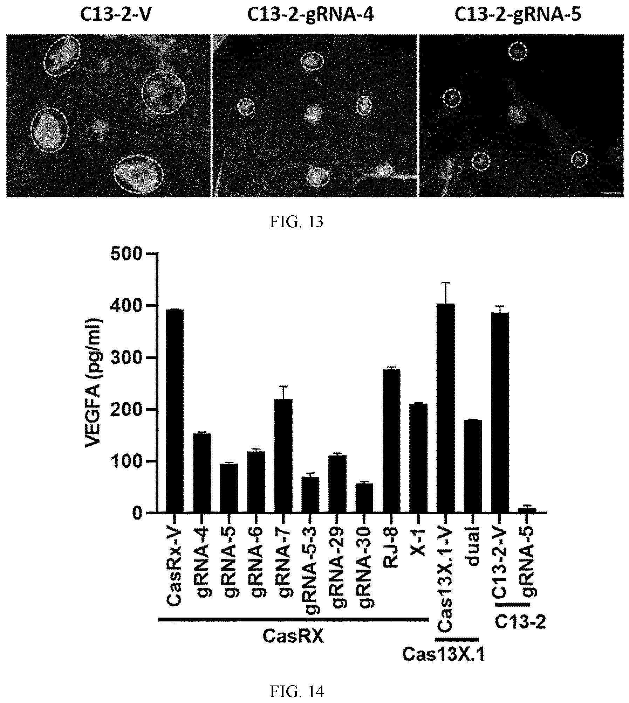

The plasmids C13-2-gRNA-4 and C13-2-gRNA-5 constructed in Example 1 were commissioned to an outsourcing company for packaging into AAV8 WT viruses. Three plasmids were transfected into 293T cells to produce AAV, and AAV was obtained by gradient centrifugation for purification, and the titer was determined by qPCR to be 1E13 vg/mL. The mice were anesthetized via intraperitoneal injection of 200 μL/20 g 2.5% tribromoethanol anesthetic solution. After pupil dilation and local anesthesia, a 30G sharp needle was first used to puncture the corneal edge, followed by subretinal injection of 1 μL of AAV using a WPI nanofil RPE kit. Successful administration was confirmed by observing the formation of a retinal bleb under microscopy. Tobramycin antibiotic was then applied to the ocular surface, and the mice were placed in cages after they woke up. Three weeks after administration, laser modeling was performed. The mice were anesthetized intraperitoneally with 380 μL/20 g 2.5% tribromoethanol anesthetic solution. After pupil dilation and local anesthesia, the fundus of the mice was observed using a Lumenis Nova Spectra 532 nm laser therapeutic instrument. At a distance of 2-3 optic disc diameters from the optic papilla, laser photocoagulation was evenly applied in four directions around the optic disc, using 100 mW, 100 ms, 100 μm, to remove bleeding, cataracts, and other adverse effects in the mice. Seven days after modeling, samples were collected to observe the area of choroidal neovascularization (CNV). The retinal pigment epithelium (RPE)/choroid/sclera complex was isolated and perfused with 4% paraformaldehyde (PFA). The new blood vessels were stained with IB4-488 nm (Thermo Fisher I21411), and the images were taken with a Lecia inverted fluorescence high-power microscope. The CNV area was statistically analyzed using ImagJ 1.52v software. The results are shown in FIG. 13. The CNV area after editing by C13-2-gRNA-4 and C13-2-gRNA-5 is significantly reduced, demonstrating that both can significantly inhibit neovascularization.

**Example 12**

The plasmid was constructed in the same method as in Example 2. The annealed products of the forward and reverse primers corresponding to the guide sequences such as gRNA-4, gRNA-5, gRNA-5-3, gRNA-6, gRNA-7, gRNA-29, gRNA-30, gRNA-RJ-8 and gRNA-X-1 in Table 28 (primers with restriction enzyme cutting sites, *i.e.,* the forward primer was obtained by adding AAAC to the 5' end of the sequence in Table 28, and the reverse primer was obtained by adding cttg to the 5' end of the reverse complementary sequence of the sequence in Table 28) were selected and linked to the BpiI-digested backbone of the pAAV-CasRx-gRNA plasmid (also known as CasRx-V, SEQ ID NO: 28). The resulting construct was transformed into competent *E. coli,* cultured in a culture medium containing the appropriate antibiotic, and subjected to colony PCR reaction. Positive clones were screened via electrophoresis, and plasmids were extracted. Sanger sequencing was performed for verification, thereby obtaining CasRx plasmid targeting VEGFA RNA, which could be used to express CasRx and gRNA (such as gRNA-5). Meanwhile, the publicly available tool Cas13X.1 was used to prepare the Cas13X.1-V vector (SEQ ID NO: 8) through conventional methods. Corresponding forward and reverse primers were synthesized according to the Cas13X.1-dual sequence (with cacc added to the 5'-end of the sense strand and cage added to the 5'-end of the antisense strand). These primers were then ligated with the BsaI-digested backbone of the Cas13X.1-V vector to construct Cas13X.1-dual, wherein Cas13X.1-V was used as a negative control. In this example, the C13-2-gRNA-5 constructed in Example 1 and its corresponding negative control C13-2-V were also used. The plasmid was transfected into 293T cells using the same method as in Example 4, and the supernatant was collected 72 hours after transfection for ELISA to detect the level of VEGFA protein. The Human VEGF-A (Vascular Endothelial Cell Growth Factor A) ELISA Kit from Elascience was used, strictly adhering to the kit's operating procedures. Standards with different concentrations were set, and samples were processed. Final data were read at 450 nm using a microplate reader (MultiskanFC from Thermo Fisher). The corresponding formula between sample concentration and OD value was calculated based on the OD value of the standard, and the experiments were repeated for 2 times.

Table 28. Primer-related sequences in this example

| Name | gRNA related sequences | SEQ ID NO |
|---|---|---|
| Guide sequence of RJ-8 | ATGTTGGACTCCTCAGTGGGCACACACTCC | 31 |
| Guide sequence of X-1 | GTGCTGTAGGAAGCTCATCTCTCCTATGTG | 33 |
| Cas13X.1-dual (tandem X-1+X-2 guide sequence for Cas13X.1 re-lated vector construction) | GTGCTGTAGGAAGCTCATCTCTCCTATGTGGCT GGAGCAGCCCCCGATTTGTGGGGTGATTACAG CGGTACTCCTGGAAGATGTCCACCAGGGTCT | 236 |
| Guide sequence of gRNA-4 | AAGCTCATCTCTCCTATGTGCTGGC | 12 |
| Guide sequence of gRNA-5 | TGGGTGCAGCCTGGGACCACTTGGCATGG | 13 |
| gRNA-6 | TCTTTCTTTGGTCTGCATTCACAT | 14 |
| Guide sequence of gRNA-7 | GGCCTTGGTGAGGTTTGATCCGCAT | 15 |
| Guide sequence of gRNA-29 | TCCGCATAATCTGCATGGTGATGTT | 58 |
| Guide sequence of gRNA-30 | TGTGCTGGCCTTGGTGAGGTTTGAT | 59 |
| Guide sequence of gRNA-5-3 | GCAGCCTGGGACCACTTGGCATGGT | 228 |

In this example, three types of Cas13 were co-transfected in the same batch in combination with different gRNAs. From the results of this example and the above examples, it can be seen that several gRNAs of the present disclosure exhibit high editing efficiency when combined with C13-2 or CasRx, respectively, and are significantly superior to the combination of Cas13X.1-dual disclosed in the existing patents. As shown in Table 29 and FIG. 14, C13-2-gRNA-5 performs best after plasmid transfection, with a target protein downregulation rate as high as 97.4%. CasRx-gRNA-30 and CasRx-gRNA-5-3 result in target protein downregulation of 85.4% and 82.2%, respectively. CasRx-gRNA-5 and CasRx-gRNA-29 result in target protein downregulation of 75.7% and 71.8%, respectively. CasRx-gRNA-4 and CasRx-gRNA-6 result in target protein downregulation of 61.0% and 69.7%, respectively. The protein downregulation efficiency of the above combinations is significantly superior to that of CasRx-X-1, CasRx-RJ-8 and Cas13X.1-dual groups (P<0.01).

Table 29. Comparison of knockdown efficiency at protein levels

| Group (transfection with different plasmids) | VEGFA protein level (%) | Protein downregulation efficiency (%) |
|---|---|---|
| CasRx-V | 100.0 | 0.0 |
| CasRx-gRNA-4 | 39.0 | 61.0 *** |
| CasRx-gRNA-5 | 24.3 | 75.7 *** |
| CasRx-gRNA-6 | 30.3 | 69.7 *** |
| CasRx-gRNA-7 | 56.0 | 44.0 *** |
| CasRx-gRNA-5-3 | 17.8 | 82.2 *** |
| CasRx-gRNA-29 | 28.2 | 71.8 *** |
| CasRx-gRNA-30 | 14.6 | 85.4 *** |
| CasRx-RJ-8 | 70.5 | 29.5 *** |
| CasRx-X-1 | 53.7 | 46.3 *** |
| Cas13X.1-V | 100.0 | 0.0 |
| Cas13X.1-dual | 44.4 | 55.6 *** |
| Cas13-2-V | 100.0 | 0.0 |
| Cas13-2-gRNA-5 | 2.6 | 97.4 *** |
| Note: The VEGFA protein levels of the negative control CasRx-V, Cas13X.1-V and C13-2-V groups are all set as 100%, and the data of other groups are calculated based on their respective negative controls. "***" indicates p<0.001 compared with the corresponding negative control group. | | |

## Example 13

In order to compare the safety of the newly discovered gRNAs with high editing efficiency in the present disclosure with that of the previously disclosed gRNAs, experiments were designed. The C13-2 plasmids targeting VEGFA constructed in the above examples were used, while the C13-2-RJ-2, C13-2-RJ-8 and C13-2-X-1 plasmids (expressing the corresponding gRNAs containing the guide sequences of RJ-2, RJ-8 and X-1, respectively) were constructed using the same method as in Example 7. The gRNA-luc targeting the luciferase gene (guide sequence: GAATGTAGCCATCCA TCCTTGTCAA, SEQ ID NO: 237) was selected and constructed into C13-2-gRNA-luc using the same method as the control group for RNA-seq. The plasmid transfection method of 293T cells was performed as described in Example 1. After 72 hours of transfection, cell pellets were collected, and total RNA was extracted. The RNA samples were then sent to a third-party gene sequencing company for RNA library construction and sequencing on the DNBSEQ-T7 (BGI Genomics) platform to obtain RNA-seq data. The raw sequencing data were subjected to quality control using fastqc and multiqc. Low-quality reads were removed using fastq, and rRNA sequence reads were removed using the STAR software. The expression levels of genes were quantified using the StringTie2 software, followed by significance of difference analysis with DESeq2 software. Genes with |log2fold change|>0.5, padj<0.05, and baseMean>2 were identified as differentially expressed genes. Transcripts were regarded as gRNA-related predicted targeting transcripts when aligned with the guide sequence, meeting the criteria of ≥21 matching bases, ≤6 mismatched bases, and a minimum of ≥8 consecutive matching bases using the EMBOSS water software. The corresponding genes were then considered as predicted target genes. The downregulated differentially expressed gene set was finally intersected with the predicted target gene set, resulting in the identification of off-target genes (with VEGFA, the on-target gene, being excluded).

The specific results are shown in Table 30. Compared with the control group, no gRNA-related differentially expressed genes are detected in gRNA-30 and gRNA-7, indicating that no off-target genes are identified. The gRNA-X-1 adjacent to its target sequence is found to contain 2 off-target genes. In terms of druggability, it is no longer merely a difference in quantity, but a qualitative distinction.

In addition, it can be seen from the above examples that the editing efficiency of gRNA-30 is also significantly better than that of gRNA-X-1, that is, when used in combination with the same Cas13 protein, the editing efficiency and safety of gRNA-30 are significantly better than those of gRNA-X-1 adjacent to its target sequence, and the drugability is better, thus achieving unexpected technical effects.

gRNA-29 has only 1 gRNA-related off-target gene. Therefore, it is also considered to have certain advantages in terms of off-target safety.

The number of differentially expressed genes downregulated by the editing of gRNA-29, gRNA-30, gRNA-7, gRNA-5-2, gRNA-5-3 and gRNA-5-5 is significantly less than that of gRNA-RJ-2, gRNA-RJ-8 and gRNA-X-1 in the prior art, indicating a smaller perturbation to the transcriptome.

The gRNA-RJ-2 is found to have tens of off-target genes, while gRNA-5-2, gRNA-5-3 and gRNA-5-5, which are adjacent to their target sequences, have much fewer off-target genes, and thus safer in terms of off-targeting.

Table 30. Statistical results of off-target analysis after editing with different gRNAs and C13-2

| Experimental group | Total number of upregulated differentially expressed genes | Total number of downregulated differentially expressed genes | Total number of predicted target genes | Number of off-target genes | Off-target genes |
|---|---|---|---|---|---|
| C13-2-gRNA-29 | 37 | 27 | 159 | 1 | AGO3 |
| C13-2-gRNA-g30 | 25 | 49 | 281 | 0 | None |
| C13-2-gRNA-5-2 | 40 | 45 | 1237 | 2 | SH2D3C, ELFN1 |
| C13-2-gRNA-5-3 | 55 | 45 | 517 | 2 | SCRIB, RAD54L2 |
| C13-2-gRNA-5-5 | 37 | 53 | 1453 | 5 | NUP98, GDF15, PRR14L, PLEKHH1, TMC8 |
| C13-2-gRNA-7 | 47 | 56 | 179 | 0 | None |
| C13-2-RJ-2 | 326 | 721 | 1108 | 41 | C3, WDR90, GPC2, KDM6B, ANKRD33B, RASD2, CCDC113, THSD4, PSCA, INSRR, ADAMTS14, PRODH, BDKRB2, ADM2, AHNAK2, CTIF, KCNB1, RDM1, ENO4, PALM, BEAN1, ENO2, B3GAT1, ABLIM3, HECW1, NDST2, IRS4, CLIP2, CHST5, SEC14L5, PPFIA4, RASGRF1, SLC6A1, TRIM7, |
| | | | | | LRFN2, OTOG, GALNT12, TMEM130, SCN4A, FAM122C, CREB3L1 |
| C13-2-RJ-8 | 128 | 170 | 266 | 4 | TBC1D8, ZBTB20, SEC14L2, GPRIN2 |
| C13-2-X-1 | 63 | 108 | 313 | 2 | FRAS1, TXNDC9 |

## Example 14: Editing efficiency of C13-2 mutant in combination with gRNA

The C13-2 mutant, as shown in Table 31, was designed by the inventor and tested for editing efficiency in combination with gRNA-5.

Expression vectors for expressing Cas13 (C13-2 and its mutants) and gRNA-5 were constructed, and transfected into 293T cells following the instructions of Lipofectamine 2000 (Thermo). A 293T cell control group was set up without transfection of any plasmid.

The target RNA level was detected by qPCR.

After 48 hours of transfection, the cells were subjected to RNA extraction using the SteadyPure Universal RNA Extraction Kit, and the RNA concentration was measured with an ultra-micro spectrophotometer. The RNA product was reverse transcribed using the Evo M-MLV Mix Kit with gDNA Clean for qPCR, and the reverse transcription product was detected using the SYBR Green Premix Pro Taq HS qPCR Kit (Low Rox Plus). The experiment was repeated three times, and the results were averaged.

The relative values of VEGFA RNA levels after editing the M01-M22 mutants of C13-2 are all lower than 0.04 compared with the VEGFA RNA levels of the C13-2-BsaI control group, that is, the editing efficiency is higher than 96%.

Table 31. C13-2 mutants

| Mutant name | Mutation site | Mutant name | Mutation site |
|---|---|---|---|
| M01 | R11A | M12 | R276A |
| M02 | N34A | M13 | R290A |
| M03 | R35A | M14 | R294A |
| M04 | R47A | M15 | N299A |
| M05 | R58A | M16 | N303A |
| M06 | R63A | M17 | R308A |
| M07 | R64A | M18 | R314A |
| M08 | N68A | M19 | R320A |
| M09 | N87A | M20 | R328A |
| M10 | N265A | M21 | N332A |
| M11 | N274A | M22 | R341A |

### Example 15: Test of combinations of C13-2 inactivated mutants and gRNA

The inactivated mutants shown in Table 32 were designed by the inventors and tested for editing efficiency in combination with gRNA-5.

The expression vectors for expressing the inactivated mutant of Cas13 and gRNA-5 were constructed, and the vectors were transfected into the 293T cell line. Transfection was performed according to the instructions of Lipofectamine 2000 (Thermo). RNA was extracted using the SteadyPure Universal RNA Extraction Kit after 72 hours. The RNA extracted from three batches of experiments was sent to a sequencing company for RNAseq sequencing, and the amount of VEGFA RNA was detected as shown in Table 32 below.

Table 32. Amount of VEGFA RNA determined by RNAseq sequencing

| Group | Reduction of VEGFA RNA after editing relative to the C13-2-BsaI group |
|---|---|
| C13-2-BsaI | 0 |
| R4xH-1-VEGFA (R210A+H215A mutations) | 10.25 |
| R4xH-2-VEGFA (R750A+H755A mutations) | 45.82 |
| R4xH-3-VEGFA (R785A+H790A mutations) | 14.85 |
| R4xH(1,3)-VEGFA (R210A+H215A and R785A+H790A mutations) | 23.34 |
| R4xH(1,2,3)-VEGFA (R210A+H215A, R750A+H755A and R785A+H790A mutations) | 4.81 |

Experimental data show that the editing activity is still high after the introduction of the R750A+H755A mutations, and the weak editing activity is retained after the introduction of the R210A+H215A and/or R785A+H790A mutations. The editing activity is almost completely lost after the introduction of the R210A+H215A, R750A+H755A and R785A+H790A mutations.

### Example 16

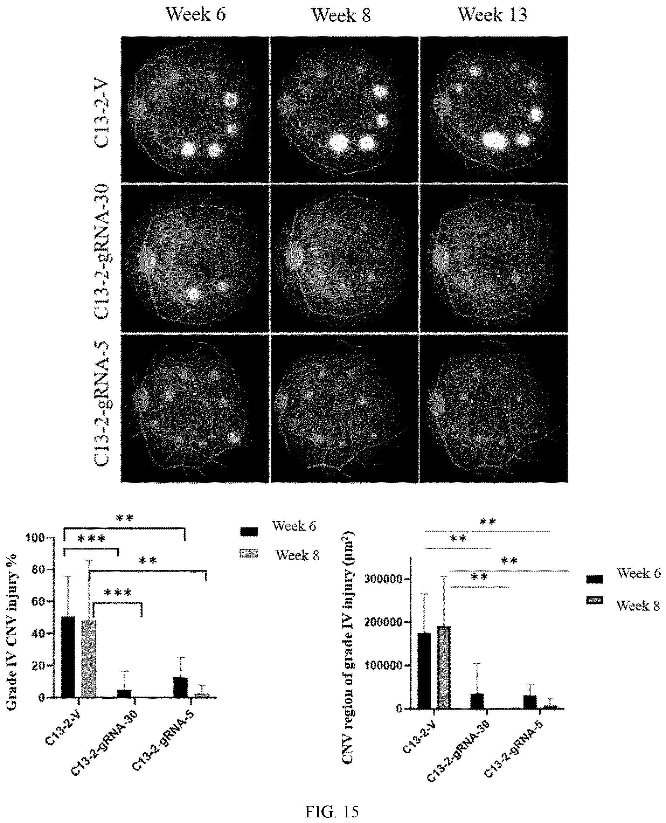

Based on the efficacy results in mice, C13-2-V, C13-2-gRNA-30 and C13-2-gRNA-5 were packaged into three types of AAV2/8 using AAV capsid plasmids and helper plasmids. These three types of AAV2/8 were then tested for inhibition of neovascularization in laser-induced non-human primate (NHP) cynomolgus monkeys. The three types of AAVs with equal volumes and doses were subretinally injected into the eyes of NHP animals. The animals were first anesthetized systemically, their pupils were dilated, and ocular surface was anesthetized, followed by subretinal injection and finally antibiotics were applied to the ocular surface while keeping warm. After the animals woke up, they were fed normally. Three animals were administered per group. After 4 weeks of administration, 6-10 photocoagulation spots were generated near the macular area of the animal's eye using an Iridex laser therapy device with the same parameters. Eyes with hemorrhage areas exceeding 2 photocoagulation spots were excluded. Sodium fluorescein was injected intraperitoneally at different time points at 6, 8 and 13 weeks of administration, and the fluorescence leakage at different time points was tracked and photographed. Finally, the spot grading was evaluated by 3 different technicians. The proportion of grade IV spots is shown in FIG. 15. The drug groups C13-2-gRNA-30 and C13-2-gRNA-5 significantly inhibited the formation of grade IV spots at different administration time points, that is, significantly inhibited the neovascularization, and the effect was sustained for a long time.

Based on the above examples, it can be seen that the editing efficiency of gRNA with a guide sequence of SEQ ID NOs: 12, 13, 15, 40-43, 45-47, 52-60, 64, 65, 73, 216, 219, 220, 223, 227, 228, 229 and 230 is high; the off-target risk of gRNA with a guide sequence of SEQ ID NOs: 12-15 is significantly reduced, and the risk of off-target is lower; the editing of gRNA with a guide sequence of SEQ ID NOs: 58, 59, 227, 228 and 230 basically does not cause intron enrichment, and the safety risk associated therewith is lower.

Although specific embodiments of the present disclosure are described above, those skilled in the art should understand that these are merely examples. Without departing from the principles and essence of the present disclosure, various changes or modifications can be made to these embodiments. Therefore, the scope of protection of the present disclosure is defined by the appended claims.

Some of the sequences used in the present disclosure are as follows:

Cas13m.3 (SEQ ID NO: 1):

MYHLSDANHGKHIAGTYYEMAMGNFIHTLSHMLVRAGIKVNKLEDNYSIEREI
MNLTAPGAYDAQRAALSRLLYRHFPFFGPIMADHTDHILSSKRKKVQSASDDGNLDLGQ
ELKDEVAGASACQMIRYLATIAGALVYYRNMYSHKNHYDNAQDIAAQQEREQKLALW
LDVVFRGARDILLTRKSHPQPDTDFLTQNGTINYYIEKNGKSAYNPNFYFKPGLKTDNG

WVMTDFGKFFFCSLFLRRADAERFAAETDLYVGSPFKITAQERARLQEAENKRAADEQA
RASSAGFPHIVNPRTIGPSESPQNNIIREMLNMHRARIPRERRIDADMSEGILAMDIMNEL
RRCPLSLYNTLSPEAKASFEKTGVTPEGGIVSNLLVRHSDRYPELALRAIDQMELLPTIRF
HVRLGSLRFRFYEKKLIDGSHTLRTVQKAVNGFGRWQEVEPRRVEKYTAIQARCQNDKG
IDQFLPDSPTTTPYITDWRTTYNIHANRIGLAWNLPQMSDGIYLPNLDTDKGDNLHRKAL
IDMPAPMCYLSIFDLPALLFYCHIYTHYHGTKYHLPSAESIIQAKYDALHKFFSFAAAQNH
SAEQLREKQLELNLADNEIPDKLRCMMQTKPFFKNGRQQLSPLGYPIMKNWIGVAEQR
KHAAQVLRDVANEAADRLASFEKKHQRVVVGGRDNRYGRRGHADIRHGSLARYLATS
MVRWQPALDQPGGDKLTSANHRALAGFLSEYGLHGSNINKLRNVLKEAGLIEGSHPHPF
LAHVLESAPANIEALYVAYLKHEQSHATALKNKFTDRNGIVQPSEVPAFVRFNSSRWRND
SATTARRYLQTPPAPGSSDSAEHNAPIMLPDGLFTTHIMTLLNKVLGQNDRVPEEDYLRH
DLPRIASIINPNGKTYGAAYIIRAWFDQVENQDVQPFYDLPRFYREISLLAPRRKPNQELIR
DYFSEEQIAQKIQTVPKKQRSEKVGHTIDTEKDIRRYRLQDITLYLTLLDMLTLMLSRNEA
ERTDRQMKSSTAERVSNMRLVDFDHSFDFDLLGSTSGEAAYSYLHQRSGITISMPALSLR
SYGSIFRVLADSRFETLMDALNRQGVTHVNFGDITSELALYDTLRSHFLLQAHNVEQDA
FSAKRGVLENHTSPFFYRSGNLQLDDQGNITNPSTDAIRNHYGELIKILDRYSLKIDKKTK
DGKSQDILLRDLMAELRNAAAHNRYPKADFFFRQFDHFLNTCKPTDSNLTAPNYIRTVL
EFLKSIVDNNFTPLLHEESPENESKSE

Cas13m.6 (SEQ ID NO: 2):

MKEQKKFSLQGVRHVCGSYFNMALNNYCRTLNGVFIKCKIKFALKEDDFPRSL
SSLRKIFSSGPIMPQTEKKVAKMIASVDTAMKLKQQLFKHFPMLGPIMDKTISCMNHHK
GSSVADAPLDLCMNAILNFGECLYHCRNFYTHFKPYNSPEDLKLQYDIQHIIALNLGTLF
DVSRRIGKKREGLTPEELEFLTGKNRFNQVGKKFLERNDWYLKIEKPSDMKDYDKTILS
DFGMVYLCSIFLAKNYALRLFDESKLFNKETIRNLFSEEQVRFLKEMLVIYRIRTPRGKQL
DSHDSKQALAMDMLNELRKCPRPLYDVLSEEYKKRTFYVPVEHENEKTEEYVKMLRSD
DRFPYFTLRYIDDMEKFSRIRFQIRLGSYRFKFYDKMNIDGTPRIRSLQKEINGFGRLSDM
ENKRKREWKDMFQATEEIDYEDQFGDYQTGVTQFVEDTADTKPYVTNHRAAYNVHSN
HIGLIWNDADSIILQDDNKLFFPDLKIDENGKADIYQPSPKASLSVFDFPAMVFYMYLRE
KTEATKEFPSAEQLIINKYDHLVRFFKDISDGRFGPSENKNAFSKKLKEEYDLKTGEIPEK
LLHWLSSESEEDPSEKYAKKLEEEIKLRRERVQRRLEKFNQDLREIRKKDSVPYGKKGH
VNIRHSQLAKYLMRSIMEWQPTRNDGKNKLTGQNFNVMTAFLATLGYTSQVKDLRDLF
SRANMLEGPNAHPFLKKVLNNNSIKDIQGFYRTYLVEELNQIEDKQRRIAKAKNVKDTV
RQFPFAHFNRMRYQKRDEDYYRNLAKRYLNIGDNEKDKAVILLPDGMFTSYIYDLIMKL

PENNEKMRINLASDVAHCNSSFLISRFFENIRNDYAQPFYREERTYELFSILNNKKVRNTL
QPLFISPHDINIQLTEKEKDGKGRLILQKIDHFCKSITQKGNFNNVEEAKEATSRKLKHLIT
DCKNNERDIRRYKTQDMVIYLMARDILKDIIPDSEKDKYAKDRKLLLKDVCEEGFLRQA
VKMEYEYSIEEKGKRTRTVKITHPNMSLKNYGEFHRLLNDERLKSLLQQLANMDEIDYT
DLMGEFADYDQKRSEIFRLAQSIEKHLYEQNEQGLNDEKSDLFYHTRYNGKKIPRRNSFS
SLLELIGEEESQMTETDKKQTISIRNAFGHNTYKVSLAEMNATELPNVAKTILKKMEELR
NKL

C13-2 (SEQ ID NO: 3):

MSKDKKTKAKRMGVKALLAHGEDKLTMTTFGKGNRSKIEFTEGYHGRALETP
KHFGIRGFEVRRIDENVDLCGDLEEGKTIEALLVNPSEKVGEDYLKLKGTLEKRFFGREF
PHDNIRIQLIYNILDIYKILGMNVADILYALGNMQDTELDIDMFGQSLNNEDNLKECLKR
MRPYMGYFGDIFKISPKGENIADREHNKKVLRCISVLRNATAHDKQDEYPWFKSSDIYET
KIFKADMWKIIKDQYREKIKKVNKDFLSKNAVNMAILFDLLNARDVEQKKQITDEFYRF
TIRKDGKNLGMNLVKIREIIIDRYASGLRDKKHDPHRQKINVIADFLIFRALSQNQGIIDKT
VSSLRLTKDEEEKDHVYQNAAELVWGMVSNCLTPYFNDPKNKYILKYKDAKTPGDFED
WITSKISEDDGEPFVKVLSFLCNFLEGKEINELLTAYIHKFECIQDFLNVISSLGENVQFQP
RFALFNNASFAQNVAVQLRILASIGKMKPDLTEAKRPLYKAAIRMLCPPEKWEKYTSDE
WLEKNMLLNSEDRKNDKKKKQVNPFRNFIAGNVIESRRFMYLVRYSKPKAVRAIMQNR
SIVNYVLHRLPSEQVHRYASVFPENFADLEQEIDFLTKKLFEFSFEELLHEKDVILNNSRSH
KPSLEIERLKAITGLYLSVAYIAIKNIVKANARYYIAFAVFERDKELVKAKDARIQTKIPET
DFPDYFCLTQYYLDRDEEKKFPGDPRDKEAFFEHLRKTKRHFSKQWREWLNEKIADAK
SSQATGLLLREARNDVEHLNVLRAIPDYIQDFRHGEKGETAMNSYFELYHYLMQRLML
KNTELDLSHWSGWIMRSGRPDRDLIQIAFVSLAYNLPRYRNLTKEHHFDDTVLQKIREKE
SLD

Cas13m.3-BsaI plasmid (SEQ ID NO: 4):

cctgcaggcagctgcgcgctcgctcgctcactgaggccgcccgggcgtcgggcgacctttggtcgcccggcctcagtgagc
gagcgagcgcgcagagagggagtggccaactccatcactagggggttcctgcggcctctagactcgaggcgttgacattgattattgactag
ttattaatagtaatcaattacggggtcattagttcatagcccatatatggagttccgcgttacataacttacggtaaatggcccgcctggctgacc
gcccaacgacccccgcccattgacgtcaataatgacgtatgttcccatagtaacgccaatagggactttccattgacgtcaatgggtggagta
tttacggtaaactgcccacttggcagtacatcaagtgtatcatatgccaagtacgccccctattgacgtcaatgacggtaaatggcccgcctg
gcattatgcccagtacatgaccttatgggactttcctacttggcagtacatctacgtattagtcatcgctattaccatggtgatgcggttttggca
gtacatcaatgggcgtggatagcggtttgactcacggggatttccaagtctccaccccattgacgtcaatgggagtttgtttggcaccaaaat

caacgggactttccaaaatgtcgtaacaactccgccccattgacgcaaatgggcggtaggcgtgtacggtgggaggtctatataagcagag

ctctctggctaactaccggtgccaccatgccggcagctaagaaaaagaaactggatggcagcgtcgacATGTACCACCTGAG

CGACGCCAACCACGGCAAGCACATCGCCGGCACCTACTACGAGATGGCCATGGGCAA

CTTCATCCACACCCTGAGCCACATGCTGGTGAGAGCCGGCATCAAGGTGAACAAGCT

GGAGGACAACTACAGCATCGAGAGAGATCATGAACCTGACCGCCCCCGGCGCCT

ACGACGCCCAGAGAGCCGCCCTGAGCAGACTGCTGTACAGACACTTCCCCTTCTTCG

GCCCCATCATGGCCGACCACACCGACCACATCCTGAGCAGCAAGAGAAAGAAGGTG

CAGAGCGCCAGCGACGACGGCAACCTGGACCTGGGCCAGGAGCTGAAGGACGAGG

TGGCCGGCGCCAGCGCCTGCCAGATGATCAGATACCTGGCCACCATCGCCGGCGCCC

TGGTGTACTACAGAAACATGTACAGCCACAAGAACCACTACGACAACGCCCAGGAC

ATCGCCGCCCAGCAGGAGAGAGCAGAAGCTGGCCCTGTGGCTGGACGTGGTGTT

CAGAGGCGCCAGAGACATCCTGCTGACCAGAAAGAGCCACCCCCAGCCCGACACCG

ACTTCCTGACCCAGAACGGCACCATCAACTACTACATCGAGAAGAACGGCAAGAGC

GCCTACAACCCCAACTTCTACTTCAAGCCCGGCCTGAAAACCGACAACGGCTGGGTG

ATGACCGACTTCGGCAAGTTCTTCTTCTGCAGCCTGTTCCTGAGAAGAGCCGACGCC

GAGAGATTCGCCGCCGAGACAGACCTGTACGTGGGCAGCCCCTTCAAGATCACCGCC

CAGGAGAGAGCCAGACTGCAGGAGGCCGAGAACAAGAGAGCCGCCGACGAGCAGG

CCAGAGCCAGCAGCGCCGGCTTCCCCCACATCGTGAACCCCAGAACCATCGGCCCCA

GCGAGAGCCCCCAGAACAACATCATCAGAGAGATGCTGAACATGCACAGAGCCAGA

ATCCCCAGAGAGAGAAGAATCGACGCCGACATGAGCGAGGGCATCCTGGCCATGGA

CATCATGAACGAGCTGAGAAGATGCCCCCTGAGCCTGTACAACACCCTGAGCCCCGA

GGCCAAGGCCAGCTTCGAGAAAACCGGCGTGACCCCCGAGGGCGGCATCGTGAGCA

ACCTGCTGGTGAGACACAGCGACAGATACCCCGAGCTGGCCCTGAGAGCCATCGAC

CAGATGGAGCTGCTGCCCACCATCAGATTCCACGTGAGACTGGGCAGCCTGAGATTC

AGATTCTACGAGAAGAAGCTGATCGACGGCAGCCACACCCTGAGAACCGTGCAGAA

GGCCGTGAACGGCTTCGGCAGATGGCAGGAGGTGGAGCCCAGAAGAGTGGAGAAG

TACACCGCCATCCAGGCCAGATGCCAGAACGACAAGGGCATCGACCAGTTCCTGCCC

GACAGCCCCACCACCACCCCCTACATCACCGACTGGAGAACCACCTACAACATCCAC

GCCAACAGAATCGGCCTGGCCTGGAACCTGCCCCAGATGAGCGACGGCATCTACCTG

CCCAACCTGGACACCGACAAGGGCGACAACCTGCACAGAAAGGCCCTGATCGACAT

GCCCGCCCCCATGTGCTACCTGAGCATCTTCGACCTGCCCGCCCTGCTGTTCTACTGC

CACATCTACACCCACTACCACGGCACCAAGTACCACCTGCCCAGCGCCGAGAGCATC

ATCCAGGCCAAGTACGACGCCCTGCACAAGTTCTTCAGCTTCGCCGCCGCCCAGAAC

CACAGCGCCGAGCAGCTGAGAGAGAAGCAGCTGGAGCTGAACCTGGCCGACAACG

AGATCCCCGACAAGCTGAGATGCATGATGCAGACCAAGCCCTTCTTCAAGAACGGCA
GACAGCAGCTGAGCCCCCTGGGCTACCCCATCATGAAGAACTGGATCGGCGTGGCCG
AGCAGAGAAAGCACGCCGCCCAGGTGCTGAGAGACGTGGCCAACGAGGCCGCCGA
CAGACTGGCCAGCTTCGAGAAGAAGCACCAGAGAGTGGTGGTGGGCGGCAGAGAC
AACAGATACGGCAGAAGAGGCCACGCCGACATCAGACACGGCAGCCTGGCCAGATA
CCTGGCCACCAGCATGGTGAGATGGCAGCCCGCCCTGGACCAGCCCGGCGGCGACA
AGCTGACCAGCGCCAACCACAGAGCCCTGGCCGGCTTCCTGAGCGAGTACGGCCTG
CACGGCAGCAACATCAACAAGCTGAGAAACGTGCTGAAGGAGGCCGGCCTGATCGA
GGGCAGCCACCCCCACCCCTTCCTGGCCCACGTGCTGGAGAGCGCCCCCGCCAACAT
CGAGGCCCTGTACGTGGCCTACCTGAAGCACGAGCAGAGCCACGCCACCGCCCTGA
AGAACAAGTTCACCGACAGAAACGGCATCGTGCAGCCCAGCGAGGTGCCCGCCTTC
GTGAGATTCAACAGCAGCAGATGGAGAAACGACAGCGCCACCACCGCCAGAAGATA
CCTGCAGACCCCCCCCGCCCCCGGCAGCAGCGACAGCGCCGAGCACAACGCCCCCA
TCATGCTGCCCGACGGCCTGTTCACCACCCACATCATGACCCTGCTGAACAAGGTGC
TGGGCCAGAACGACAGAGTGCCCGAGGAGGACTACCTGAGACACGACCTGCCCAGA
ATCGCCAGCATCATCAACCCCAACGGCAAGACCTACGGCGCCGCCTACATCATCAGA
GCCTGGTTCGACCAGGTGGAGAACCAGGACGTGCAGCCCTTCTACGACCTGCCCAG
ATTCTACAGAGAGATCAGCCTGCTGGCCCCCAGAAGAAAGCCCAACCAGGAGCTGAT
CAGAGACTACTTCAGCGAGGAGCAGATCGCCCAGAAGATCCAGACCGTGCCCAAGA
AGCAGAGAAGCGAGAAGGTGGGCCACACCATCGACACCGAGAAGGACATCAGAAG
ATACAGACTGCAGGACATCACCCTGTACCTGACCCTGCTGGACATGCTGACCCTGATG
CTGAGCAGAAACGAGGCCGAGAGAACCGACAGACAGATGAAGAGCAGCACCGCCG
AGAGAGTGAGCAACATGAGACTGGTGGACTTCGACCACAGCTTCGACTTCGACCTG
CTGGGCAGCACCAGCGGCGAGGCCGCCTACAGCTACCTGCACCAGAGAAGCGGCAT
CACCATCAGCATGCCCGCCCTGAGCCTGAGAAGCTACGGCAGCATCTTCAGAGTGCT
GGCCGACAGCAGATTCGAGACACTGATGGACGCCCTGAACAGACAGGGCGTGACCC
ACGTGAACTTCGGCGACATCACCAGCGAGCTGGCCCTGTACGACACCCTGAGAAGC
CACTTCCTGCTGCAGGCCCACAACGTGGAGCAGGACGCCTTCAGCGCCAAGAGAGG
CGTGCTGGAGAACCACACCAGCCCCTTCTTCTACAGAAGCGGCAACCTGCAGCTGG
ACGACCAGGGCAACATCACCAACCCCAGCACCGACGCCATCAGAAACCACTACGGC
GAGCTGATCAAGATCCTGGACAGATACAGCCTGAAGATCGACAAGAAAACCAAGGA
CGGCAAGAGCCAGGACATCCTGCTGAGAGACTTGATGGCCGAGCTGAGAAACGCCG
CCGCCCACAACAGATACCCCAAGGCCGACTTCTTCTTCAGACAGTTCGACCACTTCC
TGAACACCTGCAAGCCCACCGACAGCAACCTGACCGCCCCCAACTACATCAGAACC

GTGCTGGAGTTCCTGAAGAGCATCGTGGACAACAACTTCACCCCCCTGCTGCACGAG

GAGAGCCCCGAGAACGAGAGCAAGAGCGAGACAGGCGGCGGCCCCGGCGGCGGCG

CCGCCGCCGGCAGCGGCAGCcctaagaaaaaacgaaaagttggcagcggaagcaaaaggccggcggccacgaaaa

aggccggccaggcaaaaaagaaaaagctcgagTACCCATACGATGTTCCAGATTACGCTtgagaattcCCCT

TGAGCATCTGACTTCTGGCTAATAAAGGAAATTTATTTTCATTGCAATAGTGTGTTGGA

ATTTTTTGTGTCTCTCAggtaccgagggcctatttcccatgattccttcatatttgcatatacgatacaaggctgttagagagata

attggaattaatttgactgtaaacacaaagatattagtacaaaatacgtgacgtagaaagtaataatttcttgggtagtttgcagttttaaaattatg

ttttaaaatggactatcatatgcttaccgtaacttgaaagtatttcgatttcttggctttatatatcttgtggaaaggacgaaacaccggagaccac

ggcaggtctcaGTTGTAGAAGCCGTTCATTCGGGACGGTATGACAACTTTTTTgcggccgcaggaa

cccctagtgatggagttggccactccctctctgcgcgctcgctcgctcactgaggccgggcgaccaaaggtcgcccgacgcccgggcttt

gcccgggcggcctcagtgagcgagcgagcgcgcagctgcctgcaggggcgcctgatgcggtattttctccttacgcatctgtgcggtattt

cacaccgcatacgtcaaagcaaccatagtacgcgccctgtagcggcgcattaagcgcggcgggtgtggtggttacgcgcagcgtgaccg

ctacacttgccagcgccttagcgcccgctcctttcgctttcttcccttcctttctcgccacgttcgccggctttccccgtcaagctctaaatcggg

ggctccctttagggttccgatttagtgctttacggcacctcgaccccaaaaaacttgatttgggtgatggttcacgtagtgggccatcgccctg

atagacggttttttcgccctttgacgttggagtccacgttctttaatagtggactcttgttccaaactggaacaacactcaactctatctcgggctat

tctttgatttataagggattttgccgatttcggtctattggttaaaaaatgagctgatttaacaaaaatttaacgcgaattttaacaaaatattaacgt

ttacaattttatggtgcactctcagtacaatctgctctgatgccgcatagttaagccagccccgacacccgccaacacccgctgacgcgccct

gacgggcttgtctgctcccggcatccgcttacagacaagctgtgaccgtctccgggagctgcatgtgtcagaggttttcaccgtcatcaccg

aaacgcgcgagacgaaagggcctcgtgatacgcctatttttataggttaatgtcatgataataatggtttcttagacgtcaggtggcacttttcg

gggaaatgtgcgcggaacccctatttgtttatttttctaaatacattcaaatatgtatccgctcatgagacaataaccctgataaatgcttcaataa

tattgaaaaaggaagagtatgagtattcaacatttccgtgtcgcccttattccctttttgcggcattttgccttcctgttttttgctcacccagaaac

gctggtgaaagtaaaagatgctgaagatcagttgggtgcacgagtgggttacatcgaactggatctcaacagcggtaagatccttgagagtt

ttcgccccgaagaacgttttccaatgatgagcacttttaaagttctgctatgtggcgcggtattatcccgtattgacgccgggcaagagcaact

cggtcgccgcatacactattctcagaatgacttggttgagtactcaccagtcacagaaaagcatcttacggatggcatgacagtaagagaatt

atgcagtgctgccataaccatgagtgataacactgcggccaacttacttctgacaacgatcggaggaccgaaggagctaaccgcttttttgc

acaacatgggggatcatgtaactcgccttgatcgttgggaaccggagctgaatgaagccataccaaacgacgagcgtgacaccacgatgc

ctgtagcaatggcaacaacgttgcgcaaactattaactggcgaactacttactctagcttcccggcaacaattaatagactggatggaggcg

gataaagttgcaggaccacttctgcgctcggcccttccggctggctggtttattgctgataaatctggagccggtgagcgtggaagccgcgg

tatcattgcagcactggggccagatggtaagccctcccgtatcgtagttatctacacgacggggagtcaggcaactatggatgaacgaaata

gacagatcgctgagataggtgcctcactgattaagcattggtaactgtcagaccaagtttactcatatatactttagattgatttaaaacttcatttt

taatttaaaaggatctaggtgaagatcctttttgataatctcatgaccaaaatcccttaacgtgagttttcgttccactgagcgtcagaccccgta

gaaaagatcaaaggatcttcttgagatcctttttttctgcgcgtaatctgctgcttgcaaacaaaaaaaccaccgctaccagcggtggtttgtttg

ccggatcaagagctaccaactctttttccgaaggtaactggcttcagcagagcgcagataccaaatactgttcttctagtgtagccgtagttag

gccaccacttcaagaactctgtagcaccgcctacatacctcgctctgctaatcctgttaccagtggctgctgccagtggcgataagtcgtgtct

taccgggttggactcaagacgatagttaccggataaggcgcagcggtcgggctgaacggggggttcgtgcacacagcccagcttggagc

gaacgacctacaccgaactgagatacctacagcgtgagctatgagaaagcgccacgcttcccgaagggagaaaggcggacaggtatcc

ggtaagcggcagggtcggaacaggagagcgcacgagggagcttccagggggaaacgcctggtatctttatagtcctgtcgggtttcgcc

acctctgacttgagcgtcgattttttgtgatgctcgtcaggggggcggagcctatggaaaaacgccagcaacgcggcctttttacggttcctgg

ccttttgctggcctttttgctcacatgt

Cas13m.6-BsaI plasmid (SEQ ID NO: 5):

cctgcaggcagctgcgcgctcgctcgctcactgaggccgcccgggcgtcgggcgacctttggtcgcccggcctcagtgagc

gagcgagcgcgcagagagggagtggccaactccatcactagggggttcctgcggcctctagactcgaggcgttgacattgattattgactag

ttattaatagtaatcaattacggggtcattagttcatagcccatatatggagttccgcgttacataacttacggtaaatggcccgcctggctgacc

gcccaacgacccccgcccattgacgtcaataatgacgtatgttcccatagtaacgccaatagggactttccattgacgtcaatgggtggagta

tttacggtaaactgcccacttggcagtacatcaagtgtatcatatgccaagtacgcccccctattgacgtcaatgacggtaaatggcccgcctg

gcattatgcccagtacatgaccttatgggactttcctacttggcagtacatctacgtattagtcatcgctattaccatggtgatgcggttttggca

gtacatcaatgggcgtggatagcggtttgactcacggggatttccaagtctccacccattgacgtcaatgggagtttgttttggcaccaaaat

caacgggactttccaaaatgtcgtaacaactccgccccattgacgcaaatgggcggtaggcgtgtacggtgggaggtctatataagcagag

ctctctggctaactaccggtgccaccatgccggcagctaagaaaaagaaactggatggcagcgtcgacATGAAGGAGCAGA

AGAAGTTCAGCCTGCAGGGCGTGAGACACGTGTGCGGCAGCTACTTCAACATGGCC

CTGAACAACTACTGCAGAACCCTGAACGGCGTGTTCATCAAGTGCAAGATCAAGTTC

GCCCTGAAGGAGGACGACTTCCCCAGAAGCCTGAGCAGCCTGAGAAAGATCTTCAG

CAGCGGCCCCATCATGCCCCAGACCGAGAAGAAGGTGGCCAAGATGATCGCCAGCG

TGGACACCGCCATGAAGCTGAAGCAGCAGCTGTTCAAGCACTTCCCCATGCTGGGCC

CCATCATGGACAAGACCATCAGCTGCATGAACCACCACAAGGGCAGCAGCGTGGCC

GACGCCCCCTGGACCTGTGCATGAACGCCATCCTGAACTTCGGCGAGTGCCTGTAC

CACTGCAGAAACTTCTACACCCACTTCAAGCCCTACAACAGCCCCGAGGACCTGAAG

CTGCAGTACGACATCCAGCACATCATCGCCCTGAACCTGGGCACCCTGTTCGACGTG

AGCAGAAGAATCGGCAAGAAGAGAGAGGGCCTGACCCCCGAGGAGCTGGAGTTCCT

GACCGGCAAGAACAGATTCAACCAGGTGGGCAAGAAGTTCCTGGAGAGAAACGACT

GGTACTTGAAGATCGAGAAGCCCAGCGACATGAAGGACTACGACAAGACCATCCTG

AGCGACTTCGGCATGGTGTACCTGTGCAGCATCTTCCTGGCCAAGAACTACGCCCTG

AGACTGTTCGACGAGAGCAAGCTGTTCAACAAGGAGACAATCAGAAACCTGTTCAG

CGAGGAGCAGGTGAGATTCCTGAAGGAGATGCTGGTGATCTACAGAATCAGAACCCC

CAGAGGCAAGCAGCTGGACAGCCACGACAGCAAGCAGGCCCTGGCCATGGACATGC

TGAACGAGCTGAGAAAGTGCCCCAGACCCCTGTACGACGTGCTGAGCGAGGAGTAC

AAGAAGAGAACCTTCTACGTGCCCGTGGAGCACGAGAACGAGAAACCGAGGAGTA

CGTGAAGATGCTGAGAAGCGACGACAGATTCCCCTACTTCACCCTGAGATACATCGA
CGACATGGAGAAGTTCAGCAGAATCAGATTCCAGATCAGACTGGGCAGCTACAGATT
CAAGTTCTACGACAAGATGAACATCGACGGCACCCCCAGAATCAGAAGCCTGCAGA
AGGAGATCAACGGCTTCGGCAGACTGAGCGACATGGAGAACAAGAGAAAGAGAGA
GTGGAAGGACATGTTCCAGGCCACCGAGGAGATCGACTACGAGGACCAGTTCGGCG
ACTACCAGACCGGCGTGACCCAGTTCGTGGAGGACACCGCCGACACCAAGCCCTAC
GTGACCAACCACAGAGCCGCCTACAACGTGCACAGCAACCACATCGGCCTGATCTGG
AACGACGCCGACAGCATCATCCTGCAGGACGACAACAAGCTGTTCTTCCCCGACCTG
AAGATCGACGAGAACGGCAAGGCCGACATCTACCAGCCCAGCCCCAAGGCCAGCCT
GAGCGTGTTCGACTTCCCCGCCATGGTGTTCTACATGTACCTGAGAGAGAAAACCGA
GGCCACCAAGGAGTTCCCCAGCGCCGAGCAGCTGATCATCAACAAGTACGACCACCT
GGTGAGATTCTTCAAGGACATCAGCGACGGCAGATTCGGCCCCAGCGAGAACAAGA
ACGCCTTCAGCAAGAAGCTGAAGGAGGAGTACGACCTGAAAACCGGCGAGATCCCC
GAGAAGCTGCTGCACTGGCTGAGCAGCGAGAGCGAGGAGGACCCCAGCGAGAAGT
ACGCCAAGAAGCTGGAGGAGGAGATCAAGCTGAGAAGAGAGAGAGTGCAGAGAAG
ATTGGAGAAGTTCAACCAGGACCTGAGAGAGATCAGAAAGAAGGACAGCGTGCCCT
ACGGCAAGAAGGGCCACGTGAACATCAGACACAGCCAGCTGGCCAAGTACCTGATG
AGAAGCATCATGGAGTGGCAGCCCACCAGAAACGACGGCAAGAACAAGCTGACCGG
CCAGAACTTCAACGTGATGACCGCCTTCCTGGCCACCCTGGGCTACACCAGCCAGGT
GAAGGACCTGAGAGACTTGTTCAGCAGAGCCAACATGCTGGAGGGCCCCAACGCCC
ACCCCTTCCTGAAGAAGGTGCTGAACAACAACAGCATCAAGGACATCCAGGGCTTCT
ACAGAACCTACCTGGTGGAGGAGCTGAACCAGATCGAGGACAAGCAGAGAAGAATC
GCCAAGGCCAAGAACGTGAAGGACACCGTGAGACAGTTCCCCCTTCGCCCACTTCAA
CAGAATGAGATACCAGAAGAGAGACGAGGACTACTACAGAAACCTGGCCAAGAGAT
ACCTGAACATCGGCGACAACGAGAAGGACAAGGCCGTGATCCTGCTGCCCGACGGC
ATGTTCACCAGCTACATCTACGACCTGATCATGAAGCTGCCCGAGAACAACGAGAAG
ATGAGAATCAACCTGGCCAGCGACGTGGCCCACTGCAACAGCAGCTTCCTGATCAGC
AGATTCTTCGAGAACATCAGAAACGACTACGCCCAGCCCTTCTACAGAGAGGAGAGA
ACCTACGAGCTGTTCAGCATCCTGAACAACAAGAAGGTGAGAAACACCCTGCAGCC
CCTGTTCATCAGCCCCCACGACATCAACATCCAGCTGACCGAGAAGGAGAAGGACG
GCAAGGGCAGACTGATCCTGCAGAAGATCGACCACTTCTGCAAGAGCATCACCCAG
AAGGGCAACTTCAACAACGTGGAGGAGGCCAAGGAGGCCACCAGCAGAAAGCTGA
AGCACCTGATCACCGACTGCAAGAACAACGAGAGAGACATCAGAAGATACAAGACC
CAGGACATGGTGATCTACCTGATGGCCAGAGACATCCTGAAGGACATCATCCCCGAC

AGCGAGAAGGACAAGTACGCCAAGGACAGAAAGCTGCTGCTGAAGGACGTGTGCG

AGGAGGGCTTCCTGAGACAGGCCGTGAAGATGGAGTACGAGTACAGCATCGAGGAG

AAGGGCAAGAGAACCAGAACCGTGAAGATCACCCACCCCAACATGAGCCTGAAGAA

CTACGGCGAGTTCCACAGACTGCTGAACGACGAGAGACTGAAGAGCCTGCTGCAGC

AGCTGGCCAACATGGACGAGATCGACTACACCGACCTGATGGGCGAGTTCGCCGACT

ACGACCAGAAGAGAAGCGAGATCTTCAGACTGGCCCAGAGCATCGAGAAGCACCTG

TACGAGCAGAACGAGCAGGGCCTGAACGACGAGAAGAGCGACCTGTTCTACCACAC

CAGATACAACGGCAAGAAGATCCCCAGAAGAAACAGCTTCAGCAGCCTGCTGGAGC

TGATCGGCGAGGAGGAGAGCCAGATGACCGAGACAGACAAGAAGCAGACCATCAGC

ATCAGAAACGCCTTCGGCCACAACACCTACAAGGTGAGCCTGGCCGAGATGAACGC

CACCGAGCTGCCCAACGTGGCCAAGACCATCCTGAAGAAGATGGAGGAGCTGAGAA

ACAAGCTGACAGGCGGCGGCCCCGGCGGCGGCGCCGCCGCCGGCAGCGGCAGCccta

agaaaaaacgaaaagttggcagcggaagcaaaaggccggcggccacgaaaaaggccggccaggcaaaaaagaaaaagctcgagT

ACCCATACGATGTTCCAGATTACGCTtgagaattcCCCTTGAGCATCTGACTTCTGGCTAAT

AAAGGAAATTTATTTTCATTGCAATAGTGTGTTGGAATTTTTTGTGTCTCTCAggtaccgag

ggcctatttcccatgattccttcatatttgcatatacgatacaaggctgttagagagataattggaattaatttgactgtaaacacaaagatattagt

acaaaatacgtgacgtagaaagtaataatttcttgggtagtttgcagttttaaaattatgttttaaaatggactatcatatgcttaccgtaacttgaa

agtatttcgatttcttggctttatatatcttgtggaaaggacgaaacaccggagaccacggcaggtctcaGTTGTAGAAGCCTAT

CGTTAGGATAGGTATGACAACTTTTTTTgcggccgcaggaacccctagtgatggagttggccactccctctctgcg

cgctcgctcgctcactgaggccgggcgaccaaaggtcgcccgacgcccgggctttgcccgggcggcctcagtgagcgagcgagcgcg

cagctgcctgcaggggcgcctgatgcggtattttctccttacgcatctgtgcggtatttcacaccgcatacgtcaaagcaaccatagtacgcg

ccctgtagcggcgcattaagcgcggcgggtgtggtggttacgcgcagcgtgaccgctacacttgccagcgccttagcgcccgctcctttcg

ctttcttcccttcctttctcgccacgttcgccggctttccccgtcaagctctaaatcggggggctccctttagggttccgatttagtgctttacggca

cctcgaccccaaaaaacttgatttgggtgatggttcacgtagtgggccatcgccctgatagacggtttttcgccctttgacgttggagtccacg

ttctttaatagtggactcttgttccaaactggaacaacactcaactctatctcgggctattcttttgatttataagggattttgccgatttcggtctatt

ggttaaaaaatgagctgatttaacaaaaatttaacgcgaattttaacaaaatattaacgtttacaattttatggtgcactctcagtacaatctgctct

gatgccgcatagttaagccagccccgacacccgccaacacccgctgacgcgccctgacgggcttgtctgctcccggcatccgcttacaga

caagctgtgaccgtctccgggagctgcatgtgtcagaggttttcaccgtcatcaccgaaacgcgcgagacgaaagggcctcgtgatacgc

ctatttttataggttaatgtcatgataataatggtttcttagacgtcaggtggcacttttcggggaaatgtgcgcggaacccctatttgtttattttttct

aaatacattcaaatatgtatccgctcatgagacaataaccctgataaatgcttcaataatattgaaaaaggaagagtatgagtattcaacatttcc

gtgtcgcccttattccctttttgcggcattttgccttcctgttttttgctcacccagaaacgctggtgaaagtaaaagatgctgaagatcagttggg

tgcacgagtgggttacatcgaactggatctcaacagcggtaagatccttgagagttttcgccccgaagaacgttttccaatgatgagcacttt

aaagttctgctatgtggcgcggtattatcccgtattgacgccgggcaagagcaactcggtcgccgcatacactattctcagaatgacttggtt

gagtactcaccagtcacagaaaagcatcttacggatggcatgacagtaagagaattatgcagtgctgccataaccatgagtgataacactgc

ggccaacttacttctgacaacgatcggaggaccgaaggagctaaccgcttttttgcacaacatgggggatcatgtaactcgccttgatcgttg ggaaccggagctgaatgaagccataccaaacgacgagcgtgacaccacgatgcctgtagcaatggcaacaacgttgcgcaaactattaa ctggcgaactacttactctagcttcccggcaacaattaatagactggatggaggcggataaagttgcaggaccacttctgcgctcggcccttc cggctggctggtttattgctgataaatctggagccggtgagcgtggaagccgcggtatcattgcagcactggggccagatggtaagccctc ccgtatcgtagttatctacacgacggggagtcaggcaactatggatgaacgaaatagacagatcgctgagataggtgcctcactgattaagc attggtaactgtcagaccaagtttactcatatatactttagattgatttaaaacttcatttttaatttaaaaggatctaggtgaagatccttttttgataat ctcatgaccaaaatcccttaacgtgagttttcgttccactgagcgtcagaccccgtagaaaagatcaaaggatcttcttgagatcctttttttctg cgcgtaatctgctgcttgcaaacaaaaaaaccaccgctaccagcggtggtttgtttgccggatcaagagctaccaactctttttccgaaggta actggcttcagcagagcgcagataccaaatactgttcttctagtgtagccgtagttaggccaccacttcaagaactctgtagcaccgcctacat acctcgctctgctaatcctgttaccagtggctgctgccagtggcgataagtcgtgtcttaccgggttggactcaagacgatagttaccggata aggcgcagcggtcgggctgaacggggggttcgtgcacacagcccagcttggagcgaacgacctacaccgaactgagatacctacagcg tgagctatgagaaagcgccacgcttcccgaagggagaaaggcggacaggtatccggtaagcggcagggtcggaacaggagagcgcac gagggagcttccaggggaaacgcctggtatctttatagtcctgtcgggtttcgccacctctgacttgagcgtcgatttttgtgatgctcgtca ggggggcggagcctatggaaaaacgccagcaacgcggcctttttacggttcctggcctttttgctggcctttttgctcacatgt

C13-2-BsaI plasmid (SEQ ID NO: 6):

cctgcaggcagctgcgcgctcgctcgctcactgaggccgcccgggcgtcgggcgacctttggtcgcccggcctcagtgagc gagcgagcgcgcagagagggagtggccaactccatcactaggggttcctgcggcctctagactcgaggcgttgacattgattattgactag ttattaatagtaatcaattacggggtcattagttcatagcccatatatggagttccgcgttacataacttacggtaaatggcccgcctggctgacc gcccaacgacccccgcccattgacgtcaataatgacgtatgttcccatagtaacgccaatagggactttccattgacgtcaatgggtggagta tttacggtaaactgcccacttggcagtacatcaagtgtatcatatgccaagtacgccccctattgacgtcaatgacggtaaatggcccgcctg gcattatgcccagtacatgaccttatgggactttcctacttggcagtacatctacgtattagtcatcgctattaccatggtgatgcggttttggca gtacatcaatgggcgtggatagcggtttgactcacggggatttccaagtctccacccattgacgtcaatgggagtttgttttggcaccaaaat caacgggactttccaaaatgtcgtaacaactccgccccattgacgcaaatgggcggtaggcgtgtacggtgggaggtctatataagcagag ctctctggctaactaccggtgccaccatgccggcagctaagaaaaagaaactggatggcagcgtcgacATGAGCAAGGACA AGAAAACCAAGGCCAAGAGAATGGGCGTGAAGGCCCTGCTGGCCCACGGCGAGGA CAAGCTGACCATGACCACCTTCGGCAAGGGCAACAGAAGCAAGATCGAGTTCACCG AGGGCTACCACGGCAGAGCCCTGGAGACACCCAAGCACTTCGGCATCAGAGGCTTC GAGGTGAGAAGAATCGACGAGAACGTGGACCTGTGCGGCGACCTGGAGGAGGGCA AGACCATCGAGGCCCTGCTGGTGAACCCCAGCGAGAAGGTGGGCGAGGACTACCTG AAGCTGAAGGGCACCCTGGAGAAGAGATTCTTCGGCAGAGAGTTCCCCCACGACAA CATCAGAATCCAGCTGATCTACAACATCCTGGACATCTACAAGATCCTGGGCATGAAC GTGGCCGACATCCTGTACGCCCTGGGCAACATGCAGGACACCGAGCTGGACATCGAC ATGTTCGGCCAGAGCCTGAACAACGAGGACAACCTGAAGGAGTGCCTGAAGAGAAT

GAGGCCCTACATGGGCTACTTCGGCGACATCTTCAAGATCAGCCCCAAGGGCGAGAA

CATCGCCGACAGAGAGCACAACAAGAAGGTGCTGAGATGCATCAGCGTGCTGAGAA

ACGCCACCGCCCACGACAAGCAGGACGAGTACCCCTGGTTCAAGAGCAGCGACATC

TACGAGACAAAGATCTTCAAGGCCGACATGTGGAAGATCATCAAGGACCAGTACAGA

GAGAAGATCAAGAAGGTGAACAAGGACTTCCTGAGCAAGAACGCCGTGAACATGGC

CATCCTGTTCGACCTGCTGAACGCCAGAGACGTGGAGCAGAAGAAGCAGATCACCG

ACGAGTTCTACAGATTCACCATCAGAAAGGACGGCAAGAACCTGGGCATGAACCTG

GTGAAGATCAGAGAGATCATCATCGACAGATACGCCAGCGGCCTGAGAGACAAGAA

GCACGACCCCCACAGACAGAAGATCAACGTGATCGCCGACTTCCTGATCTTCAGAGC

CCTGAGCCAGAACCAGGGCATCATCGACAAGACCGTGAGCAGCCTGAGACTGACCA

AGGACGAGGAGGAGAAGGACCACGTGTACCAGAACGCCGCCGAGCTGGTGTGGGG

CATGGTGAGCAACTGCCTGACCCCCTACTTCAACGACCCCAAGAACAAGTACATCCT

GAAGTACAAGGACGCCAAGACCCCCGGCGACTTCGAGGACTGGATCACCAGCAAGA

TCAGCGAGGACGACGGCGAGCCCTTCGTGAAGGTGCTGAGCTTCCTGTGCAACTTCC

TGGAGGGCAAGGAGATCAACGAGCTGCTGACCGCCTACATCCACAAGTTCGAGTGC

ATCCAGGACTTCCTGAACGTGATCAGCAGCCTGGGCGAGAACGTGCAGTTCCAGCCC

AGATTCGCCCTGTTCAACAACGCCAGCTTCGCCCAGAACGTGGCCGTGCAGCTGAGA

ATCCTGGCCAGCATCGGCAAGATGAAGCCCGACCTGACCGAGGCCAAGAGGCCCCT

GTACAAGGCCGCCATCAGAATGCTGTGCCCCCCCGAGAAGTGGGAGAAGTACACCA

GCGACGAGTGGCTGGAGAAGAACATGCTGCTGAACAGCGAGGACAGAAAGAACGA

CAAGAAGAAGAAGCAGGTGAACCCCTTCAGAAACTTCATCGCCGGCAACGTGATCG

AGAGCAGAAGATTCATGTACCTGGTGAGATACAGCAAGCCCAAGGCCGTGAGAGCC

ATCATGCAGAACAGAAGCATCGTGAACTACGTGCTGCACAGACTGCCCAGCGAGCA

GGTGCACAGATACGCCAGCGTGTTCCCCGAGAACTTCGCCGACCTGGAGCAGGAGAT

CGACTTCCTGACCAAGAAGCTGTTCGAGTTCAGCTTCGAGGAGCTGCTGCACGAGA

AGGACGTGATCCTGAACAACAGCAGAAGCCACAAGCCCAGCCTGGAGATCGAGAGA

CTGAAGGCCATCACCGGCCTGTACCTGAGCGTGGCCTACATCGCCATCAAGAACATC

GTGAAGGCCAACGCCAGATACTACATCGCCTTCGCCGTGTTCGAGAGAGACAAGGAG

CTGGTGAAGGCCAAGGACGCCAGAATCCAGACCAAGATCCCCGAGACAGACTTCCC

CGACTACTTCTGCCTGACCCAGTACTACCTGGACAGAGACGAGGAGAAGAAGTTCCC

CGGCGACCCCAGAGACAAGGAGGCCTTCTTCGAGCACCTGAGAAAGACCAAGAGAC

ACTTCAGCAAGCAGTGGAGAGAGTGGCTGAACGAGAAGATCGCCGACGCCAAGAGC

AGCCAGGCCACCGGCCTGCTGCTGAGAGAGGCCAGAAACGACGTGGAGCACCTGAA

CGTGCTGAGAGCCATCCCCGACTACATCCAGGACTTCAGACACGGCGAGAAGGGCG

AGACAGCCATGAACAGCTACTTCGAGCTGTACCACTACCTGATGCAGAGACTGATGC
TGAAGAACACCGAGCTGGACCTGAGCCACTGGAGCGGCTGGATCATGAGAAGCGGC
AGACCCGACAGAGACTTGATCCAGATCGCCTTCGTGAGCCTGGCCTACAACCTGCCC
AGATACAGAAACCTGACCAAGGAGCACCACTTCGACGACACCGTGCTGCAGAAGAT
CAGAGAGAAGGAGAGCCTGGACACAGGCGGCGGCCCCGGCGGCGGCGCCGCCGCC
GGCAGCGGCAGCcctaagaaaaaacgaaaagttggcagcggaagcaaaaggccggcggccacgaaaaaggccggccagg
caaaaaagaaaaagctcgagTACCCATACGATGTTCCAGATTACGCTtgagaattcCCCTTGAGCATCT
GACTTCTGGCTAATAAAGGAAATTTATTTTCATTGCAATAGTGTGTTGGAATTTTTTGT
GTCTCTCAggtaccgagggcctatttcccatgattccttcatatttgcatatacgatacaaggctgttagagagataattggaattaatttg
actgtaaacacaaagatattagtacaaaatacgtgacgtagaaagtaataatttcttgggtagtttgcagttttaaaattatgttttaaaatggacta
tcatatgcttaccgtaacttgaaagtatttcgatttcttggctttatatatcttgtggaaaggacgaaacaccGGAAGATAACTCTAC
AAACCTGTAGGGTTCTGAGACggagaccacggcaggtctcaTTTTTTgcggccgcaggaacccctagtgatgga
gttggccactccctctctgcgcgctcgctcgctcactgaggccgggcgaccaaaggtcgcccgacgcccgggctttgcccgggcggcct
cagtgagcgagcgagcgcgcagctgcctgcaggggcgcctgatgcggtattttctccttacgcatctgtgcggtatttcacaccgcatacgt
caaagcaaccatagtacgcgccctgtagcggcgcattaagcgcggcgggtgtggtggttacgcgcagcgtgaccgctacacttgccagc
gccttagcgcccgctcctttcgctttcttcccttcctttctcgccacgttcgccggctttccccgtcaagctctaaatcggggggctcccttaggg
ttccgatttagtgctttacggcacctcgaccccaaaaaacttgatttgggtgatggttcacgtagtgggccatcgccctgatagacggtttttcg
ccctttgacgttggagtccacgttctttaatagtggactcttgttccaaactggaacaacactcaactctatctcgggctattcttttgatttataag
ggattttgccgatttcggtctattggttaaaaaatgagctgatttaacaaaaatttaacgcgaattttaacaaaatattaacgtttacaattttatggt
gcactctcagtacaatctgctctgatgccgcatagttaagccagccccgacacccgccaacacccgctgacgcgccctgacgggcttgtct
gctcccggcatccgcttacagacaagctgtgaccgtctccgggagctgcatgtgtcagaggttttcaccgtcatcaccgaaacgcgcgaga
cgaaagggcctcgtgatacgcctatttttataggttaatgtcatgataataatggtttcttagacgtcaggtggcacttttcggggaaatgtgcgc
ggaacccctatttgtttattttctaaatacattcaaatatgtatccgctcatgagacaataaccctgataaatgcttcaataatattgaaaaaggaa
gagtatgagtattcaacatttccgtgtcgcccttattcccttttttgcggcattttgccttcctgtttttgctcacccagaaacgctggtgaaagtaa
aagatgctgaagatcagttgggtgcacgagtgggttacatcgaactggatctcaacagcggtaagatccttgagagttttcgccccgaagaa
cgttttccaatgatgagcacttttaaagttctgctatgtggcgcggtattatcccgtattgacgccgggcaagagcaactcggtcgccgcatac
actattctcagaatgacttggttgagtactcaccagtcacagaaaagcatcttacggatggcatgacagtaagagaattatgcagtgctgccat
aaccatgagtgataacactgcggccaacttacttctgacaacgatcggaggaccgaaggagctaaccgcttttttgcacaacatggggggatc
atgtaactcgccttgatcgttgggaaccggagctgaatgaagccataccaaacgacgagcgtgacaccacgatgcctgtagcaatggcaa
caacgttgcgcaaactattaactggcgaactacttactctagcttcccggcaacaattaatagactggatggaggcggataaagttgcagga
ccacttctgcgctcggcccttccggctggctggtttattgctgataaatctggagccggtgagcgtggaagccgcggtatcattgcagcactg
gggccagatggtaagccctcccgtatcgtagttatctacacgacggggagtcaggcaactatggatgaacgaaatagacagatcgctgag
ataggtgcctcactgattaagcattggtaactgtcagaccaagtttactcatatatactttagattgatttaaaacttcattttttaatttaaaaggatct
aggtgaagatcctttttgataatctcatgaccaaaatcccttaacgtgagttttcgttccactgagcgtcagaccccgtagaaaagatcaaagg

atcttcttgagatcctttttttctgcgcgtaatctgctgcttgcaaacaaaaaaaccaccgctaccagcggtggtttgtttgccggatcaagagct accaactcttttccgaaggtaactggcttcagcagagcgcagataccaaatactgttcttctagtgtagccgtagttaggccaccacttcaag aactctgtagcaccgcctacatacctcgctctgctaatcctgttaccagtggctgctgccagtggcgataagtcgtgtcttaccgggttggact caagacgatagttaccggataaggcgcagcggtcgggctgaacggggggttcgtgcacacagcccagcttggagcgaacgacctacac cgaactgagatacctacagcgtgagctatgagaaagcgccacgcttcccgaagggagaaaggcggacaggtatccggtaagcggcagg gtcggaacaggagagcgcacgagggagcttccaggggggaaacgcctggtatctttatagtcctgtcgggtttcgccacctctgacttgagc gtcgattttgtgatgctcgtcaggggggcggagcctatggaaaaacgccagcaacgcggcctttttacggttcctggccttttgctggccttt tgctcacatgt

Unordered sequence (SEQ ID NO: 7):
ggagaccacggcaggtctca

Guide sequence of gRNA-1 (SEQ ID NO: 9):
GGTACTCCTGGAAGATGTCCACCAGGGTCT

Guide sequence of gRNA-2 (SEQ ID NO: 10):
CCACTGCGGCCCCCTCTCCTCTTCCTTC

Guide sequence of gRNA-3 (SEQ ID NO: 11):
GGCTGGAGCACTGTCTGCGCACA

Guide sequence of gRNA-4 (SEQ ID NO: 12):
AAGCTCATCTCTCCTATGTGCTGGC

Guide sequence of gRNA-5 (SEQ ID NO: 13):
TGGGTGCAGCCTGGGACCACTTGGCATGG

Guide sequence of gRNA-6 (SEQ ID NO: 14):
TCTTTCTTTGGTCTGCATTCACAT

Guide sequence of gRNA-7 (SEQ ID NO: 15):
GGCCTTGGTGAGGTTTGATCCGCAT

Guide sequence of gRNA-8 (SEQ ID NO: 16):
ACAAACAAATGCTTTCTCCGCTCTGA

Guide sequence of gRNA-9 (SEQ ID NO: 17):
GCAGGAACATTTACACGTCTGCGGATCT

Guide sequence of gRNA-10 (SEQ ID NO: 18):
AGTACGTTCGTTTAACTCAAGCTGCC

Direct repeat sequence of gRNA encoded by Cas13m.3 vector (SEQ ID NO: 19):
GTTGTAGAAGCCGTTCATTCGGGACGGTATGACAAC

Direct repeat sequence of gRNA encoded by Cas13m.6 vector (SEQ ID NO: 20):
GTTGTAGAAGCCTATCGTTAGGATAGGTATGACAAC

Direct repeat sequence of gRNA encoded by C13-2 vector (SEQ ID NO: 21):
GGAAGATAACTCTACAAACCTGTAGGGTTCTGAGAC

qGAPDHF1 primer (SEQ ID NO: 22):
CCATGGGGAAGGTGAAGGTC

qGAPDHR1 primer (SEQ ID NO: 23):
GAAGGGGTCATTGATGGCAAC

qVegfa-F1 primer (SEQ ID NO: 24):
ACCTCCACCATGCCAAGTGG

qVegfa-R1 primer (SEQ ID NO: 25):
CAGGGTCTCGATTGGATGGC

qVegfa-F2 primer (SEQ ID NO: 26):
CAATGACGAGGGCCTGGAGT

qVegfa-R2 primer (SEQ ID NO: 27):
TCTTTGGTCTGCATTCACAT

pAAV-CasRx-gRNA plasmid (SEQ ID NO: 28):

cctgcaggcagctgcgcgctcgctcgctcactgaggccgcccgggcgtcgggcgacctttggtcgcccggcctcagtgagc

gagcgagcgcgcagagagggagtggccaactccatcactaggggttcctgcggcctctagactcgaggcgttgacattgattattgactag

ttattaatagtaatcaattacggggtcattagttcatagcccatatatggagttccgcgttacataacttacggtaaatggcccgcctggctgacc

gcccaacgaccccgcccattgacgtcaataatgacgtatgttcccatagtaacgccaatagggactttccattgacgtcaatgggtggagta

tttacggtaaactgcccacttggcagtacatcaagtgtatcatatgccaagtacgccccctattgacgtcaatgacggtaaatggcccgcctg

gcattatgcccagtacatgaccttatgggactttcctacttggcagtacatctacgtattagtcatcgctattaccatggtgatgcggttttggca

gtacatcaatgggcgtggatagcggtttgactcacggggatttccaagtctccaccccattgacgtcaatgggagtttgttttggcaccaaaat

caacgggactttccaaaatgtcgtaacaactccgccccattgacgcaaatgggcggtaggcgtgtacggtgggaggtctatataagcagag

ctctctggctaactaccggtgccaccATGAGCCCCAAGAAGAAGAGAAAGGTGGAGGCCAGCATCG

AAAAAAAAAGTCCTTCGCCAAGGGCATGGGCGTGAAGTCCACACTCGTGTCCGGC

TCCAAAGTGTACATGACAACCTTCGCCGAAGGCAGCGACGCCAGGCTGGAAAAGAT

CGTGGAGGGCGACAGCATCAGGAGCGTGAATGAGGGCGAGGCCTTCAGCGCTGAAA

TGGCCGATAAAAACGCCGGCTATAAGATCGGCAACGCCAAATTCAGCCATCCTAAGG

GCTACGCCGTGGTGGCTAACAACCCTCTGTATACAGGACCCGTCCAGCAGGATATGCT

CGGCCTGAAGGAAACTCTGGAAAAGAGGTACTTCGGCGAGAGCGCTGATGGCAATG

ACAATATTTGTATCCAGGTGATCCATAACATCCTGGACATTGAAAAAATCCTCGCCGA

ATACATTACCAACGCCGCCTACGCCGTCAACAATATCTCCGGCCTGGATAAGGACATT

ATTGGATTCGGCAAGTTCTCCACAGTGTATACCTACGACGAATTCAAAGACCCCGAGC

ACCATAGGGCCGCTTTCAACAATAACGATAAGCTCATCAACGCCATCAAGGCCCAGTA

TGACGAGTTCGACAACTTCCTCGATAACCCCAGACTCGGCTATTTCGGCCAGGCCTTT

TTCAGCAAGGAGGGCAGAAATTACATCATCAATTACGGCAACGAATGCTATGACATTC

TGGCCCTCCTGAGCGGACTGAGGCACTGGGTGGTCCATAACAACGAAGAAGAGTCC

AGGATCTCCAGGACCTGGCTCTACAACCTCGATAAGAACCTCGACAACGAATACATC

TCCACCCTCAACTACCTCTACGACAGGATCACCAATGAGCTGACCAACTCCTTCTCCA

AGAACTCCGCCGCCAACGTGAACTATATTGCCGAAACTCTGGGAATCAACCCTGCCG

AATTCGCCGAACAATATTTCAGATTCAGCATTATGAAAGAGCAGAAAAACCTCGGATT

CAATATCACCAAGCTCAGGGAAGTGATGCTGGACAGGAAGGATATGTCCGAGATCAG

GAAAAATCATAAGGTGTTCGACTCCATCAGGACCAAGGTCTACACCATGATGGACTTT

GTGATTTATAGGTATTACATCGAAGAGGATGCCAAGGTGGCTGCCGCCAATAAGTCCC

TCCCCGATAATGAGAAGTCCCTGAGCGAGAAGGATATCTTTGTGATTAACCTGAGGGG

CTCCTTCAACGACGACCAGAAGGATGCCCTCTACTACGATGAAGCTAATAGAATTTGG

AGAAAGCTCGAAAATATCATGCACAACATCAAGGAATTTAGGGGAAACAAGACAAG

AGAGTATAAGAAGAAGGACGCCCCTAGACTGCCCAGAATCCTGCCCGCTGGCCGTGA
TGTTTCCGCCTTCAGCAAACTCATGTATGCCCTGACCATGTTCCTGGATGGCAAGGAG
ATCAACGACCTCCTGACCACCCTGATTAATAAATTCGATAACATCCAGAGCTTCCTGA
AGGTGATGCCTCTCATCGGAGTCAACGCTAAGTTCGTGGAGGAATACGCCTTTTTCAA
AGACTCCGCCAAGATCGCCGATGAGCTGAGGCTGATCAAGTCCTTCGCTAGAATGGG
AGAACCTATTGCCGATGCCAGGAGGGCCATGTATATCGACGCCATCCGTATTTTAGGA
ACCAACCTGTCCTATGATGAGCTCAAGGCCCTCGCCGACACCTTTTCCCTGGACGAG
AACGGAAACAAGCTCAAGAAAGGCAAGCACGGCATGAGAAATTTCATTATTAATAAC
GTGATCAGCAATAAAAGGTTCCACTACCTGATCAGATACGGTGATCCTGCCCACCTCC
ATGAGATCGCCAAAAACGAGGCCGTGGTGAAGTTCGTGCTCGGCAGGATCGCTGAC
ATCCAGAAAAAACAGGGCCAGAACGGCAAGAACCAGATCGACAGGTACTACGAAAC
TTGTATCGGAAAGGATAAGGGCAAGAGCGTGAGCGAAAAGGTGGACGCTCTCACAA
AGATCATCACCGGAATGAACTACGACCAATTCGACAAGAAAAGGAGCGTCATTGAGG
ACACCGGCAGGGAAAACGCCGAGAGGGAGAAGTTTAAAAAGATCATCAGCCTGTAC
CTCACCGTGATCTACCACATCCTCAAGAATATTGTCAATATCAACGCCAGGTACGTCAT
CGGATTCCATTGCGTCGAGCGTGATGCTCAACTGTACAAGGAGAAAGGCTACGACAT
CAATCTCAAGAAACTGGAAGAGAAGGGATTCAGCTCCGTCACCAAGCTCTGCGCTG
GCATTGATGAAACTGCCCCCGATAAGAGAAAGGACGTGGAAAAGGAGATGGCTGAA
AGAGCCAAGGAGAGCATTGACAGCCTCGAGAGCGCCAACCCCAAGCTGTATGCCAA
TTACATCAAATACAGCGACGAGAAGAAAGCCGAGGAGTTCACCAGGCAGATTAACA
GGGAGAAGGCCAAAACCGCCCTGAACGCCTACCTGAGGAACACCAAGTGGAATGTG
ATCATCAGGGAGGACCTCCTGAGAATTGACAACAAGACATGTACCCTGTTCAGAAAC
AAGGCCGTCCACCTGGAAGTGGCCAGGTATGTCCACGCCTATATCAACGACATTGCC
GAGGTCAATTCCTACTTCCAACTGTACCATTACATCATGCAGAGAATTATCATGAATGA
GAGGTACGAGAAAAGCAGCGGAAAGGTGTCCGAGTACTTCGACGCTGTGAATGACG
AGAAGAAGTACAACGATAGGCTCCTGAAACTGCTGTGTGTGCCTTTCGGCTACTGTAT
CCCCAGGTTTAAGAACCTGAGCATCGAGGCCCTGTTCGATAGGAACGAGGCCGCCAA
GTTCGACAAGGAGAAAAAGAAGGTGTCCGGCAATTCCGGATCCGGACCTAAGAAAA
AGAGGAAGGTGGCGGCCGCTTACCCATACGATGTTCCAGATTACGCTtgaggtaccctagagct
cgctgatcagcctcgactgtgccttctagttgccagccatctgttgtttgcccctccccgtgccttccttgaccctggaaggtgccactcccac
tgtcctttcctaataaaatgaggaaattgcatcgcattgtctgagtaggtgtcattctattctggggggtgggtggggcaggacagcaaggg
ggaggattgggaagagaatagcaggcatgctggggaGAGGGCCTATTTCCCATGATTCCTTCATATTTGCA
TATACGATACAAGGCTGTTAGAGAGATAATTGGAATTAATTTGACTGTAAACACAAAG
ATATTAGTACAAAATACGTGACGTAGAAAGTAATAATTTCTTGGGTAGTTTGCAGTTTT

AAAATTATGTTTTAAAATGGACTATCATATGCTTACCGTAACTTGAAAGTATTTCGATTT
CTTGGCTTTATATATCTTGTGGAAAGGACGAAACACCGCAAGTAAACCCCTACCAACT
GGTCGGGGTTTGAAACGGGTCTTCGAGAAGACCTCAAGTAAACCCCTACCAACTGGT
CGGGGTTTGAACTTTTTTTcccgggaatggccgcaggaaccccctagtgatggagttggccactccctctctgcgcgctc
gctcgctcactgaggccgggcgaccaaaggtcgcccgacgcccgggctttgcccgggcggcctcagtgagcgagcgagcgcgcagct
gcctgcaggggcgcctgatgcggtattttctccttacgcatctgtgcggtatttcacaccgcatacgtcaaagcaaccatagtacgcgccctg
tagcggcgcattaagcgcggcgggtgtggtggttacgcgcagcgtgaccgctacacttgccagcgccctagcgcccgctcctttcgctttc
ttcccttcctttctcgccacgttcgccggctttcccccgtcaagctctaaatcggggggctccctttagggttccgatttagtgctttacggcacctc
gacccccaaaaaacttgatttgggtgatggttcacgtagtgggccatcgccctgatagacggttttttcgccctttgacgttggagtccacgttctt
taatagtggactcttgttccaaactggaacaacactcaacccctatctcgggctattcttttgatttataagggattttgccgatttcggcctattggt
taaaaaatgagctgatttaacaaaaatttaacgcgaattttaacaaaatattaacgtttacaattttatggtgcactctcagtacaatctgctctgat
gccgcatagttaagccagccccgacacccgccaacacccgctgacgcgccctgacgggcttgtctgctcccggcatccgcttacagacaa
gctgtgaccgtctccgggagctgcatgtgtcagaggttttcaccgtcatcaccgaaacgcgcgagacgaaagggcctcgtgatacgcctat
ttttataggttaatgtcatgataataatggtttcttagacgtcaggtggcacttttcggggaaatgtgcgcggaacccctatttgtttatttttctaaa
tacattcaaatatgtatccgctcatgagacaataacccctgataaatgcttcaataatattgaaaaaggaagagtatgagtattcaacatttccgtg
tcgcccttattcccttttttgcggcattttgccttcctgttttttgctcacccagaaacgctggtgaaagtaaaagatgctgaagatcagttgggtgc
acgagtgggttacatcgaactggatctcaacagcggtaagatccttgagagttttcgccccgaagaacgttttccaatgatgagcacttttaaa
gttctgctatgtggcgcggtattatcccgtattgacgccgggcaagagcaactcggtcgccgcatacactattctcagaatgacttggttgagt
actcaccagtcacagaaaagcatcttacggatggcatgacagtaagagaattatgcagtgctgccataaccatgagtgataacactgcggc
caacttacttctgacaacgatcggaggaccgaaggagctaaccgcttttttgcacaacatggggggatcatgtaactcgccttgatcgttggga
accggagctgaatgaagccataccaaacgacgagcgtgacaccacgatgcctgtagcaatggcaacaacgttgcgcaaactattaactgg
cgaactacttactctagcttcccggcaacaattaatagactggatggaggcggataaagttgcaggaccacttctgcgctcggcccttccgg
ctggctggtttattgctgataaatctggagccggtgagcgtggaagccgcggtatcattgcagcactggggccagatggtaagccctcccgt
atcgtagttatctacacgacggggagtcaggcaactatggatgaacgaaatagacagatcgctgagataggtgcctcactgattaagcattg
gtaactgtcagaccaagtttactcatatatactttagattgatttaaaacttcatttttaatttaaaaggatctaggtgaagatcctttttgataatctca
tgaccaaaatcccttaacgtgagttttcgttccactgagcgtcagacccccgtagaaaagatcaaaggatcttcttgagatcctttttttctgcgcg
taatctgctgcttgcaaacaaaaaaaccaccgctaccagcggtggtttgtttgccggatcaagagctaccaactctttttccgaaggtaactgg
cttcagcagagcgcagataccaaatactgtccttctagtgtagccgtagttaggccaccacttcaagaactctgtagcaccgcctacatacct
cgctctgctaatcctgttaccagtggctgctgccagtggcgataagtcgtgtcttaccgggttggactcaagacgatagttaccggataaggc
gcagcggtcgggctgaacggggggttcgtgcacacagcccagcttggagcgaacgacctacaccgaactgagatacctacagcgtgag
ctatgagaaagcgccacgcttcccgaagggagaaaggcggacaggtatccggtaagcggcagggtcggaacaggagagcgcacgag
ggagcttccaggggggaaacgcctggtatctttatagtcctgtcgggtttcgccacctctgacttgagcgtcgatttttgtgatgctcgtcaggg
gggcggagcctatggaaaaacgccagcaacgcggcctttttacggttcctggccttttgctggccttttgctcacatgt

Guide sequence of RJ-2 (SEQ ID NO: 29):
GCAGCCTGGGACCACTTGGCATGGTGGAG

Guide sequence of RJ-5 (SEQ ID NO: 30):
CCTGGAAGATGTCCACCAGGGTCTCGATTG

Guide sequence of RJ-8 (SEQ ID NO: 31):
ATGTTGGACTCCTCAGTGGGCACACACTCC

Tandem RJ-5+RJ-8 (SEQ ID NO: 32):

CCTGGAAGATGTCCACCAGGGTCTCGATTGcaagtaaacccctaccaactggtcggggtttgaaa

cATGTTGGACTCCTCAGTGGGCACACACTCC

Guide sequence of X-1 (SEQ ID NO: 33):
GTGCTGTAGGAAGCTCATCTCTCCTATGTG

Guide sequence of X-2 (SEQ ID NO: 34):
GGTACTCCTGGAAGATGTCCACCAGGGTCT

X-dual (tandem X-1+X-2) (SEQ ID NO: 35):

GGTACTCCTGGAAGATGTCCACCAGGGTCTcaagtaaacccctaccaactggtcggggtttgaaac

GTGCTGTAGGAAGCTCATCTCTCCTATGTG

qmVA-F primer (SEQ ID NO: 36):
CTTCAAGCCGTCCTGTGTGC

qmVA-R primer (SEQ ID NO: 37):
GCTCATCTCTCCTATGTGCT

mGAPDH-F primer (SEQ ID NO: 38):
GCTGAGTATGTCGTGGAGTCTA

mGAPDH-R primer (SEQ ID NO: 39):
GTGGTTCACACCCATCACAA

The sequences of SEQ ID NO: 40 to SEQ ID NO: 235 are as described above.

Cas13X.1-dual related sequence (tandem X-1+X-2 guide sequence for Cas13X.1 related vector construction) (SEQ ID NO: 236):

GTGCTGTAGGAAGCTCATCTCTCCTATGTGGCTGGAGCAGCCCCCGATTTGTG

GGGTGATTACAGCGGTACTCCTGGAAGATGTCCACCAGGGTCT

Guide sequence of gRNA-luc targeting luciferase gene (SEQ ID NO: 237):
GAATGTAGCCATCCATCCTTGTCAA

Cas13X.1-V vector (SEQ ID NO: 8):

cctgcaggcagctgcgcgctcgctcgctcactgaggccgcccgggcgtcgggcgacctttggtcgcccggcctcagtgagc
gagcgagcgcgcagagagggagtggccaactccatcactaggggttcctgcggcctctagactcgaggcgttgacattgattattgactag
ttattaatagtaatcaattacggggtcattagttcatagcccatatatggagttccgcgttacataacttacggtaaatggcccgcctggctgacc
gcccaacgacccccgcccattgacgtcaataatgacgtatgttcccatagtaacgccaataggggactttccattgacgtcaatgggtggagta
tttacggtaaactgcccacttggcagtacatcaagtgtatcatatgccaagtacgccccctattgacgtcaatgacggtaaatggcccgcctg
gcattatgcccagtacatgaccttatgggactttcctacttggcagtacatctacgtattagtcatcgctattaccatggtgatgcggttttggca
gtacatcaatgggcgtggatagcggtttgactcacggggatttccaagtctccaccccattgacgtcaatgggagtttgttttggcaccaaaat
caacgggactttccaaaatgtcgtaacaactccgccccattgacgcaaatgggcggtaggcgtgtacggtgggaggtctatataagcagag
ctctctggctaactaccggtgccaccatgccggcagctaagaaaaagaaactggatggcagcgtcgacGCCCAGGTGTCCA
AACAGACCAGCAAGAAAAGGGAACTGAGCATCGATGAGTACCAGGGTGCTAGAAAG
TGGTGCTTCACAATCGCCTTTAACAAAGCTCTGGTCAATAGGGATAAAAACGATGGCC
TCTTCGTGGAGTCCCTGCTGAGACACGAGAAGTACAGCAAACACGACTGGTACGAC
GAGGATACCAGAGCCCTGATCAAGTGCTCCACTCAGGCCGCCAATGCAAAGGCCGA
GGCTCTGAGAAATTATTTCAGCCACTACCGGCACTCCCCCGGGTGTCTGACCTTCACT
GCAGAGGATGAGCTTAGGACAATTATGGAGCGCGCCTACGAGAGGGCCATTTTTGAG
TGTCGGCGAAGAGAAACTGAGGTGATCATCGAGTTTCCATCTCTGTTTGAAGGGGAC
CGCATCACAACCGCCGGAGTGGTATTTTTTGTGAGTTTCTTCGTGGAACGGAGAGTG
CTGGACCGGCTGTACGGGGCAGTTAGTGGCCTGAAGAAGAACGAGGGGCAGTACAA
GCTGACAAGGAAAGCTCTGAGCATGTACTGTCTGAAAGATTCCAGATTTACAAAGGC
ATGGGATAAGAGAGTGCTCCTCTTTCGCGATATCCTGGCCCAGCTGGGCAGAATCCCA
GCTGAGGCTTACGAGTACTACCACGGCGAACAGGGCGACAAAAAGAGGGCCAACGA
CAATGAGGGCACCAACCCCAAAAGGCACAAGGATAAGTTTATTGAGTTTGCACTGCA

CTACCTGGAGGCCCAGCACTCTGAGATCTGCTTTGGCAGAAGGCACATCGTGAGAGA
GGAAGCTGGCGCCGGCGATGAGCACAAGAAGCACAGAACCAAAGGGAAGGTGGTG
GTGGACTTCTCCAAGAAGGATGAGGATCAGTCTTACTACATCAGTAAGAACAACGTG
ATTGTGAGGATCGATAAGAATGCCGGCCCTCGCTCCTACCGCATGGGGCTCAATGAAC
TGAAGTACCTGGTCCTGCTCTCTGCAGGGGAAGGGCGATGACGCTATCGCCAAGC
TGTATAGGTATCGGCAGCATGTGGAGAACATTCTGGACGTGGTGAAGGTGACTGATAA
AGATAACCACGTGTTCCTGCCAAGGTTTGTGCTCGAACAGCACGGAATCGGCCGGAA
GGCCTTCAAGCAGAGAATTGACGGGCGCGTGAAACATGTGCGCGGGGTGTGGGAAA
AGAAGAAGGCCGCCACAAACGAGATGACGCTGCACGAGAAGGCCCGCGACATCCTC
CAGTACGTGAATGAGAATTGCACTCGCAGCTTCAATCCTGGCGAGTATAACAGGCTG
CTGGTGTGCCTGGTGGGTAAAGACGTGGAGAACTTCCAGGCCGGCCTGAAGCGGCT
TCAGCTGGCTGAGAGAATTGACGGCCGGGTGTATTCTATCTTCGCCCAGACTTCTACC
ATCAATGAAATGCACCAGGTGGTGTGTGACCAGATCCTCAATCGGCTGTGTCGAATTG
GGGATCAGAAGCTGTACGACTATGTGGGCCTGGGCAAGAAAGATGAGATCGACTATA
AACAGAAAGTGGCTTGGTTCAAAGAGCACATCAGTATCAGACGCGGCTTTCTGAGGA
AGAAATTCTGGTACGACTCTAAAAAGGGCTTCGCTAAGCTGGTGGAGGAGCACCTGG
AATCCGGAGGCGGACAGCGGGATGTGGGGCTGGATAAGAAATACTACCACATCGATG
CCATCGGGAGATTCGAGGGCGCCAATCCTGCTCTGTACGAAACCCTGGCCAGGGACA
GGCTGTGCCTGATGATGGCCCAGTACTTTCTGGGCTCCGTTCGCAAAGAGCTGGGAA
ACAAAATTGTGTGGAGCAACGACTCCATAGAGCTGCCGGTGGAGGGATCTGTCGGGA
ATGAAAAATCGATCGTGTTCTCTGTGAGCGATTATGGAAAACTGTACGTCCTGGACGA
TGCCGAGTTTCTGGGTAGAATCTGTGAGTATTTTATGCCACATGAAAAGGGCAAGATC
AGATACCACACCGTGTATGAAAAGGGGTTCAGAGCCTACAACGACCTGCAGAAAAA
ATGCGTGGAGGCCGTGCTGGCATTCGAAGAGAAGGTGGTGAAGGCCAAGAAAATGT
CTGAGAAAGAGGGAGCCCACTACATCGACTTTCGCGAGATCCTGGCCCAGACCATGT
GCAAGGAAGCTGAAAAGACAGCCGTCAATAAGGTGAGGAGGGCCTTCTTTCACCAC
CACCTTAAATTCGTGATCGACGAATTTGGCCTGTTCAGCGATGTGATGAAAAAATACG
GCATCGAGAAGGAGTGGAAGTTTCCAGTGAAAACAGGCGGCGGCCCCGGCGGCGGC
GCCGCCGCCGGCAGCGGCAGCcctaagaaaaaacgaaaagttggcagcggaagcaaaaggccggcggccacgaa
aaaggccggccaggcaaaaaagaaaaagctcgagTACCCATACGATGTTCCAGATTACGCTtgagaattcCCC
TTGAGCATCTGACTTCTGGCTAATAAAGGAAATTTATTTTCATTGCAATAGTGTGTTGG
AATTTTTTGTGTCTCTCAggtaccgagggcctatttcccatgattccttcatatttgcatatacgatacaaggctgttagagaga
taattggaattaatttgactgtaaacacaaagatattagtacaaaatacgtgacgtagaaagtaataatttcttgggtagtttgcagttttaaaatta
tgttttaaaatggactatcatatgcttaccgtaacttgaaagtatttcgatttcttggctttatatatcttgtggaaaggacgaaacaccggagacc

acggcaggtctcagctggagcagcccccgatttgtggggtgattacagcTTTTTTgcggccgcaggaacccctagtgatggagttgg

ccactccctctctgcgcgctcgctcgctcactgaggccgggcgaccaaaggtcgcccgacgcccgggctttgcccgggcggcctcagtg

agcgagcgagcgcgcagctgcctgcaggggcgcctgatgcggtattttctccttacgcatctgtgcggtatttcacaccgcatacgtcaaag

caaccatagtacgcgccctgtagcggcgcattaagcgcggcgggtgtggtggttacgcgcagcgtgaccgctacacttgccagcgcctta

gcgcccgctcctttcgctttcttcccttccttctcgccacgttcgccggctttccccgtcaagctctaaatcggggggctccctttagggttccga

tttagtgctttacggcacctcgaccccaaaaaacttgatttgggtgatggttcacgtagtgggccatcgccctgatagacggttttcgcccttt

gacgttggagtccacgttctttaatagtggactcttgttccaaactggaacaacactcaactctatctcgggctattcttttgatttataagggattt

tgccgatttcggtctattggttaaaaaatgagctgatttaacaaaaatttaacgcgaattttaacaaaatattaacgtttacaattttatggtgcactc

tcagtacaatctgctctgatgccgcatagttaagccagccccgacacccgccaacacccgctgacgcgccctgacgggcttgtctgctccc

ggcatccgcttacagacaagctgtgaccgtctccgggagctgcatgtgtcagaggttttcaccgtcatcaccgaaacgcgcgagacgaaa

gggcctcgtgatacgcctatttttataggttaatgtcatgataataatggtttcttagacgtcaggtggcactttcggggaaatgtgcgcggaa

cccctatttgtttattttctaaatacattcaaatatgtatccgctcatgagacaataaccctgataaatgcttcaataatattgaaaaaggaagagt

atgagtattcaacatttccgtgtcgcccttattccctttttgcggcattttgccttcctgtttttgctcacccagaaacgctggtgaaagtaaaaga

tgctgaagatcagttgggtgcacgagtgggttacatcgaactggatctcaacagcggtaagatccttgagagttttcgccccgaagaacgttt

tccaatgatgagcacttttaaagttctgctatgtggcgcggtattatcccgtattgacgccgggcaagagcaactcggtcgccgcatacactat

tctcagaatgacttggttgagtactcaccagtcacagaaaagcatcttacggatggcatgacagtaagagaattatgcagtgctgccataacc

atgagtgataacactgcggccaacttacttctgacaacgatcggaggaccgaaggagctaaccgcttttttgcacaacatgggggatcatgt

aactcgccttgatcgttgggaaccggagctgaatgaagccataccaaacgacgagcgtgacaccacgatgcctgtagcaatggcaacaac

gttgcgcaaactattaactggcgaactacttactctagcttcccggcaacaattaatagactggatggaggcggataaagttgcaggaccact

tctgcgctcggcccttccggctggctggtttattgctgataaatctggagccggtgagcgtggaagccgcggtatcattgcagcactggggc

cagatggtaagccctcccgtatcgtagttatctacacgacggggagtcaggcaactatggatgaacgaaatagacagatcgctgagatagg

tgcctcactgattaagcattggtaactgtcagaccaagtttactcatatatactttagattgatttaaaacttcatttttaatttaaaaggatctaggtg

aagatcctttttgataatctcatgaccaaaatcccttaacgtgagttttcgttccactgagcgtcagacccccgtagaaaagatcaaaggatcttct

tgagatcctttttttctgcgcgtaatctgctgcttgcaaacaaaaaaaccaccgctaccagcggtggtttgtttgccggatcaagagctaccaac

tcttttttccgaaggtaactggcttcagcagagcgcagataccaaatactgttcttctagtgtagccgtagttaggccaccacttcaagaactctg

tagcaccgcctacatacctcgctctgctaatcctgttaccagtggctgctgccagtggcgataagtcgtgtcttaccgggttggactcaagac

gatagttaccggataaggcgcagcggtcgggctgaacggggggttcgtgcacacagcccagcttggagcgaacgacctacaccgaact

gagatacctacagcgtgagctatgagaaagcgccacgcttcccgaagggagaaaggcggacaggtatccggtaagcggcagggtcgg

aacaggagagcgcacgagggagcttccaggggggaaacgcctggtatctttatagtcctgtcgggtttcgccacctctgacttgagcgtcga

tttttgtgatgctcgtcaggggggcggagcctatggaaaaacgccagcaacgcggcctttttacggttcctggccttttgctggccttttgctca

catgt

.

Claims

1. A guide RNA for a gene editing system, comprising a guide sequence having at least 80% sequence identity to the sequence as shown in any one of SEQ ID NOs: 10-18 and 40-230;

optionally, the guide sequence has at least 80% sequence identity to the sequence as shown in any one of SEQ ID NOs: 12, 13, 15, 40-43, 45-47, 52-60, 64, 65, 73, 216, 219, 220, 223, 227, 228, 229 and 230;
optionally, the guide sequence has at least 80% sequence identity to the sequence as shown in any one of SEQ ID NOs: 12, 13, 15, 47, 58, 59, 227, 228 and 230.

2. The guide RNA according to claim 1, wherein the guide sequence is engineered to hybridize with a target RNA;
optionally, the guide sequence is engineered to hybridize with a target RNA with no more than 6 nucleotide mismatches.

3. The guide RNA according to claim 1, wherein the guide RNA is capable of forming a complex with an RNA-guided nuclease and guiding the sequence-specific binding of the complex to a target RNA.

4. The guide RNA according to claim 1, wherein the guide RNA is capable of forming a complex with an RNA-guided nuclease and guiding the complex to bind to and cleave a target RNA.

5. The guide RNA according to claim 3 or 4, wherein the complex reduces the level of the target RNA in a cell.

6. The guide RNA according to claim 3 or 4, wherein the complex reduces the level of the target RNA in a cell by at least 5%.

7. The guide RNA according to claim 4, wherein the number of off-target genes when the complex binds to and cleaves the target RNA is less than 40.

8. The guide RNA according to claim 7, wherein the number of off-target genes is determined by taking the intersection of a set of differentially expressed genes determined by RNA sequencing with a set of off-target genes predicted by a program.

9. The guide RNA according to claim 3 or 4, wherein the complex reduces the level of the protein encoded by the target RNA in a cell.

10. The guide RNA according to claim 3 or 4, wherein the complex reduces the level of the protein encoded by the target RNA in a cell by at least 5%.

11. The guide RNA according to any one of claims 3-10, wherein the RNA-guided nuclease is a Cas protein;

optionally, the Cas protein is a Cas13 protein;
optionally, the Cas protein is a Cas13a protein, a Cas13b protein, a Cas13c protein or a Cas13d protein.

12. The guide RNA according to claim 11, wherein the Cas protein is a Cas13 protein,

optionally, the Cas protein comprises an amino acid sequence having ≥50% sequence identity to the sequence as shown in any one of SEQ ID NOs: 1-3;
optionally, the Cas protein comprises an amino acid sequence having ≥50% sequence identity to the sequence as shown in SEQ ID NO: 3, and the Cas protein: (a) mutates to other residues at the positions corresponding to residues R210 and H215 in the sequence as shown in SEQ ID NO: 3; (b) mutates to other residues at the positions corresponding to residues R785 and H790 in the sequence as shown in SEQ ID NO: 3; (c) mutates to other residues at the positions corresponding to residues R210, H215, R785 and H790 in the sequence as shown in SEQ ID NO: 3; or (d) mutates to other residues at the positions corresponding to residues R210, H215, R750A, H755A, R785 and H790 in the sequence as shown in SEQ ID NO: 3;
optionally, the Cas protein comprises a sequence having ≥50% sequence identity to CasRx.

13. The guide RNA according to any one of claims 3-12, wherein the RNA-guided nuclease comprises a nuclease portion and a fused homologous or heterologous domain;
optionally, the RNA-guided nuclease comprises any one or more of the following: a subcellular localization signal, a deaminase domain, a translation activation domain, a translation repression domain, an RNA methylation domain, an RNA demethylation domain, a nuclease domain, a splicing factor domain, a reporter tag and an affinity tag.

14. The guide RNA according to any one of claims 1-13, wherein the guide sequence comprises 20-40 nucleotides.

15. The guide RNA according to any one of claims 1-14, wherein the guide RNA comprises a guide sequence and a direct repeat sequence, and the direct repeat sequence interacts with the RNA-guided nuclease.

16. The guide RNA according to claim 15, wherein the guide sequence is located at the 3' end or the 5' end of the direct repeat sequence.

17. The guide RNA according to any one of claims 15-16, wherein the direct repeat sequence comprises the sequence as shown in any one of SEQ ID NOs: 19-21.

18. The guide RNA according to any one of claims 1-17, wherein the guide RNA comprises an aptamer sequence.

19. The guide RNA according to claim 18, wherein the aptamer sequence is inserted into a loop of the secondary structure of the direct repeat sequence of the guide RNA.

20. The guide RNA according to any one of claims 1-19, wherein the guide RNA comprises a modified nucleotide.

21. The guide RNA according to claim 20, wherein the modified nucleotide comprises 2'-O-methyl modification, 2'-O-methyl-3'-thiophosphate modification or 2'-O-methyl-3'-thio PACE modification.

22. The guide RNA according to claim 20, wherein the modified nucleotide is selected from a deoxyribonucleotide and a locked nucleic acid.

23. The guide RNA according to any one of claims 2-22, wherein the target RNA is VEGFA RNA;

   optionally, the target RNA is human VEGFA RNA;
   optionally, the target RNA is VEGFA pre-mRNA or mature VEGFA mRNA.

24. The guide RNA according to any one of claims 2-23, wherein the target RNA is located in the nucleus or cytoplasm of a cell.

25. An isolated nucleic acid encoding the guide RNA according to any one of claims 1-24.

26. A vector comprising the nucleic acid according to claim 25, and a regulatory sequence for regulating the expression of the guide RNA.

27. The vector according to claim 26, wherein the vector is an adeno-associated virus vector.

28. The vector according to claim 26, wherein the regulatory sequence is a promoter or an enhancer.

29. The vector according to claim 26, wherein the regulatory sequence is a U6 promoter, an eye-specific promoter or a CBh promoter.

30. A gene editing system, comprising:

   (a) the guide RNA according to any one of claims 1-24 or a polynucleotide encoding the guide RNA; and
   (b) an RNA-guided nuclease or a polynucleotide encoding the RNA-guided nuclease;

   the guide RNA is capable of forming a complex with the RNA-guided nuclease and guiding the sequence-specific binding of the complex to a target RNA.

31. The gene editing system according to claim 30, wherein the guide RNA is capable of forming a complex with the RNA-guided nuclease and guiding the complex to bind to and cleave the target RNA.

32. The gene editing system according to claim 30, wherein the RNA-guided nuclease is a Cas protein;

   optionally, the Cas protein is a Cas13 protein;

optionally, the Cas protein is a Cas13a protein, a Cas13b protein, a Cas13c protein or a Cas13d protein.

33. The gene editing system according to claim 32, wherein the Cas protein is a Cas13 protein,

optionally, the Cas protein comprises a sequence having ≥50% sequence identity to the sequence as shown in any one of SEQ ID NOs: 1-3;

optionally, the Cas protein comprises a sequence having ≥50% sequence identity to the sequence as shown in SEQ ID NO: 3, and the Cas protein: (a) mutates to other residues at the positions corresponding to residues R210 and H215 in the sequence as shown in SEQ ID NO: 3; (b) mutates to other residues at the positions corresponding to residues R785 and H790 in the sequence as shown in SEQ ID NO: 3; (c) mutates to other residues at the positions corresponding to residues R210, H215, R785 and H790 in the sequence as shown in SEQ ID NO: 3; or (d) mutates to other residues at the positions corresponding to residues R210, H215, R750A, H755A, R785 and H790 in the sequence as shown in SEQ ID NO: 3;

optionally, the Cas protein comprises a sequence having ≥50% sequence identity to CasRx.

34. The gene editing system according to any one of claims 30-33, wherein the RNA-guided nuclease comprises a nuclease portion and a fused homologous or heterologous domain;

optionally, the RNA-guided nuclease comprises any one or more of the following: a subcellular localization signal, a deaminase domain, a translation activation domain, a translation repression domain, an RNA methylation domain, an RNA demethylation domain, a nuclease domain, a splicing factor domain, a reporter tag and an affinity tag;

optionally, the subcellular localization signal is selected from a nuclear localization signal and a nuclear export signal.

35. The gene editing system according to claim 30, wherein the sequence of the polynucleotide encoding the guide RNA is linked to a first regulatory sequence, wherein the first regulatory sequence is used to regulate the expression of the guide RNA;

the sequence of the polynucleotide encoding the RNA-guided nuclease is linked to a second regulatory sequence, wherein the second regulatory sequence is used to regulate the expression of the RNA-guided nuclease.

36. A vector system comprising the gene editing system according to any one of claims 30-35, wherein the vector system comprises a polynucleotide sequence encoding the guide RNA and a first regulatory sequence for regulating the expression of the guide RNA; and a polynucleotide sequence encoding the RNA-guided nuclease and a second regulatory sequence for regulating the expression of the RNA-guided nuclease.

37. The vector system according to claim 36, wherein the first regulatory sequence and/or the second regulatory sequence are a promoter or an enhancer.

38. An adeno-associated viral vector comprising the gene editing system according to any one of claims 30-35, wherein the adeno-associated viral vector comprises a DNA encoding the RNA-guided nuclease and the guide RNA.

39. A lipid nanoparticle comprising the gene editing system according to any one of claims 30-35, wherein the lipid nanoparticle comprises the guide RNA and an mRNA encoding the RNA-guided nuclease.

40. A lentiviral vector comprising the gene editing system according to any one of claims 30-35, wherein the lentiviral vector comprises the guide RNA and an mRNA encoding the RNA-guided nuclease; optionally, the lentiviral vector is pseudotyped with an envelope protein; optionally, the mRNA encoding the RNA-guided nuclease is linked to an aptamer sequence.

41. A ribonucleoprotein complex comprising the gene editing system according to any one of claims 30-35, wherein the ribonucleoprotein complex is formed by the guide RNA and the RNA-guided nuclease.

42. A virus-like particle comprising the gene editing system according to any one of claims 30-35, wherein the virus-like particle comprises a ribonucleoprotein complex formed by the guide RNA and the RNA-guided nuclease; optionally, the RNA-guided nuclease is fused with a gag protein.

43. A eukaryotic cell comprising the gene editing system according to any one of claims 30-35.

44. A pharmaceutical composition, comprising the gene editing system according to any one of claims 30-35, the vector system according to claim 36 or 37, the adeno-associated virus vector according to claim 38, the lipid nanoparticle according to claim 39, the lentiviral vector according to claim 40, the ribonucleoprotein complex according to claim 41, the virus-like particle according to claim 42, or the eukaryotic cell according to claim 43.

45. The pharmaceutical composition according to claim 44, further comprising a pharmaceutically acceptable excipient.

46. Use of the guide RNA according to any one of claims 1-24, the isolated nucleic acid according to claim 25, the vector according to any one of claims 26-29, the gene editing system according to any one of claims 30-35, the vector system according to claim 36 or 37, the adeno-associated virus vector according to claim 38, the lipid nanoparticle according to claim 39, the lentiviral vector according to claim 40, the ribonucleoprotein complex according to claim 41, the virus-like particle according to claim 42, the eukaryotic cell according to claim 43, or the pharmaceutical composition according to claim 44 or 45 in any one of the following or in the manufacture of a reagent to achieve any one of the following solutions:
cleaving one or more target RNA molecules or nicking one or more target RNA molecules, activating or upregulating one or more target RNA molecules, activating or inhibiting the translation of one or more target RNA molecules, inactivating one or more target RNA molecules, visualizing, labeling or detecting one or more target RNA molecules, binding to one or more target RNA molecules, transporting one or more target RNA molecules, and masking one or more target RNA molecules.

47. The use according to claim 46, wherein the target RNA is VEGFA RNA;
optionally, the target RNA is human VEGFA RNA.

48. A method for diagnosing, treating or preventing a disease or disorder associated with a target RNA, wherein the method comprises administering an effective amount of the guide RNA according to any one of claims 1-24, the isolated nucleic acid according to claim 25, the vector according to any one of claims 26-29, the gene editing system according to any one of claims 30-35, the vector system according to any one of claims 36-37, the adeno-associated virus vector according to claim 38, the lipid nanoparticle according to claim 39, the lentiviral vector according to claim 40, the ribonucleoprotein complex according to claim 41, the virus-like particle according to claim 42, or the pharmaceutical composition according to claim 44 or 45 to a sample of a subject in need thereof or to a subject in need thereof.

49. The method according to claim 48, wherein the disease or disorder associated with the target RNA refers to a disease or disorder caused by abnormal expression of the target RNA.

50. The method according to claim 48, wherein the target RNA is VEGFA RNA;
optionally, the target RNA is human VEGFA RNA.

51. Use of the guide RNA according to any one of claims 1-24, the isolated nucleic acid according to claim 25, the vector according to any one of claims 26-29, the gene editing system according to any one of claims 30-35, the vector system according to any one of claims 36-37, the adeno-associated virus vector according to claim 38, the lipid nanoparticle according to claim 39, the lentiviral vector according to claim 40, the ribonucleoprotein complex according to claim 41, and the virus-like particle according to claim 42 in the manufacture of a medicament for diagnosing, treating or preventing a disease or disorder associated with a target RNA.

52. The use according to claim 51, wherein the disease or disorder associated with the target RNA refers to a disease or disorder caused by abnormal expression of the target RNA.

53. The use according to claim 51, wherein the target RNA is VEGFA RNA;
optionally, the target RNA is human VEGFA RNA.

FIG. 1

FIG. 2

FIG. 3

FIG. 4A

FIG. 4B

FIG. 4C

FIG. 5

FIG. 6

FIG. 7

FIG. 8

FIG. 9A

FIG. 9B

FIG. 10

FIG. 11

FIG. 12

C13-2-V
C13-2-gRNA-5
C13-2-gRNA-18
C13-2-gRNA-29
C13-2-gRNA-30
C13-2-gRNA-5-2
C13-2-gRNA-5-3
C13-2-gRNA-5-4
C13-2-gRNA-5-5

C13-2-V  C13-2-gRNA-4  C13-2-gRNA-5

FIG. 13

FIG. 14

FIG. 15

## INTERNATIONAL SEARCH REPORT

| International application No. |
|---|
| **PCT/CN2023/125680** |

| A. | CLASSIFICATION OF SUBJECT MATTER |
|---|---|

C12N15/113(2010.01)i; C12N15/85(2006.01)i; C12N5/10(2006.01)i; A61K31/7105(2006.01)i; A61K48/00(2006.01)i; A61P3/10(2006.01)i; A61P13/12(2006.01)i; A61P17/06(2006.01)i; A61P19/02(2006.01)i; A61P27/02(2006.01)i; A61P35/00(2006.01)i; A61P37/02(2006.01)i

According to International Patent Classification (IPC) or to both national classification and IPC

| B. | FIELDS SEARCHED |
|---|---|

Minimum documentation searched (classification system followed by classification symbols)

IPC:C12N A61K A61P

Documentation searched other than minimum documentation to the extent that such documents are included in the fields searched

Electronic data base consulted during the international search (name of data base and, where practicable, search terms used)

CNABS; CNTXT; CJFD; WPABS; WPABSC; ENTXTC; ENTXT; DWPI; CNKI; 万方, WANFANG; ISI Web of Science; STN; GenBank; EMBL; 中国专利生物序列检索系统, China Patent Biological Sequence Search System: 广州瑞风生物科技有限公司, 梁峻彬, 指导RNA, CRISPR效应蛋白, 血管内皮生长因子A, 基于序列10的检索, search based on sequence 10, GUANGZHOU REFORGENE MEDICINE, gRNA, Cas, VEGFA

| C. | DOCUMENTS CONSIDERED TO BE RELEVANT |
|---|---|

| Category* | Citation of document, with indication, where appropriate, of the relevant passages | Relevant to claim No. |
|---|---|---|
| X | CN 112662674 A (GUANGZHOU REFORGENE BIOLOGICAL TECHNOLOGY CO., LTD.) 16 April 2021 (2021-04-16) claims 1-10, and description, paragraph [0060] | 1-47, 51-53 |

☐ Further documents are listed in the continuation of Box C.   ☑ See patent family annex.

| * | Special categories of cited documents: |
|---|---|
| "A" | document defining the general state of the art which is not considered to be of particular relevance |
| "D" | document cited by the applicant in the international application |
| "E" | earlier application or patent but published on or after the international filing date |
| "L" | document which may throw doubts on priority claim(s) or which is cited to establish the publication date of another citation or other special reason (as specified) |
| "O" | document referring to an oral disclosure, use, exhibition or other means |
| "P" | document published prior to the international filing date but later than the priority date claimed |

| "T" | later document published after the international filing date or priority date and not in conflict with the application but cited to understand the principle or theory underlying the invention |
|---|---|
| "X" | document of particular relevance; the claimed invention cannot be considered novel or cannot be considered to involve an inventive step when the document is taken alone |
| "Y" | document of particular relevance; the claimed invention cannot be considered to involve an inventive step when the document is combined with one or more other such documents, such combination being obvious to a person skilled in the art |
| "&" | document member of the same patent family |

| Date of the actual completion of the international search | Date of mailing of the international search report |
|---|---|
| **02 January 2024** | **21 January 2024** |

| Name and mailing address of the ISA/CN | Authorized officer |
|---|---|
| **China National Intellectual Property Administration (ISA/CN)** **China No. 6, Xitucheng Road, Jimenqiao, Haidian District, Beijing 100088** | |
| | Telephone No. |

Form PCT/ISA/210 (second sheet) (July 2022)

## INTERNATIONAL SEARCH REPORT

| International application No. |
| --- |
| **PCT/CN2023/125680** |

**Box No. I        Nucleotide and/or amino acid sequence(s) (Continuation of item 1.c of the first sheet)**

1.  With regard to any nucleotide and/or amino acid sequence disclosed in the international application, the international search was carried out on the basis of a sequence listing:

   a.  ☑ forming part of the international application as filed.

   b.  ☐ furnished subsequent to the international filing date for the purposes of international search (Rule 13*ter*.1(a)),

   ☐ accompanied by a statement to the effect that the sequence listing does not go beyond the disclosure in the international application as filed.

2.  ☐ With regard to any nucleotide and/or amino acid sequence disclosed in the international application, this report has been established to the extent that a meaningful search could be carried out without a WIPO Standard ST.26 compliant sequence listing.

3.  Additional comments:

Form PCT/ISA/210 (continuation of first sheet) (July 2022)

## INTERNATIONAL SEARCH REPORT

| International application No. |
| --- |
| **PCT/CN2023/125680** |

**Box No. II    Observations where certain claims were found unsearchable (Continuation of item 2 of first sheet)**

This international search report has not been established in respect of certain claims under Article 17(2)(a) for the following reasons:

1. ☑ Claims Nos.: **48-50**
   because they relate to subject matter not required to be searched by this Authority, namely:

   Claims 48-50 relate to a method for diagnosing, treating or preventing a disease or condition associated with a target RNA, which is a method for diagnosing a disease or a method for treatment of the human or animal body by therapy, and falls within subject matter for which no search is required by the searching authority as defined in PCT Rule 39.1(iv).

2. ☐ Claims Nos.:
   because they relate to parts of the international application that do not comply with the prescribed requirements to such an extent that no meaningful international search can be carried out, specifically:

3. ☐ Claims Nos.:
   because they are dependent claims and are not drafted in accordance with the second and third sentences of Rule 6.4(a).

**Box No. III    Observations where unity of invention is lacking (Continuation of item 3 of first sheet)**

This International Searching Authority found multiple inventions in this international application, as follows:

Invention 1: the solution in claims 1-53 which relates to a guide RNA as shown in SEQ ID NO: 10

Inventions 2-200: the solutions in claims 1-53 which relate to guide RNAs as shown in SEQ ID NOs: 11-18 and 40-230

This International Authority is of the opinion that the claims include 200 inventions. The same or corresponding technical feature of the 200 inventions is a guide RNA targeting VEGFA. However, the prior art (for example, CN 112662674 A) discloses gRNA for targeted editing of a VEGFA gene exon region, and the use thereof (see claims 1-6). Therefore, the 200 inventions do not have a same or corresponding special technical feature that defines a contribution which the inventions make over the prior art, do not have a technical relationship therebetween, do not fall within a single general inventive concept, and therefore do not comply with the requirement of unity of invention and do not comply with PCT Rule 13.1.

Form PCT/ISA/210 (continuation of first sheet) (July 2022)

## INTERNATIONAL SEARCH REPORT

| International application No. |
| --- |
| **PCT/CN2023/125680** |

**Box No. III**      **Observations where unity of invention is lacking (Continuation of item 3 of first sheet)**

1. ☐ As all required additional search fees were timely paid by the applicant, this international search report covers all searchable claims.

2. ☐ As all searchable claims could be searched without effort justifying additional fees, this Authority did not invite payment of additional fees.

3. ☐ As only some of the required additional search fees were timely paid by the applicant, this international search report covers only those claims for which fees were paid, specifically claims Nos.:

4. ☑ No required additional search fees were timely paid by the applicant. Consequently, this international search report is restricted to the invention first mentioned in the claims; it is covered by claims Nos.: **the solution in claims 1-53 which relates to a guide RNA as shown in SEQ ID NO: 10**

**Remark on Protest**
    ☐ The additional search fees were accompanied by the applicant's protest and, where applicable, the payment of a protest fee.

    ☐ The additional search fees were accompanied by the applicant's protest but the applicable protest fee was not paid within the time limit specified in the invitation.

    ☐ No protest accompanied the payment of additional search fees.

Form PCT/ISA/210 (continuation of first sheet) (July 2022)

## INTERNATIONAL SEARCH REPORT
### Information on patent family members

International application No.

**PCT/CN2023/125680**

| Patent document cited in search report | | | Publication date (day/month/year) | Patent family member(s) | Publication date (day/month/year) |
|---|---|---|---|---|---|
| CN | 112662674 | A | 16 April 2021 | None | |

Form PCT/ISA/210 (patent family annex) (July 2022)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- CN 2022113208591
- CN 2023100880960
- CN 2023103961404
- CN 112143701 A
- CN 112430597 A